(19) Europäisches Patentamt
European Patent Office
Office européen des brevets

(11) EP 4 006 033 A1

(12) **EUROPEAN PATENT APPLICATION**
published in accordance with Art. 153(4) EPC

(43) Date of publication:
**01.06.2022 Bulletin 2022/22**

(21) Application number: **20847935.2**

(22) Date of filing: **29.07.2020**

(51) International Patent Classification (IPC):
***C07D 487/04*** (2006.01) ***C07D 471/04*** (2006.01)
***A61K 31/53*** (2006.01) ***A61P 25/00*** (2006.01)
***A61P 35/00*** (2006.01) ***A61P 1/16*** (2006.01)

(52) Cooperative Patent Classification (CPC):
**A61K 31/53; A61K 45/06; A61P 1/00; A61P 1/16; A61P 3/10; A61P 7/04; A61P 9/00; A61P 9/04; A61P 9/10; A61P 11/00; A61P 11/02; A61P 11/06; A61P 15/00; A61P 17/00; A61P 17/06;** (Cont.)

(86) International application number:
**PCT/CN2020/105429**

(87) International publication number:
**WO 2021/018173 (04.02.2021 Gazette 2021/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.07.2019 CN 201910695723**

(71) Applicant: **Xiamen Biotime Biotechnology Co., Ltd.**
**Haicang District**
**Xiamen**
**Fujian 361026 (CN)**

(72) Inventors:
- **YU, Zhiyong**
  **Hangzhou, Zhejiang 311121 (CN)**
- **LIU, Shifeng**
  **Hangzhou, Zhejiang 311121 (CN)**
- **ZHAO, Meng**
  **Hangzhou, Zhejiang 311121 (CN)**
- **LI, Gang**
  **Hangzhou, Zhejiang 311121 (CN)**
- **SHI, Yongqiang**
  **Hangzhou, Zhejiang 311121 (CN)**
- **LV, Meng**
  **Hangzhou, Zhejiang 311121 (CN)**

(74) Representative: **Müller, Christian Stefan Gerd**
**ZSP Patentanwälte PartG mbB**
**Hansastraße 32**
**80686 München (DE)**

# (54) ADENOSINE RECEPTOR ANTAGONIST

(57) A compound represented by formula (I) and a pharmaceutical composition thereof. The compound represented by formula (I) may be used as an adenosine receptor inhibitor, especially as an A2A and/or A2B inhibitor, for example, the compound may be used to prevent or treat diseases related to A2A and/or A2B activity or expression.

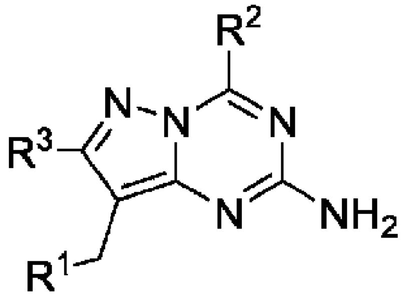

(I)

EP 4 006 033 A1

(52) Cooperative Patent Classification (CPC): (Cont.)
**A61P 19/02; A61P 19/06; A61P 25/00;**
**A61P 25/16; A61P 25/28; A61P 29/00;**
**A61P 35/00; A61P 35/02; A61P 37/06;**
**A61P 37/08; C07D 471/04; C07D 487/04**

## Description

This application claims priority to Chinese Patent Application No. 201910695723.0 entitled "Adenosine Receptor Antagonists" filed to China National Intellectual Property Administration on July 30, 2019, which is incorporated herein by reference in its entirety.

## Technical Field

The present invention provides a novel class of heterocyclic compounds, preparation methods therefor and uses thereof as adenosine receptor (particularly A2A and/or A2B receptor) antagonists.

## Background Art

Some breakthrough progress has been made on checkpoint inhibitors in the field of immunotherapy. A large number of clinical studies have demonstrated that PD-1/PDL-1 antibodies have certain clinical effects on a variety of tumors (small cell lung cancer, melanoma, head and neck cancer, renal cancer, etc.), but the response rate of single-agent is generally low, ranging from 20% to 40%. Solid tumors include not only tumor cell components, but also a considerable number of other non-tumor cell components found in tumor tissues, which constitute the so-called tumor microenvironment and play an important role in tumor invasion, proliferation, and metastasis. Studies have shown that the combination of PD-1/PDL-1 antibodies and other immunomodulatory small molecules (eliminating the immune tolerance of tumor microenvironment) is one of the effective means to solve the low response rate.

Normally, a human body may effectively control tumor cells by relying on a complete immune mechanism, and T lymphocytes, NK cells, and macrophages have killing effects on the tumor cells, however, when the functions of the cancer cells or the immune cells are changed, the cancer cells can escape from the immune system of the body, forming tumors by malignant hyperplasia. As a signaling molecule used in vivo to limit inflammation and immune responses, adenosine will be greatly elevated in metabolic disorders and cell injury. Activated adenosine receptors are involved in the immune regulation of the body, maintaining higher levels in many different types of tumor microenvironments. The adenosine produced by tumors may interact with adenosine receptors (ARs) on the surface of invading immune cells; there are four subtypes of ARs, described as A1, A2A, A2B, and A3, belonging to a group of G protein-coupled receptors (GPCR) and mainly coupling to Gs and Ga proteins. Each receptor shows a different affinity for adenosine, wherein AIR, A2AR and A3R are high-affinity receptors that may be activated by adenosine at a lower concentration (250-700 nM); while A2BR is a low-affinity receptor which requires higher concentrations (25 μM) of adenosine for activation. Adenosine receptors may also be classified according to the induction of downstream signal small molecule cAMP, when A2A and A2B receptors are activated, a conformational change in the receptor results in the release of activated Gs proteins, activation of adenylyl cyclase, and acceleration of the conversion of ATP to cAMP. The increased production of cAMP often accompanied by strong immunosuppression is inhibited by activation of A1 and A3 receptors, thus the activation is generally considered to activate immunization.

Adenosine A2A receptors are predominantly localized in the striatum of the brain, the spleen, the thymus, lymphocytes, and platelets, also found to be localized in the heart, lung, and blood vessels, frequently expressed in cells of the immune system such as T cells, NK cells, macrophages, and dendritic cells. Early use of A2A receptor antagonists was primarily used for the treatment of nervous system diseases in the early stage, such as Parkinson's disease, Huntington's disease, Alzheimer's disease, attention-deficit related disorders, and psychosis. In recent studies, A2A receptor antagonists have been found to improve the antigen presentation of dendritic cells, the activation and killing ability of T cells and natural killer cells, inhibit regulatory T cells (T-regs), MDSCs and TAM, eliminate tumor immune tolerance, and promote the occurrence of an anti-tumor immune response, leading to tumor regression. Therefore, A2A receptor antagonists may be one of the effective methods for the treatment of tumors. A2A receptor antagonists may be used alone or in combination with other anti-tumor drugs, especially in combination with immune checkpoint inhibitors, which is a hotspot in clinical research.

Adenosine A2B receptors are expressed in a variety of tissues, vasculature, the brain, small intestine, and tumors, and also in a variety of cells, including mast cells, dendritic cells, neutrophils, macrophages, and lymphocytes in the immune system, as well as endothelial cells, neural cells, and glial cells. The wide expression of the A2B receptor makes it a target for the study of a variety of diseases, including cardiovascular diseases, pulmonary diseases, diabetes, and cancer. Studies have shown that A2B antagonists can prevent the growth of tumors (bladder cancer, breast cancer), and high expression of A2B in patients with triple-negative breast cancer can also lead to a reduced survival rate in the treatment.

International Patent Application Publication Nos. WO2018/178338, WO2011/095625, WO2001/92264, WO2003/048165, WO2004/09443, WO2002/055083, etc. disclose compounds useful as A2A receptor antagonists, and International Patent Application Publication WO2005/040155, WO2016/164838, WO2016/150901, WO20161/35048,

WO2015/05206, WO2012/076974, WO2011/005871, and Chinese Patent Application Publication No. CN102532137, and U.S. Patent Application Publication No. Us 20140142113, etc. disclose compounds useful as A2B receptor antagonists. However, there is still a need for inhibitors having a better inhibitory effect on adenosine receptors; in particular, the antagonists capable of inhibiting both A2A and A2B receptors have important clinical value and therapeutic significance, but few reports are available. There is an urgent need in the art for adenosine inhibitors with better inhibitory effects, in particular novel antagonist compounds capable of simultaneously inhibiting A2A and A2B receptors.

## Summary of the Invention

After long and intensive studies, we have unexpectedly found a class of heterocyclic compounds with good A2A and/or A2B receptor inhibitory activity.

Based on the above findings, in a first aspect of the present invention, there is provided a compound of Formula (I), or a pharmaceutically acceptable salt, a prodrug, an isotopic derivative, a hydrate, an isomer, a solvate, or a metabolite thereof,

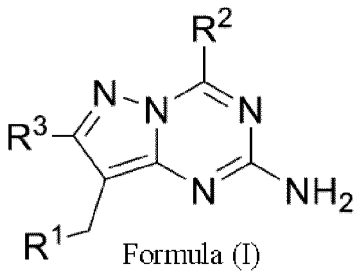

Formula (I)

wherein:

$R^1$ and $R^2$ each independently represent: $C_3$-$C_{12}$ cycloalkyl, 3-12 membered heterocycloalkyl, $C_6$-$C_{12}$ aryl, 5-12 membered heteroaryl;

wherein the $R^1$ and $R^2$ are optionally each independently substituted with 0, 1, 2, 3, 4, or 5 substituents selected from $R^4$, wherein $R^4$ is selected from the group consisting of $C_1$-$C_6$ alkyl, hydroxy ($C_1$-$C_6$ alkyl)-, $C_2$-$C_6$ alkenyl, $C_2$-$C_6$ alkynyl, $C_3$-$C_6$ cycloalkyl, $C_6$-$C_{12}$ aryl, 5-12 membered heteroaryl, $C_1$-$C_6$ haloalkyl, halogen, oxo, nitro, cyano, $-NR^aR^b$, $-OR^a$, $-SR^a$, $-SOR^a$, $-SO_2R^a$, $-SO_3R^a$, $-NR^aC(O)R^a$, $-NR^aC(O)OR^a$, $-NR^aS(O)_2R^a$, $-C(O)OR^a$, $-C(O)NR^aR^b$, $-C(O)R^a$, $(CR^aR^b)_m$-$OR^a$, $(CR^aR^b)_m$-$NR^aR^b$, $-(CR^aR^b)_m$-$NR^a$-$(CR^aR^b)_n$-$OR^a$, $-(CR^aR^b)_m$-O-$(CR^aR^b)_n$-$NR^aR^b$, $-(CR^aR^b)_m$-O-$(CR^aR^b)_n$-$OR^a$ and $-(CR^aR^b)_m$-$NR^a$-$(CR^aR^b)_n$-$NR^aR^b$; or when the number of substituents is 2, and the 2 substituents are located in adjacent positions, they are optionally cyclized with each other into saturated or unsaturated 4- to 7-membered rings, and further, the ring optionally contains 0, 1 or 2 heteroatoms selected from O, S, or N;

$R^3$ represents hydrogen, $C_1$-$C_6$ alkyl, hydroxy ($C_1$-$C_6$ alkyl)-, $C_2$-$C_6$ alkenyl, $C_2$-$C_6$ alkynyl, $C_3$-$C_6$ cycloalkyl, $C_6$-$C_{12}$ aryl, 5-12 membered heteroaryl, $C_1$-$C_6$ haloalkyl, halogen, nitro, cyano, $-NR^aR^b$, $-OR^a$, $-SR^a$, $-SOR^a$, $-SO_2R^a$, $-SO_3R^a$, $-NR^aC(O)R^a$, $-NR^aC(O)OR^a$, $-NR^aS(O)_2R^a$, $-C(O)OR^a$, $-C(O)NR^aR^b$, $-C(O)R^a$, $(CR^aR^b)_m$-$OR^a$, $(CR^aR^b)_m$-$NR^aR^b$, $(CR^aR^b)_m$-$NR^a$-$(CR^aR^b)_n$-$OR^a$, $-(CR^aR^b)_m$-O-$(CR^aR^b)_n$-$NR^aR^b$, $-(CR^aR^b)_m$-O-$(CR^aR^b)_n$-$OR^a$, or $-(CR^aR^b)_m$-$NR^a$-$(CR^aR^b)_n$-$NR^aR^b$;

wherein $R^a$ and $R^b$ each independently represent hydrogen, halogen, $C_1$-$C_6$ alkyl, $C_3$-$C_6$ cycloalkyl, -($C_0$-$C_6$ alkylene)-($C_6$-$C_{12}$)aryl;

alternatively, when being attached to the same atom, $R^a$ and $R^b$ together with the atom bound thereto are optionally cyclized to each other into 3- to 7-membered rings optionally containing 0, 1, or 2 heteroatoms selected from O, N, or S;

n and m each independently represent 0, 1, 2, 3, or 4.

Preferably, the compound of Formula (I) has the following structure of Formula (II):

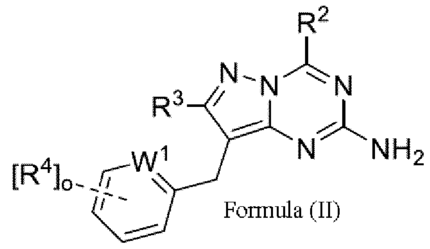

Formula (II)

wherein $W_1$ is selected from $CR^5$ or N, and $R^5$ has the same definition as $R^4$; $R^2$, $R^3$, and $R^4$ are as defined for Formula (I), and o is 0, 1, 2, or 3.

Preferably, the compound of Formula (I) has the following structure of Formula (III):

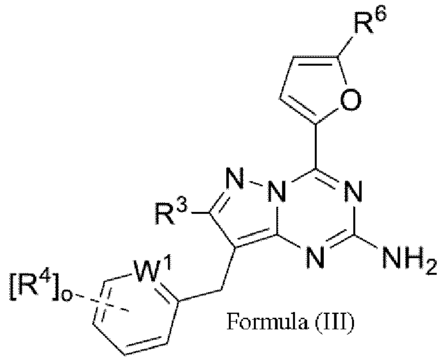

Formula (III)

wherein $R^6$ is selected from hydrogen, $C_1$-$C_6$ alkyl, $C_2$-$C_6$ alkenyl, $C_2$-$C_6$ alkynyl, $C_3$-$C_6$ cycloalkyl, $C_1$-$C_6$ haloalkyl, $C_1$-$C_6$ alkoxy, $C_1$-$C_6$ alkylthio, halogen, nitro, $-NR^cR^d$, cyano, $-SO_2R^c$, $-SO_3R^c$; $W_1$ is selected from $CR^5$ or N, $R^5$ has the same definition as $R^4$; $R^3$ and $R^4$ as defined in Formula (I), and o is 0, 1, 2, or 3.

where $R^c$ and $R^d$ each independently represent hydrogen, halogen, $C_1$-$C_6$ alkyl, or $C_3$-$C_6$ cycloalkyl.

alternatively, when being attached to the same atom, $R^c$ and $R^d$ together with the atom bound thereto are optionally cyclized to each other into 3- to 7-membered rings optionally containing 0, 1, or 2 heteroatoms selected from O, N, or S.

Preferably, the compound of the present invention is selected from the following structures:

| | | |
|---|---|---|
| 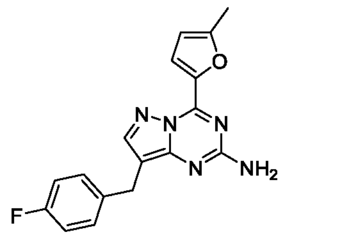 | 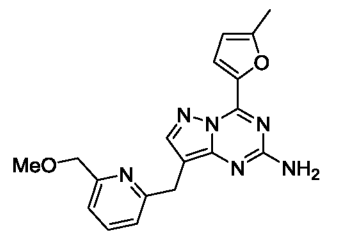 | 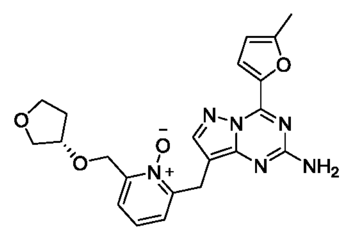 |
| 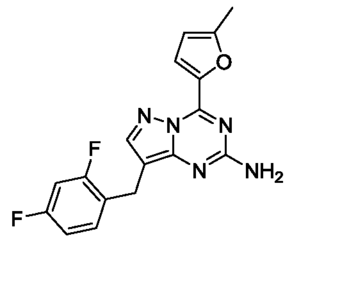 | 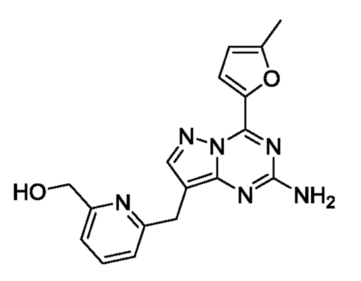 | 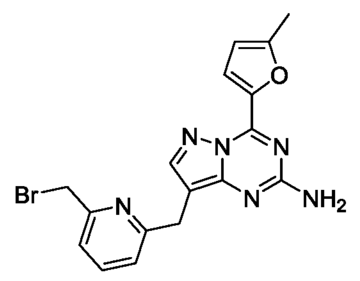 |
| 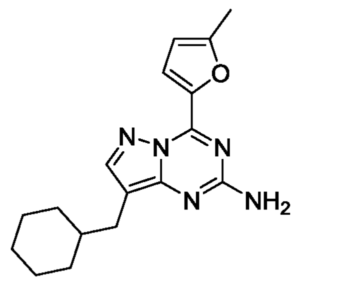 | 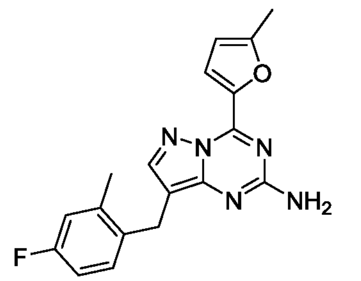 | 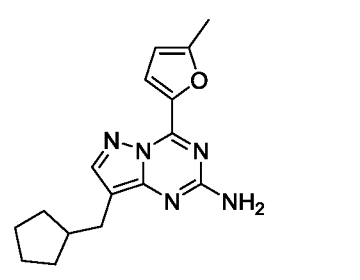 |
| 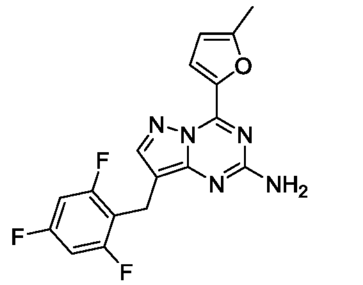 | 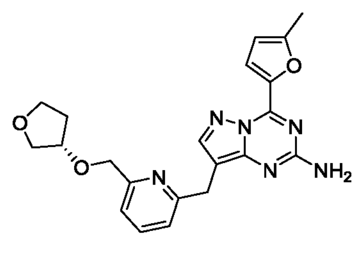 | 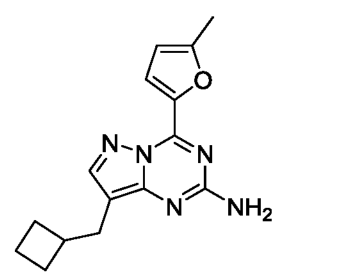 |
| 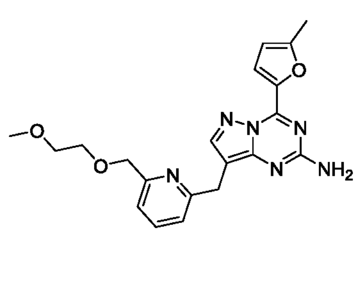 | 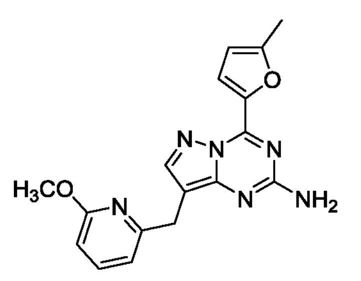 | 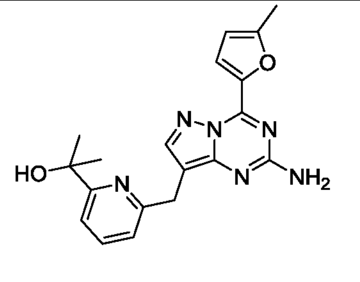 |
| 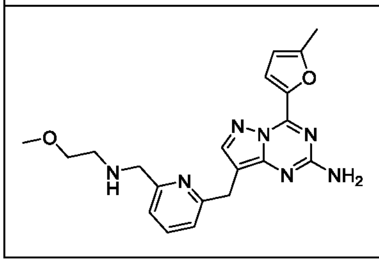 | 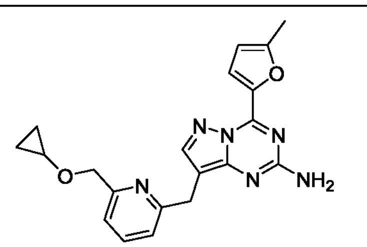 | 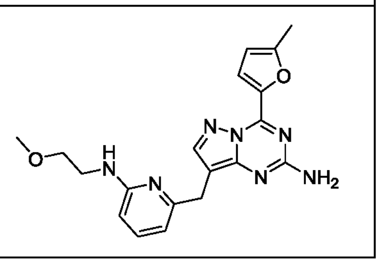 |

| | | |
|---|---|---|
| 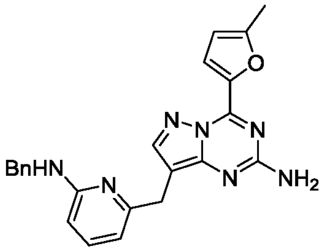 | 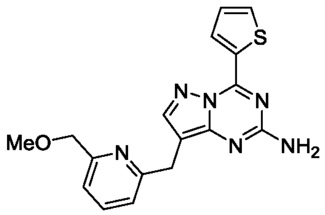 | 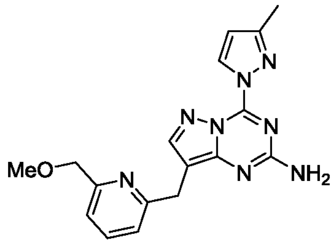 |
| 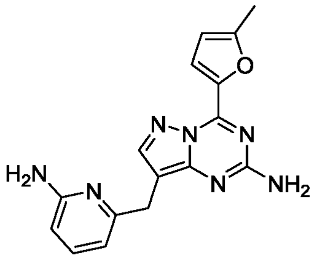 | 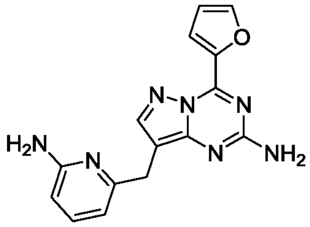 | 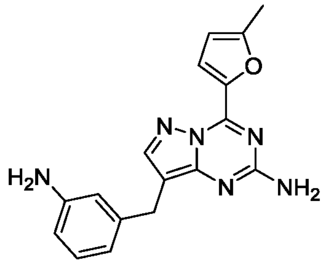 |
| 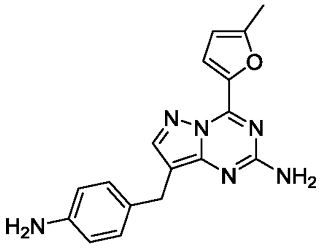 | 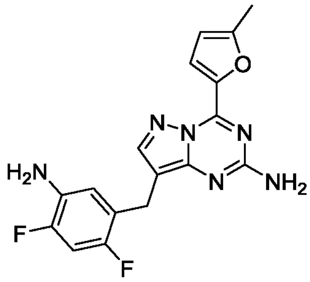 | 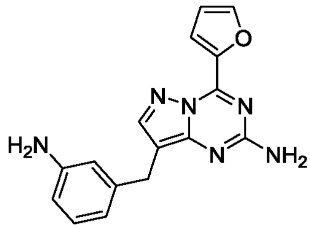 |
| 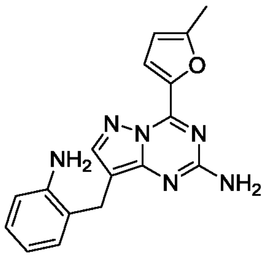 | 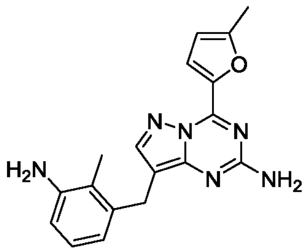 | 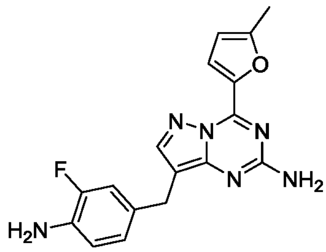 |
| 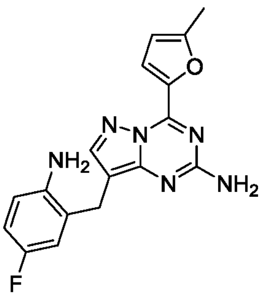 | 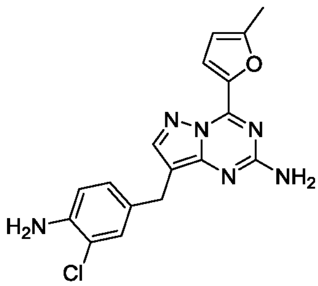 | 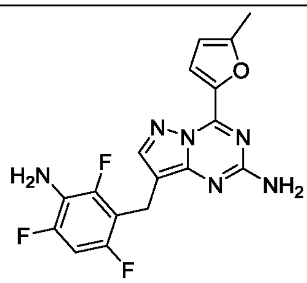 |
| 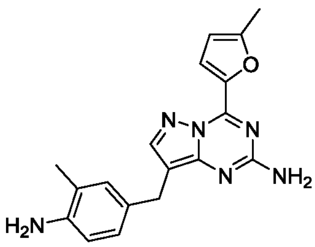 | 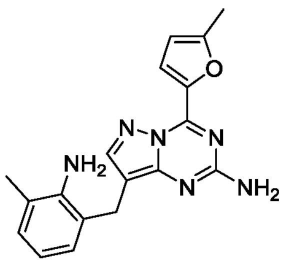 | 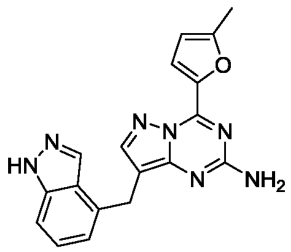 |

| | | |
|---|---|---|
| 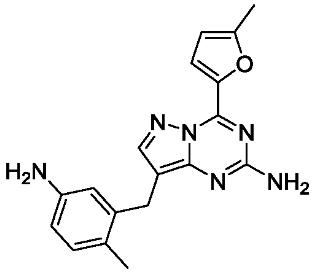 | 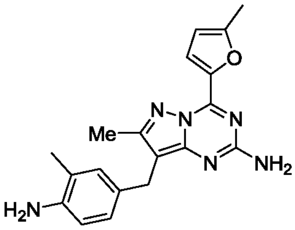 | 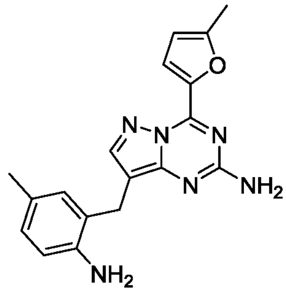 |
| 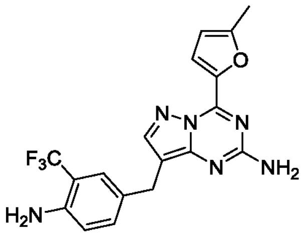 | 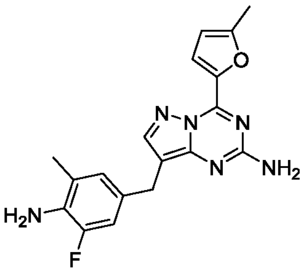 | 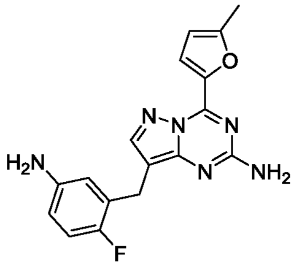 |
| 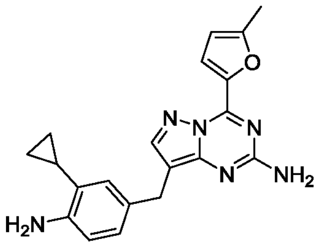 | 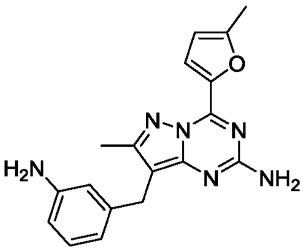 | 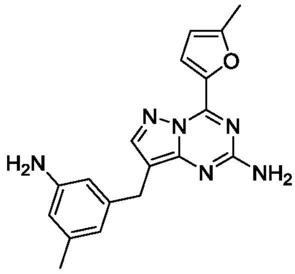 |
| 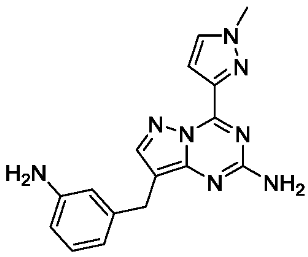 | 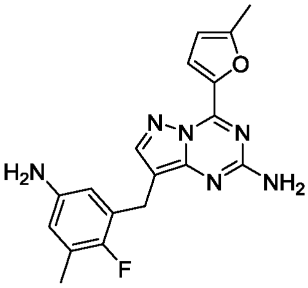 | 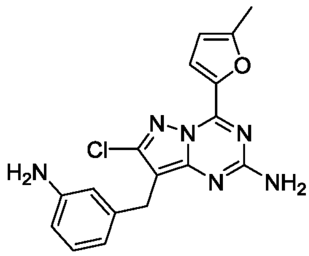 |
| 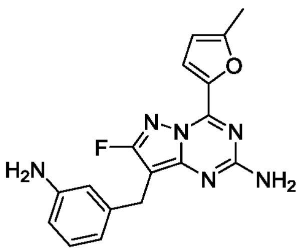 | 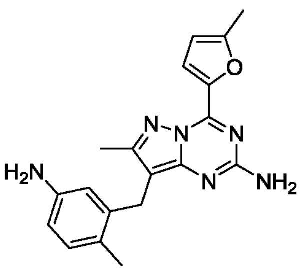 | 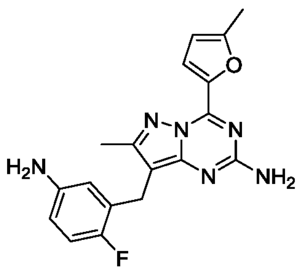 |
| 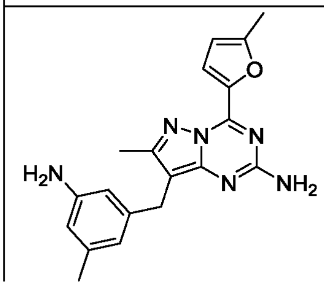 | 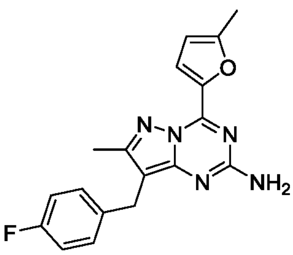 | 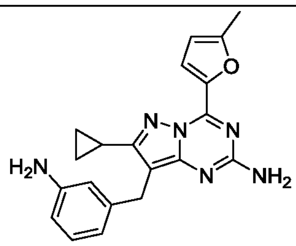 |

| | | |
|---|---|---|
| 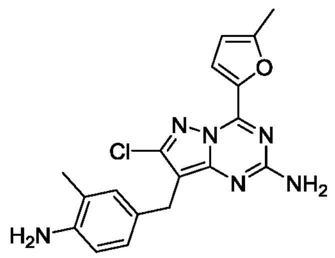 | 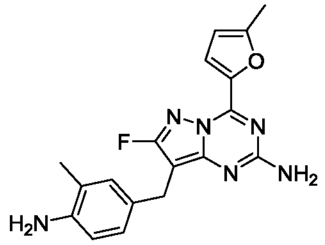 | 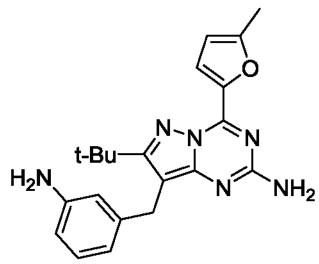 |
| 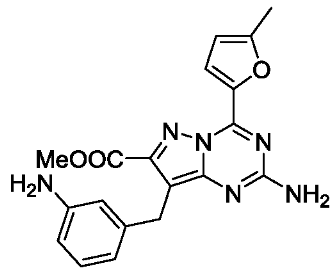 | 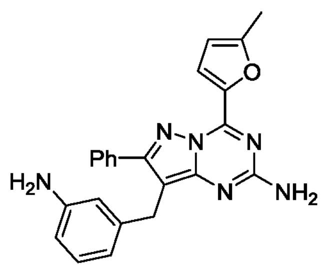 | 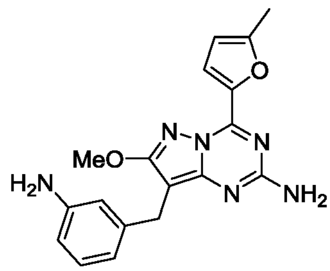 |
| 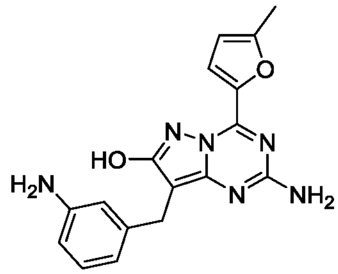 | 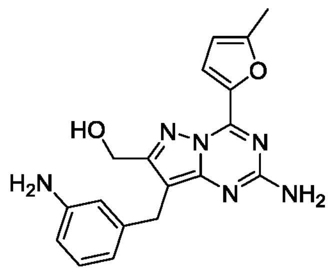 | 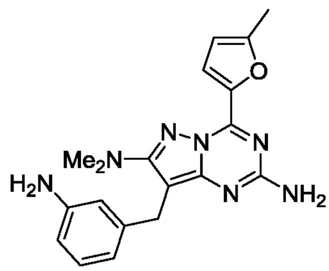 |

The compounds of the present invention may also be prepared in the form of pharmaceutically acceptable salts which may be formed with inorganic or organic acids such as hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methanesulfonic acid, benzenesulfonic acid or toluenesulfonic acid.

The pharmaceutically acceptable salts of the present invention may be prepared by a conventional method, for example, by dissolving the compound of the present invention in a water-miscible organic solvent (e.g., acetone, methanol, ethanol, and acetonitrile), adding an excess of an aqueous organic or inorganic acid thereto to precipitate the salt from the resulting mixture, removing the solvent and remaining free acid therefrom, and then isolating the precipitated salt.

The compounds of the present invention (or pharmaceutically acceptable salts thereof) may include solvate forms, preferably, the solvates are hydrates.

The present invention also provides use of the compound of the present invention in the manufacture of a medicament for the prevention and treatment of a disease which may be regulated by the inhibition of the activity of an adenosine receptor. Preferably, the disease is selected from the group consisting of cancer, tumors, inflammatory diseases, autoimmune diseases, and immune-mediated diseases.

Furthermore, the present invention provides a pharmaceutical composition for the prevention or treatment of cancer, tumors, inflammatory diseases, autoimmune diseases, neurodegenerative diseases, attention-related diseases, or immune-mediated diseases, including the compound Formula (I) of the present invention as an active ingredient.

Furthermore, the present invention provides a method of inhibiting an adenosine receptor comprising exposing the adenosine receptor to a compound of the present invention. Preferably, the present invention provides a method of inhibiting the A2A and/or A2B receptor comprising exposing the A2A and/or A2B receptor to a compound of the present invention. Furthermore, the present invention provides a method for the prevention or treatment of cancer, tumors, inflammatory diseases, autoimmune diseases, neurodegenerative diseases, attention-related diseases or immune-mediated diseases, including administering to a mammal in need thereof a compound of the present invention.

Representative examples of cancers or tumors may include, but are not limited to, skin cancer, bladder cancer, ovarian cancer, breast cancer, gastric cancer, pancreatic cancer, prostate cancer, colon cancer, lung cancer, bone

cancer, brain cancer, neurocytoma, rectal cancer, colon cancer, familial adenomatous polyposis cancer, hereditary nonpolyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid cancer, renal cancer, carcinoma of renal parenchyma, ovarian cancer, cervical cancer, corpus carcinoma, endometrial cancer, choriocarcinoma, pancreatic cancer, prostate cancer, testicular cancer, carcinoma of urinary system, melanoma, brain tumors such as glioblastoma, astrocytoma, meningioma, medulloblastoma and peripheral neuroectodermal tumors, Hodgkin's lymphoma, non-Hodgkin's lymphoma, Burkitt's lymphoma, acute lymphoblastic leukemia (ALL), chronic lymphocytic leukemia (CLL), acute myelogenous leukemia (AML), chronic myelogenous leukemia (CML), adult T-cell leukemia lymphoma, diffuse large B-cell lymphoma (DLBCL), hepatocellular carcinoma, gallbladder carcinoma, bronchial carcinoma, small cell lung carcinoma, non-small cell lung carcinoma, multiple myeloma, basal cell tumor, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing's sarcoma, or plasmacytoma.

When a compound of the present invention or a pharmaceutically acceptable salt thereof is administered in combination with another anticancer agent or a checkpoint inhibitor for the treatment of cancer or tumors, the compound of the present invention or a pharmaceutically acceptable salt thereof may provide an enhanced anticancer effect.

Representative examples of anticancer agent for the treatment of a cancer or tumor may include, but are not limited to, cell signal transduction inhibitors, Chlorambucil, Melphalan, Cyclophosphamide, Ifosfamide, Busulfan, Carmustine, Lomustine, Streptozotocin, Cisplatin, Carboplatin, Oxaliplatin, Dacarbazine, Temozolomide, Procarbazine, Methotrexate, Fluorouracil, Cytarabine, Gemcitabine, Mercaptopurine, Fludarabine, Vinblastine, Vincristine, Vinorelbine, Paclitaxel, Docetaxel, Topotecan, Irinotecan, Etoposide, Trabectedin, Dactinomycin, Doxorubicin, Epirubicin, Daunorubicin, Mitoxantrone, Bleomycin, Mitomycin C, Ixabepilone, Tamoxifen, Flutamide, Gonadorelin Analogs, Megestrol, Prednisone, Dexamethasone, Methylprednisolone, Thalidomide, Interferon $\alpha$, Calcium Folinate, Sirolimus, Sirolimus Lipide, Everolimus, Afatinib, Alisertib, Amuvatinib, Apatinib, Axitinib, Bortezomib, Bosutinib, Brivanib, Cabozantinib, Cediranib, Crenolanib, Crizotinib, Dabrafenib, Dacomitinib, Danusertib, Dasatinib, Dovitinib, Erlotinib, Foretinib, Ganetespib, Gefitinib, Ibrutinib, Icotinib, Imatinib, Iniparib, Lapatinib, Lenvatinib, Linifanib, Linsitinib, Masitinib, Momelotinib, Motesanib, Neratinib Nilotinib, Niraparib, Oprozomib, Olaparib, Pazopanib, Pictiliisib, Ponatinib, Quizartinib, Regorafenib, Rigosertib, Rucaparib, Ruxolitinib, Saracatinib, Saridegib, Sorafenib, Sunitinib, Telatinib, Tivantinib, Tivozanib, Tofacitinib, Trametinib, Vandetanib, Veliparib, Vemurafenib, Erivedge, Volasertib, Alemtuzumab, Bevacizumab, Brentuximab Vedotin, Catumaxomab, Cetuximab, Denosumab, Gemtuzumab, Ipilimumab, Nimotuzumab, Ofatumumab, Panitumumab, Rituximab, Tositumomab, and Trastuzumab; the checkpoint inhibitor includes but not limited to anti-PD-1 antibodies, anti-PD-Ll antibodies, LAG3 antibodies, TIM-3 antibodies, and anti-CTLA-4 antibodies, or any combination thereof.

Representative examples of inflammatory diseases, autoimmune diseases, and immune-mediated diseases may include, but are not limited to, arthritis, rheumatoid arthritis, spondyloarthritis, gouty arthritis, osteoarthritis, juvenile arthritis, other arthritic conditions, lupus, systemic lupus erythematosus (SLE), skin-related diseases, psoriasis, eczema, dermatitis, allergic dermatitis, pain, lung disease, lung Inflammation, adult respiratory distress syndrome (ARDS), pulmonary sarcoidosis, chronic pulmonary inflammatory disease, chronic obstructive pulmonary disease (COPD), cardiovascular disease, atherosclerosis, myocardial infarction, congestive heart failure, myocardial ischemia-reperfusion injury, inflammatory bowel disease, Crohn's disease, ulcerative colitis, irritable bowel syndrome, asthma, Sjogren's syndrome, autoimmune thyroid disease, urticaria (rubella), multiple sclerosis, scleroderma, organ transplant rejection, xenotransplantation, idiopathic thrombocytopenic purpura (ITP), Parkinson's disease, Alzheimer's disease, diabetes-related diseases, inflammation, pelvic inflammatory diseases, allergic rhinitis, allergic bronchitis, allergic sinusitis, leukemia, lymphoma, B-cell lymphoma, T-cell lymphoma, myeloma, acute lymphocytic leukemia (ALL), chronic lymphocytic leukemia (CLL), acute myelogenous leukemia (AML), chronic myelogenous leukemia (CML), hairy cell leukemia, Hodgkin's disease, non-Hodgkin's lymphoma, multiple myeloma, myelodysplastic syndrome (MDS), myeloproliferative tumor (MPN), diffuse large B-cell lymphoma, and follicular lymphoma. When a compound of the present invention or a pharmaceutically acceptable salt thereof is administered in combination with another therapeutic agent for the treatment of inflammatory diseases, autoimmune diseases and immune-mediated diseases, the compound of the present invention or a pharmaceutically acceptable salt thereof may provide an enhanced therapeutic effect.

Representative examples of therapeutic agents for the treatment of inflammatory diseases, autoimmune diseases, and immune-mediated diseases may include, but are not limited to, steroidal drugs (e.g., prednisone, prednisolone, methylprednisolone, cortisone, hydroxycortisone, betamethasone, dexamethasone, etc.), methotrexate, leflunomide, anti-TNF $\alpha$ agents (e.g., etanercept, infliximab, adalimumab, etc.), calcineurin inhibitors (e.g., tacrolimus, pimecrolimus, etc.), and antihistamines (e.g., diphenhydramine, hydroxyzine, loratadine, ebastine, ketotifen, cetirizine, levocetirizine, fexofenadine, etc.), and at least one therapeutic agent selected therefrom may be included in the pharmaceutical compositions of the present invention.

The compound of the present invention or a pharmaceutically acceptable salt thereof can be administered orally or parenterally as an active ingredient in an effective amount ranging from 0.1 mg/kg body weight/day to 2,000 mg/kg body weight/day, preferably 1 mg/kg body weight/day to 1,000 mg/kg body weight/day in the case of mammals

including humans (body weight about 70 kg), and administered in a single or four divided doses per day, or following/not following a predetermined time. The dosage of the active ingredient may be adjusted according to several relevant factors, such as the condition of the subject to be treated, the type and severity of the disease, the rate of administration, and the opinion of the physician). In some cases, amounts less than the above doses may be suitable. If it does not cause harmful side effects, an amount larger than the above dose can be used and the amount can be administered in divided doses per day. The pharmaceutical compositions of the present invention may be formulated into dosage forms, such as tablets, granules, powders, capsules, syrups, emulsions, microemulsions, solutions, or suspensions, for oral or parenteral administration (including intramuscular, intravenous, and subcutaneous routes) according to any of the conventional methods.

The pharmaceutical compositions of the present invention for oral administration may be prepared by mixing the active ingredients with carriers such as cellulose, calcium silicate, corn starch, lactose, sucrose, dextrose, calcium phosphate, stearic acid, magnesium stearate, calcium stearate, gelatin, talc, surfactants, suspending agents, emulsifying agents, and diluents. Examples of carriers employed in the injectable compositions of the present invention consist of water, saline solutions, dextrose solutions, glucose-like solutions, alcohols, glycols, ethers (e.g., polyethylene glycol 400), oils, fatty acids, fatty acid esters, glycerides, surfactants, suspending agents, and emulsifying agents.

Additional features of the present invention will become apparent from the description of exemplary embodiments of the present invention which are presented for purposes of illustration and are not intended to be limiting thereof, the following examples being prepared, isolated, and characterized using the methods disclosed herein.

The compounds of the present invention may be prepared in a variety of ways known to those skilled in the art of organic synthesis and may be synthesized using the synthetic methods known in the art of organic synthetic chemistry, or by variations thereof known to those skilled in the art. The required reaction may be carried out in a solvent or solvent mixture suitable for the kit materials used and for the transformations achieved.

## Detailed Description of the Invention

After long-term and intensive research, the inventors have unexpectedly found a class of heterocyclic compounds as shown in Formula (I) having adenosine receptor inhibitory activity, particularly A2A and/or A2B inhibitory activity. Based on the above findings, the inventors have completed the present invention.

## Terms

Terms used in the present application, including the specification and claims, are defined as follows, unless otherwise indicated. It must be noted that, in the description and the appended claims, the nouns without a numerical modification typically include plural referents unless the context clearly dictates otherwise. If not stated otherwise, conventional methods of mass spectrometry, nuclear magnetic, HPLC, protein chemistry, biochemistry, recombinant DNA techniques, and pharmacology are used.

Throughout the specification and claims, a given chemical formula or name shall encompass all stereo and optical isomers and racemates in which such isomers exist. Unless otherwise indicated, all chiral (enantiomer and diastereoisomer) and racemic forms are within the scope of the present invention. Many geometric isomers of C = C double bonds, C = N double bonds, and ring systems may also be present in the compounds, and all the above-mentioned stable isomers are encompassed in the present invention. Cis- and trans-(or E- and Z-) geometric isomers of the compounds of the present invention are described herein and may be isolated as mixtures of isomers or as separated isomeric forms. The compounds of the present invention may be isolated in optically active or racemic forms. All methods for preparing the compounds of the present invention and intermediates prepared therein are considered part of the present invention. In preparing enantiomeric or diastereomeric products, they can be isolated by conventional methods, for example, by chromatography or fractional crystallization. Depending on the process conditions, the final products of the present invention are obtained in free (neutral) or salt form. Both the free forms and salts of these end products are within the scope of the present invention. If desired, one form of the compound may be converted to another form. The free base or acid may be converted to its salt form; the salt may be converted to the free compound or another salt; mixtures of isomeric compounds of the present invention may be isolated into the individual isomers. The compounds, free forms, and salts thereof of the present invention may exist in a variety of tautomeric forms in which hydrogen atoms are transposed onto other parts of the molecule and the chemical bonds between the atoms of the molecule are thus rearranged. It is to be understood that all tautomeric forms which may exist are included in the present invention.

Unless otherwise defined, when a substituent is labeled "optionally substituted", the substituent is selected from, for example, the following substituents consisting of alkyl, cycloalkyl, aryl, heterocyclyl, halogen, hydroxy, alkoxy, oxo, alkanoyl, aryloxy, alkanoyloxy, amino, alkylamino, arylamino, arylalkylamino, disubstituted amine group (in which two amino substituents are selected from alkyl, aryl or arylalkyl), alkanoylamino, aroylamino, aralkanoylamino, substituted alkanoylamino, substituted arylamino, substituted aralkanoylamino, thio, alkylthio, arylthio, arylalkylthio, arylthiocarbonyl,

arylalkylthiocarbonyl, alkylsulfonyl, arylsulfonyl, arylalkylsulfonyl, sulfonamido such as $-SO_2NH_2$, substituted sulfonamido, nitro, cyano, carboxy, carbamoyl such as $-CONH_2$, substituted carbamoyl such as -CONH alkyl, -CONH aryl, -CONH arylalkyl or the case where there are two substituents selected from alkyl, aryl or arylalkyl on the nitrogen, alkoxycarbonyl, aryl, substituted aryl, guanidino, heterocyclyl such as indolyl, imidazolyl, furanyl, thienyl, thiazolyl, pyrrolidinyl, pyridyl, pyrimidinyl, pyrrolidinyl, piperidinyl, morpholinyl, piperazinyl, and homopiperazinyl, and substituted heterocyclyl.

As used herein, the term "alkyl" or "alkylene" is intended to include both branched- and straight-chain saturated aliphatic hydrocarbon groups having the specified number of carbon atoms. For example, "$C_1$-$C_6$ alkyl" denotes an alkyl group having 1 to 6 carbon atoms. Examples of alkyl groups include, but are not limited to, methyl (Me), ethyl (Et), propyl (e.g., n-propyl and isopropyl), butyl (e.g., n-butyl, isobutyl, tert-butyl), and pentyl (e.g., n-pentyl, isopentyl, neopentyl). Preferred alkyl groups are $C_1$-$C_6$ alkyl groups, and more preferred alkyl groups are $C_1$-$C_4$ alkyl groups.

The term "alkenyl" denotes a straight or branched chain hydrocarbon group containing one or more double bonds and typically 2 to 20 carbon atoms in length. For example, "$C_2$-$C_8$ alkenyl" contains 2 to 8 carbon atoms. Alkenyl groups include, but are not limited to, for example, ethenyl, propenyl, butenyl, 1-methyl-2-buten-1-yl, heptenyl, and octenyl. Preferred alkenyl groups are $C_2$-$C_8$ alkenyl groups, and more preferred alkenyl groups are $C_2$-$C_6$ alkenyl groups.

The term "alkynyl" denotes a straight or branched chain hydrocarbon group containing one or more triple bonds and typically 2 to 20 carbon atoms in length. For example, "$C_2$-$C_8$ alkynyl" contains 2 to 8 carbon atoms. Preferred alkenyl groups are $C_2$-$C_8$ alkynyl groups, and more preferred alkenyl groups are $C_2$-$C_6$ alkynyl groups. Representative alkynyl groups include, but are not limited to, for example, ethynyl, 1-propynyl, 1-butynyl, heptynyl, and octynyl.

The term "alkoxy" or "alkyloxy" refers to -O-alkyl. "$C_1$-$C_6$ alkoxy" (or alkyloxy) is intended to include $C_1$, $C_2$, $C_3$, $C_4$, $C_5$, and $C_6$ alkoxy groups. Preferred alkoxy groups are $C_1$-$C_6$ alkoxy groups, more preferred alkoxy groups are $C_1$-$C_4$ alkoxy groups. Examples of alkoxy groups include, but are not limited to, methoxy, ethoxy, propoxy (e.g., n-propoxy and isopropoxy), and t-butoxy. Similarly, "alkylthio" or "thioalkoxy" means an alkyl group, as defined above, with the specified number of carbon atoms linked via a sulfur bridge; for example, methyl-S- and ethyl-S-.

The term "carbonyl" refers to an organic functional group (C=O) composed of two carbon and oxygen atoms linked by a double bond.

The term "aryl", alone or as part of a larger moiety such as "aralkyl", "aralkoxy", or "aryloxyalkyl", refers to a monocyclic, bicyclic, or tricyclic ring system having a total of 5 to 12 ring members, where at least one ring in the system is aromatic and where each ring in the system contains 3 to 7 ring members. Monocyclic aromatic refers to phenyl, bicyclic or polycyclic aromatic group refers to naphthyl, anthryl, etc., meanwhile, the aryl bicyclic ring may be formed by fusing a cycloalkyl group, or a cycloalkenyl group or a cycloalkynyl group on a benzene ring. Preferred aryl groups are $C_6$-$C_{12}$ aryl groups. In certain embodiments of the present invention, "aryl" refers to an aromatic ring system including, but not limited to, phenyl, biphenyl, indanyl, 1-naphthyl, 2-naphthyl, and tetrahydronaphthyl. The term "aralkyl" or "arylalkyl" refers to an alkyl residue attached to an aryl ring. Non-limiting examples include benzyl, and phenethyl. The fused aryl group may be attached to another group at a suitable position on the cycloalkyl ring or the aromatic ring. For example, an arrow line drawn from a ring system indicates that the bond may be attached to any suitable ring atom.

The term "cycloalkyl" refers to a monocyclic or bicyclic alkyl group. Monocyclic alkyl refers to $C_3$-$C_8$ cyclic alkyl including, but not limited to, cyclopropyl, cyclobutyl, cyclopentyl, cyclohexyl, and norbornyl. Branched cycloalkyl such as 1-methylcyclopropyl and 2-methylcyclopropyl are included in the definition of "cycloalkyl". Bicyclic alkyl includes bridged, spiro, or fused cycloalkyl . Preferred cycloalkyl groups are $C_3$-$C_6$ cycloalkyl groups.

The term "cycloalkenyl" refers to a monocyclic or bicyclic alkenyl group. Monocyclic alkenyl refers to $C_3$-$C_8$ cyclic alkenyl including, but not limited to, cyclopropenyl, cyclobutenyl, cyclopentenyl, cyclohexenyl, and norbornenyl. Branched cycloalkenyl such as 1-methylcyclopropenyl and 2-methylcyclopropenyl are included in the definition of "cycloalkenyl". Bicyclic alkenyl includes bridged, spiro or fused cyclic alkenyl.

"Halo" or "halogen" includes fluoro, chloro, bromo, and iodo. "Haloalkyl" is intended to include both branched and straight-chain saturated aliphatic hydrocarbon groups having the specified number of carbon atoms and substituted with one or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl, and heptachloropropyl. Examples of haloalkyl also include "fluoroalkyl" groups intended to include branched- and straight-chain saturated aliphatic hydrocarbon groups having the specified number of carbon atoms and substituted with one or more fluorine atoms.

"Haloalkoxy" or "haloalkyloxy" denotes a haloalkyl group, as defined above, having the indicated number of carbon atoms linked via an oxygen bridge. For example, "$C_1$-$C_6$ haloalkoxy" is intended to include $C_1$, $C_2$, $C_3$, $C_4$, $C_5$, and $C_6$ haloalkoxy groups. Examples of haloalkoxy include, but are not limited to, trifluoromethoxy, 2,2,2-trifluoroethoxy, and pentafluoroethoxy. Similarly, "haloalkylthio" or "thiohaloalkoxy" denotes a haloalkyl group, as defined above, having the indicated number of carbon atoms linked via a sulfur bridge; for example, trifluoromethyl-S- and pentafluoroethyl-S-.

The term "heteroaryl" means a stable 3-, 4-, 5-, 6-, or 7-membered aromatic monocyclic or aromatic bicyclic or 7-, 8-, 9-, 10-, 11-, 12-, 13-, or 14-membered aromatic polycyclic heterocycle, which is fully unsaturated, partially unsaturated, and contains carbon atoms and 1, 2, 3 or 4 heteroatoms independently selected from N, O and S; and

includes any polycyclic group in which any heterocycle defined above is fused to a benzene ring. The nitrogen and sulfur heteroatoms may optionally be oxidized. The nitrogen atom is substituted or unsubstituted (i.e., N or NR, where R is H or another substituent if defined). The heterocycle may be attached to its pendant group at any heteroatom or carbon atom that results in a stable structure. If the resulting compound is stable, the heterocyclyl groups described herein may be substituted on a carbon or nitrogen atom. The nitrogen in the heterocycle may be optionally quaternized. Preferably, when the total number of S and O atoms in the heterocycle exceeds 1, then these heteroatoms are not adjacent to each other. Preferably, the total number of S and O atoms in the heterocycle is not greater than 1. Preferred heteroaryl groups are 5-12-membered heteroaryl groups. Examples of heteroaryls include, but are not limited to, acridinyl, azetidinyl, azocinyl, benzimidazolyl, benzofuranyl, benzothiofuranyl, benzothienyl, benzoxazolyl, benzoxazolinyl, benzothiazolyl, benzotriazolyl, benzotetrazolyl, benzisoxazolyl, benzisothiazolyl, benzimidazolinyl, carbazolyl, 4aH-carbazolyl, carbolinyl, chromanyl, chromenyl, cinnolinyl, decahydroquinolinyl, 2H, 6H-1,5,2-dithiazinyl, dihydrofuro[2,3-b]tetrahydrofuranyl, furanyl, furazanyl, imidazolidinyl, imidazolinyl, imidazolyl, 1H-indazolyl, imidazopyridinyl, indolenyl, indolinyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridinyl, isoxazolyl, isoxazolopyridinyl, methylenedioxyphenyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinyl, perimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridooxazolyl, pyridoimidazolyl, pyridothiazolyl, pyridinyl, pyrimidinyl, pyrrolidinyl, pyrrolinyl, 2-pyrrolidonyl, 2H-pyrrolyl, pyrrolyl, quinazolinyl, quinolinyl, 4H-quinolizinyl, quinoxalinyl, quinuclidinyl, tetrazolyl, tetrahydrofuranyl, tetrahydroisoquinolinyl, tetrahydroquinolinyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridyl, thienothiazolyl, thienooxazolyl, thienoimidazolyl, thienyl, triazinyl, 1,2,3-triazolyl, 1,2,4-triazolyl, 1,2,5-triazolyl, 1,3,4-triazolyl and xanthenyl, quinolinyl, isoquinolinyl, phthalazinyl, quinazolinyl, indolyl, isoindolyl, indolinyl, 1H-indazolyl, benzimidazolyl, 1,2,3,4-tetrahydroquinolinyl, 1,2,3,4-tetrahydroisoquinolinyl, 5,6,7,8-tetrahydro-quinolinyl, 2,3-dihydro-benzofuranyl, chromanyl, 1,2,3,4-tetrahydro-quinoxalinyl and 1,2,3,4-tetrahydro-quinazolinyl. The present invention also includes fused and spiro compounds containing, for example, the above heterocycles.

As used herein, the term "heterocycloalkyl" refers to a monocyclic heterocycloalkyl system or a bicyclic heterocycloalkyl system, and also includes spiroheterocycles or bridged heterocycloalkyl groups. The monocyclic heterocycloalkyl refers to a saturated or unsaturated but not aromatic 3- to 8-membered cyclic alkyl system containing at least one atom selected from O, N, S, and P. The bicyclic heterocycloalkyl system refers to a heterocycloalkyl group fused with a phenyl, or a cycloalkyl, or a cycloalkenyl, or a heterocycloalkyl, or a heteroaryl group. Preferred heterocycloalkyl groups are 3-12-membered heterocycloalkyl groups.

As used herein, the term "substituted" means that at least one hydrogen atom is replaced with a non-hydrogen group, provided that normal valency is maintained and that the substitution results in a stable compound. As used herein, the ring double bond is a double bond (e.g., C = C, C = N, or N = N) formed between two adjacent ring atoms.

In the case where nitrogen atoms (e.g., amines) are present on the compounds of the present invention, these nitrogen atoms may be converted to N-oxides by treatment with an oxidizing agent (e.g., *m*-CPBA and/or hydrogen peroxide) to obtain other compounds of the present invention. Thus, the nitrogen atoms shown and claimed are considered to encompass both the nitrogen shown and its N-oxides to obtain the derivatives of the present invention.

When any variable occurs more than once in any composition or formula of a compound, its definition at each occurrence is independent of its definition at every other occurrence. Thus, for example, if a group is shown to be substituted with 0, 1, 2, or 3 R groups, the group may be optionally substituted with up to three R groups, and at each occurrence, R is independently selected from the definition of R. Furthermore, combinations of substituents and/or variables are permissible only if such combinations result in stable compounds.

The term "solvate" means a physical association of a compound of the present invention with one or more solvent molecules (whether organic or inorganic). The physical association includes hydrogen bonding. In some cases, for example, when one or more solvent molecules are incorporated in the crystal lattice of the crystalline solid, the solvate will be capable of being isolated. The solvent molecules in the solvate may be present in a regular and/or disordered arrangement. Solvates may include stoichiometric or non-stoichiometric solvent molecules. "Solvate" encompasses both solution-phase and isolatable solvates. Exemplary solvates include, but are not limited to, hydrates, ethanolates, methanolates, and isopropanolates. Methods of solvation are well known in the art.

As used herein, the term "patient" refers to an organism treated by the methods of the present invention. Such organisms preferably include but are not limited to, mammals (e.g., murine, ape/monkey, equine, bovine, swine, canine, feline, etc.) and most preferably refer to humans.

As used herein, the term "effective amount" means an amount of a drug or pharmaceutical agent (i.e., a compound of the present invention) that will elicit the biological or medical response of a tissue, system, animal, or human that is being sought, for example, by a researcher or clinician. Furthermore, the term "therapeutically effective

amount" means an amount that results in improved treatment, cure, prevention or alleviation of a disease, condition, or side effect, or a reduction in the rate of progression of a disease or condition, as compared to a corresponding subject not receiving such an amount. An effective amount can be administered in one or more dosing, administrations, or dosages and is not intended to be limited by the particular formulation or route of administration. The term also includes an amount effective that enhances normal physiological function within its scope.

As used herein, the term "treating" includes any effect that results in amelioration of a condition, disease, or disorder, for example, alleviation, reduction, modulation, amelioration or elimination, or amelioration of a symptom thereof. As used herein, a compound or pharmaceutical composition, upon administration, may result in amelioration of a disease, symptom, or condition, particularly amelioration of the severity, delay of the onset, alleviation of the progression, or reduction of the duration of the condition. Regardless of fixed administration or temporary administration, continuous administration, or intermittent administration, it may be attributed to or related to the administration. The terms "prevent", "preventing" and "prevention" as used herein refer to preventing, blocking, eliminating the onset of disease, or interfering with or slowing the progression of a disease before the onset of a disease or condition.

The term "pharmaceutical composition" as used herein refers to the combination of an active agent with an inert or active carrier, making the composition particularly suitable for in vivo or ex vivo diagnosis or treatment. Examples of bases include, but are not limited to, alkali metal (e. g., sodium) hydroxides, alkaline earth metal (e. g., magnesium) hydroxides, ammonia, etc. Salts of the compounds of the present invention are expected to be pharmaceutically acceptable for therapeutic use. However, non-pharmaceutically acceptable salts of acids and bases may also be used, for example, in the preparation or purification of pharmaceutically acceptable compounds. The term "pharmaceutically acceptable" is used herein to refer to those compounds, materials, compositions, and/or dosage forms as follows: within the scope of sound medical judgment, suitable for use in contact with the tissues of humans and animals without undue toxicity, irritation, allergic response, and/or other problems or complications, commensurate with a reasonable benefit/risk ratio.

As used herein, the phrase "pharmaceutically acceptable carrier" or "medicinal carrier" refers to a pharmaceutical material, composition, or a vehicle, such as a liquid or solid filler, diluent, excipient, manufacturing adjuvant (e.g., lubricant, talc, magnesium stearate, calcium stearate or zinc stearate or stearic acid), or solvent encapsulating material, which refers to carrying or transporting the active compound from one organ or portion of the body to another organ or portion of the body. Each carrier must be "acceptable" in the sense of being compatible with the other ingredients of the formulation and not deleterious to the patient.

**Specific pharmaceutical and medical terms**

The term "acceptable", as used herein, refers to a prescription component or active ingredient that does not unduly adversely affect the health of the general therapeutic target.

The term "cancer", as used herein, refers to uncontrolled abnormal growth of cells and is capable of metastasis (transmission) under certain conditions. This type of cancer includes, but is not limited to, solid tumors (e.g., bladder, bowel, brain, chest, uterus, heart, kidney, lung, lymphoid tissue (lymphoma), ovary, pancreas, or other endocrine organs (e.g., thyroid), prostate, skin (melanoma), or hematological tumors (e.g., aleukemic leukemia).

The term "administered in combination" or similar terms, as used herein, refers to the administration of several selected therapeutic agents to a patient in the same or different modes of administration at the same or different times.

The term "enhance" or "potentiate", as used herein, means that the desired result can be increased or prolonged in potency or duration. Thus, in enhancing the therapeutic effect of a drug, the term "enhance" refers to the ability of the drug to increase or prolong potency or duration in the system.

"Synergistic value", as used herein, refers to the ability to maximize the ability of another therapeutic agent in an ideal system.

The term "immunological disease" refers to a disease or condition that responds adversely or deleteriously to endogenous or exogenous antigens. The result is often a dysfunction of the cells, or thus destruction and dysfunction, or destruction of organs or tissues that may produce immune symptoms.

The term "kit" is synonymous with "product package".

The term "subject" or "object" includes mammals and non-mammals. Mammals include, but are not limited to, mammals: human, non-human primates such as chimpanzees, apes, and monkeys; agricultural animals such as bovines, equines, goats, sheep, swine; domestic animals such as rabbits, canines; experimental animals include rodents, such as rats, mice, and guinea pigs. Non-mammalian animals include, but are not limited to, birds, and fish. In a preferred embodiment, the selected mammal is a human.

**Route of administration**

Suitable routes of administration include but are not limited to, oral, intravenous, rectal, aerosol, parenteral,

ophthalmic, pulmonary, transdermal, vaginal, aural, nasal, and topical administration. In addition, by way of example only, parenteral administration includes intramuscular, subcutaneous, intravenous, intramedullary, ventricular, intraperitoneal, intralymphatic, and intranasal injections.

In one aspect, the compounds described herein are administered locally rather than systemically. In particular embodiments, the
prolonged action preparation is administered by implantation (e.g., subcutaneously or intramuscularly) or by intramuscular injection. Further, in another embodiment, the drug is administered by a targeted drug delivery system, for example, liposomes encapsulated by organ-specific antibodies. In this particular embodiment, the liposomes are selectively targeted to specific organs and absorbed.

**Pharmaceutical compositions and dosages**

The present invention also provides pharmaceutical compositions including a therapeutically effective amount of one or more compounds of the present invention formulated together with one or more pharmaceutically acceptable carriers (additives) and/or diluents, and optionally one or more of the other therapeutic agents described above. The compounds of the present invention may be administered for any of the above-mentioned uses by any suitable means, for example by orally, such as in the form of tablets, pills, powders, granules, elixirs, tinctures, suspensions (including nanosuspensions, micro suspensions, spray-dried dispersions), syrups and emulsions; by sublingually; by buccally; by parenterally, such as by subcutaneous, intravenous, intramuscular or intrasternal injection or infusion techniques (e.g., in the form of sterile injectable aqueous or nonaqueous solutions or suspensions); by nasally, including administration to the nasal mask, such as by inhalation spray; by topically, such as in the form of a cream or ointment; or by rectally, such as in the form of suppositories. They may be administered alone, but are generally administered using pharmaceutically acceptable carriers selected based on the chosen route of administration and standard pharmaceutical practice.

The pharmaceutically acceptable carriers are formulated according to several factors within the knowledge of those skilled in the art. These factors include but are not limited to: types and properties of the formulated active agents; a subject to be administered the composition containing the active agent; the intended route of administration of the composition; and targeted therapeutic indications. The pharmaceutically acceptable carriers include aqueous and non-aqueous liquid media and various solid and semi-solid dosage forms.

The above-mentioned carrier may include many different ingredients and additives in addition to the active agent, and the above-mentioned other ingredients, for example, stabilizing active agent and binder, are included in the formulation for various reasons known to those skilled in the art. For a description of suitable pharmaceutically acceptable carriers and factors involved in the selection of carrier, see several readily available sources, such as Allen L.V.Jr. et al. Remington: The Science and Practice of Pharmacy (2 Volumes), 22nd Edition (2012), Pharmaceutical Press. The dosage regimen for the compounds of the present invention will, of course, vary depending upon known factors such as the pharmacodynamic characteristics of the particular agent and its mode and route of administration; species, age, sex, health, medical condition, and weight of the recipient; the nature and extent of symptoms; kind of concurrent treatment; treatment frequency; routes of administration, renal and hepatic function and desired effects in patients. According to general guidelines, when used for a given effect, the daily oral dosage of each active ingredient should be from about 0.001 mg/day to about 10-5000 mg/day, preferably from about 0.01 mg/day to about 1000 mg/day, and most preferably from about 0.1 mg/day to about 250 mg/day. During constant infusion, the most preferred intravenous dose should be from about 0.01 mg/kg/min to about 10 mg/kg/min. The compounds of the present invention may be administered in a single daily dose, or the total daily dose may be administered in divided doses of two, three, or four times daily.

The compounds are generally administered in the form of a mixture of suitable pharmaceutical diluents, excipients, or carriers (collectively referred to herein as pharmaceutically acceptable carriers) suitably selected concerning the intended form of administration (e.g., oral tablets, capsules, elixirs, and syrups) and consistent with conventional pharmaceutical practice.

Dosage forms (pharmaceutical compositions) suitable for administration may contain from about 1 mg to about 2000 mg of active ingredient per dosage unit. In these pharmaceutical compositions, the active ingredient will generally be present in an amount of about 0.1-95% by weight, based on a total weight of the composition.

Typical capsules for oral administration contain at least one compound of the present invention (250 mg), lactose (75 mg), and magnesium stearate (15 mg). The mixture was processed through a 60 meshes screen and packaged into No.1 gelatin capsules.

A typical injectable formulation may be prepared as follows: at least one compound of the present invention (250 mg) was placed in a vial in a sterile manner, and lyophilized and sealed in a sterile manner. For use, the contents in the vial were mixed with 2 mL of normal saline to produce an injectable formulation.

The scope of the present invention includes (alone or in combination with a pharmaceutically acceptable carrier) pharmaceutical compositions containing a therapeutically effective amount of at least one compound of the present invention as an active ingredient. Optionally, the compounds of the present invention may be used alone, in combination

with other compounds of the present invention, or in combination with one or more other therapeutic agents (e.g., anticancer agents or other pharmaceutically active agents).

Regardless of the selected route of administration, the compounds of the present invention (which may be used in suitable hydrated forms) and/or the pharmaceutical compositions of the present invention are formulated into pharmaceutically acceptable dosage forms by conventional methods known to those skilled in the art.

The actual dosage level of the active ingredient in the pharmaceutical compositions of the present invention may be varied to obtain an amount of the active ingredient that is effective to achieve the desired therapeutic response, composition, and mode of administration for a particular patient without being toxic to the patient.

The selected dosage level will depend upon a variety of factors, including the factors well known in the medical field such as the activity of the employed specific compound of the present invention, or an ester, salt or amide thereof; routes of administration; administration time; the discharge rate of the employed specific compound; the absorption rate and extent; duration of treatment; other drugs, compounds and/or substances used in combination with the employed specific compounds; the age, sex, weight, condition, general health and prior medical history of the patient being treated.

A physician or veterinarian having ordinary skill in the art can readily determine and prescribe an effective amount of the desired pharmaceutical composition. For example, to achieve the desired therapeutic effect, the physician or veterinarian may start a relatively small amount of the compound of the present invention used in the pharmaceutical composition below the desired level and gradually increase the dosage until the desired effect is achieved. In general, a suitable daily dose of a compound of the present invention will be that amount of the compound that is the lowest dose effective to produce a therapeutic effect. Such an effective dose will generally depend on such factors. In general, oral, intravenous, intracerebroventricular, and subcutaneous doses of a compound of the present invention for a patient range from about 0.01 to about 50 mg/kg body weight/day. If desired, an effective daily dose of the active compound may be administered in two, three, four, five, six, or more sub-doses respectively at appropriate intervals throughout the day, optionally in a unit dosage form. In certain aspects of the present invention, the medication is administered once a day.

Although the compound of the present invention may be administered alone, it is preferably administered in the form of a pharmaceutical preparation (composition).

**Kit/Product package**

Kits/product packages are also described herein for the treatment of the above-mentioned indications. These kits may be composed of a conveyor, a medicine pack, or a container box. The container box can be divided into multiple compartments to accommodate one or more containers, such as vials, and test tubes, where each container contains a single component in the method. Suitable containers consist of bottles, vials, syringes, and test tubes. The container is made of an acceptable glass or plastic material.

For example, the container may contain one or more of the compounds described herein; the compound may exist either in the form of a pharmaceutical composition or may exist as a mixture with other ingredients described herein. The container may have a sterile outlet (e.g., the container may be an intravenous infusion bag or bottle and the stopper may be pierced by a hypodermic needle). Such kits may contain a compound and descriptions, labels, or instructions for the method of use described herein.

A typical kit may include one or more containers, each containing one or more materials (e.g., reagents, concentrated stock solutions, and/or equipment) to accommodate commercial promotions and the needs of the user for the use of compounds. Such materials include, but are not limited to, buffers, diluents, filters, needles, syringes, conveyors, bags, containers, bottles, and/or tubes, with a list of contents and/or instructions for use, and with a built-in package. The entire set of instructions must be included.

The label may be displayed on or closely related to the container. The appearance of the label on the container means that the label letters, numbers, or other features are pasted, molded, or engraved on the container; the label can also appear in the container box or shipping box containing a variety of containers, such as in the product insert. A label may be used to indicate a particular therapeutic use of the contents. The label may also indicate directions for the use of contents, such as described in the methods described above.

All of the features described in this specification (including any accompanying claims, abstract, and drawings), and/or all of the steps involved in any method or process, may be present in any combination unless some features or steps are mutually exclusive in the same combination.

The features mentioned above, or the features mentioned in the embodiments mentioned herein, may be combined in any combination. All of the features disclosed in this specification may be combined in any combination, and each feature disclosed in this specification may be replaced by any alternative feature serving the same, equivalent, or similar purpose. Thus, unless otherwise specified, the features disclosed are only general examples of equivalent or similar features.

The present invention will be described in detail below in connection with specific examples. It should be understood that these examples are only used to describe the present invention and are not intended to limit the scope

of the present invention. The experimental methods in the following examples which are not specified with specific conditions are generally carried out according to conventional conditions or according to the conditions recommended by the manufacturer. All percentages, ratios, ratios, or parts are calculated by weight unless otherwise stated.

The units in weight-volume percent in the present invention are well known to those skilled in the art and refer, for example, to the weight of solute in a 100 milliliters of solution. Unless otherwise defined, all professional and scientific terms used in the text have the same meaning as those familiar to those skilled in the art. In addition, any methods and materials similar or equivalent to those described can be used in the methods of the present invention. The preferred embodiments and materials described herein are exemplary only.

In preferred embodiments of the present invention, the following compounds are provided, but are not limited to: The present invention will be described in detail below in connection with specific examples. It should be understood that these examples are only used to describe the present invention and are not intended to limit the scope of the present invention. The experimental methods in the following examples which are not specified with specific conditions are generally carried out according to conventional conditions or according to the conditions recommended by the manufacturer. All percentages and parts are calculated by weight unless otherwise stated.

**Specific Examples**

When no preparative route is described, related intermediates are commercially available (e.g. from Sigma Aldrich, Alfa).

**General Procedure**

Commercial reagents were used without further purification. Room temperature refers to 20-27°C. 1 H-NMR spectra were recorded on a Bruker instrument at 500 MHz. Chemical shift values are expressed in parts per million, i.e. δ value. The following abbreviations are used for the multiplicity of NMR signals: s = singlet, brs = broad, d = doublet, t = triplet, m = multiplet. Coupling constants were listed as J values, measured in Hz. NMR and mass spectrum results were corrected for background peaks. Chromatography refers to column chromatography performed using 100 meshes silica gel and completed under nitrogen pressure (flash chromatography). TLC used to monitor the reaction refers to TLC performed using a specific mobile phase and silica gel $F_{254}$ from Merck as stationary phase.

**LC-MS measurements were performed under the following conditions:**

Instruments: Thermo U3000, ALLtech ELSD, MSQ, UV detector combined ELSD and MSD (flow ratio of 4:1). Column: Waters X-Bridge C-18, 3.5μm, 4.6x50mm; Column temperature: 30°C. Gradient [Time (min)/solvent B in A (%)]: 0.00/5.0, 0.70/95, 1.40/95, 1.41/5, 1.50/5. (Solvent A = 0.01% trifluoroacetic acid (TFA) in water; solvent B = 0.01% trifluoroacetic acid in acetonitrile). UV Detection: 214/254/280/300 nm; DAD detection: 200-400nm; Flow Rate: 4 mL/min; MS: ESI, 100-1500 m/z.

Preparative HPLC typically used acidic process (gradient of acetonitrile and water, each containing 0.1% formic acid) with a Thermo U3000 AFC-3000; Column: Globalsil C-18 C-18 12 nm, 250 × 20 mm, 10μm, or equivalent; Flow Rate: 20 mL/min for separation.

**Synthesis of intermediates:**

Preparation of compound **INT-1**

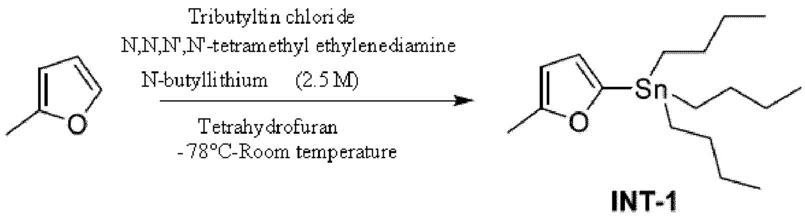


**INT-1**

2-Methylfuran (3.28 g, 40.0 mmol) and *N,N,N',N'*-tetramethyl ethylenediamine (17.0 g, 146 mmol) were dissolved in anhydrous tetrahydrofuran (80 mL). N-Butyllithium (2.5 M in n-hexane, 16 mL) was slowly added dropwise under a nitrogen atmosphere at -78°C, the resulting solution was agitated at a temperature ranging from -40°C to -20°C

for 3 hours, then tributyltin chloride (13.0 g, 40.0 mmol) was added at -78°C, and the resulting solution was slowly heated to room temperature and left standing overnight. The reaction solution was quenched with saturated ammonium chloride (60 mL) and extracted with ethyl acetate (100 mL × 3). The combined organic phase was washed with saturated saline solution (100 mL), dried over anhydrous sodium sulfate, and filtered, the filtrate passed through a mixed column of silica gel (100 g) and potassium fluoride (10 g), rinsed with ethyl acetate (300 mL), and the eluent was concentrated under reduced pressure to give the compound **INT-1** (12.1 g, yield 81%). $^1$H NMR (500 MHz, Chloroform-*d*) $\delta$ 6.44 (d, *J* = 3.0 Hz, 1H), 6.05 - 5.94 (m, 1H), 2.33 (s, 3H), 1.62 - 1.48 (m, 6H), 1.37 - 1.29 (m, 6H), 1.12 - 0.98 (m, 6H), 0.94 - 0.87 (m, 9H).

Preparation of compound INT-2

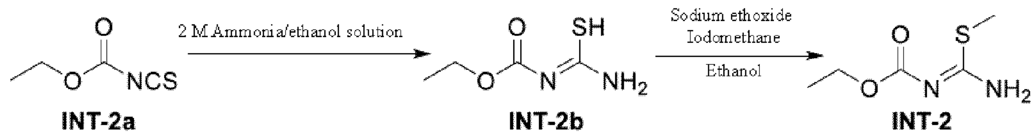

INT-2a INT-2b INT-2

To a solution of ammonia in ethanol (2 M, 7.50 mL) was added compound **INT-2a** (1.31 g, 10.0 mmol) in an ice bath. The resulting reaction solution was agitated for 1 hour in an ice bath. The reaction solution was concentrated under reduced pressure to give the compound **INT-2b** as a yellow solid, which was used in the next step without purification (1.31 g, yield 88%). $^1$H NMR (500 MHz, Chloroform-d) $\delta$ 9.20 (s, 1H), 8.29 (s, 1H), 7.11 (s, 1H), 4.25 (q, *J* = 7.1 Hz, 2H), 1.32 (t, *J* = 7.1 Hz, 3H).

To a solution of compound **INT-2b** (215 mg, 1.45 mmol) in ethanol (5 mL) were added iodomethane (721 mg, 5.08 mmol) and sodium ethoxide (109 mg, 1.60 mmol) in sequence in an ice bath. The resulting reaction solution was agitated for 1 hour at 25°C. The reaction solution was concentrated under reduced pressure, added into water (20 mL), and extracted with ethyl acetate (20 mL × 3). The combined organic phase was dried over anhydrous sodium sulfate and filtered, and the filtrate was concentrated under reduced pressure, and then was purified by column chromatography (PE/EA = 5/1) to give the compound **INT-2** as a yellow oil (200 mg, yield 85%). $^1$H NMR (500 MHz, Chloroform-*d*) $\delta$ 4.19 (q, *J* = 7.1 Hz, 2H), 2.50 (s, 3H), 1.33 (t, *J* = 7.1 Hz, 3H).

Preparation of compound **INT-3**

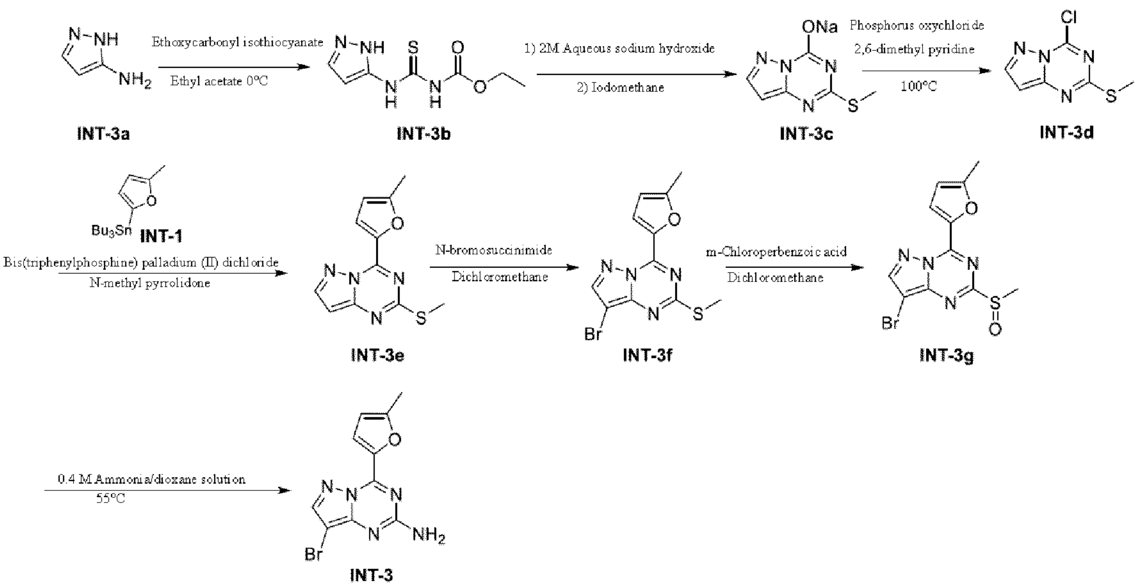

INT-3a INT-3b INT-3c INT-3d INT-1 INT-3e INT-3f INT-3g INT-3

To a solution of 5-aminopyrazole **INT-3a** (6.00 g, 72.2 mmol) in ethyl acetate (180 mL) was added ethoxycarbonyl isothiocyanate (9.47 g, 72.2 mmol) in an ice bath, and the resulting reaction solution was agitated for 2 hours in an ice bath. The reaction solution was concentrated under reduced pressure to give the compound **INT-3b** as an orange-yellow

solid (15.5 g, yield > 99% ), which was used in the next step without purification. MS: 179.1 $[M+H]^+$.

To a reaction vial containing crude compound **INT-3b** (15.5 g) was added aqueous sodium hydroxide (2 M, 144 mL), the mixture was agitated for 1 hour at 15°C, then ethanol (72 mL) and iodomethane (10.3 g, 72.2 mmol) were added, and the resulting reaction solution was agitated for 1 hour at 15°C. The precipitated white solid was filtered and the filter cake was dried to give the compound **INT-3c** as a white solid (12.9 g, yield 87%). $^1H$ NMR (500 MHz, DMSO-*d6*) $\delta$ 7.58 (s, 1H), 5.83 (s, 1H), 2.33 (s, 3H).

To a reaction vial containing compound **INT-3c** (5.00 g, 24.5 mmol) was added phosphorus oxychloride (75.1 g, 45.5 mL), followed by the addition of 2,6-dimethyl pyridine (10.5 g, 98.0 mmol), and the resulting reaction solution was agitated for 1 hour at 100°C. The reaction solution was concentrated under reduced pressure, then ethyl acetate (200 mL) was added, the resulting suspension was poured into ice water (350 mL), the extraction and liquid separation were conducted, and the aqueous phase was extracted with ethyl acetate (150 mL $\times$ 2). The combined organic phase was washed with water (100 mL) and saturated saline solution (150 mL), dried over anhydrous sodium sulfate, and filtered, and the filtrate was concentrated under reduced pressure to give the compound **INT-3d** as a red solid (4.22 g, yield 86%). MS: 200.9 $[M+H]^+$.

To a reaction vial were added compound **INT-3d** (4.30 g, 21.4 mmol), compound **INT-1** (8.75 g, 23.6 mmol) and bis (triphenylphosphine) palladium (II) dichloride (376 mg, 536 $\mu$mol), followed by the addition of *N*-methylpyrrolidinone (43 mL), and the resulting reaction solution was agitated for 1 hour at 85°C under liquid nitrogen. The reaction solution was cooled, poured into water (400 mL), and extracted with ethyl acetate (150 mL $\times$ 3). The combined organic phase was washed with water (100 mL) and saturated saline solution (100 mL), dried over anhydrous sodium sulfate, and filtered, and the filtrate was concentrated under reduced pressure to obtain a crude product, which was recrystallized from ethyl acetate (45 mL) to give the compound **INT-3e** as a yellow solid (3.62 g, yield 68%). $^1H$ NMR (500 MHz, DMSO-*d6)* $\delta$ 8.33 (d, *J* = 2.0 Hz, 1H), 8.30 (d, *J* = 3.5 Hz, 1H), 6.60 (d, *J* = 3.5 Hz, 1H), 6.58 (d, *J* = 2.0 Hz, 1H), 2.56 (s, 3H), 2.49 (s, 3H); MS: 247.2 $[M+H]^+$.

*N*-Bromosuccinimide (1.45 g, 8.12 mmol) was added to a suspension of compound **INT-3e** (2.00 g, 8.12 mmol) in dichloromethane (60 mL) at 15°C, and the resulting reaction solution was agitated for 30 minutes at 15°C. Dichloromethane (150 mL) was added, the mixture was washed with water (50 mL), saturated aqueous sodium bicarbonate (50 mL), and saturated saline solution (50 mL), the organic phase was dried over anhydrous sodium sulfate, and filtered, and the filtrate was concentrated under reduced pressure to give the compound **INT-3f** as a yellow solid (2.62 g, yield 99%). $^1H$ NMR (500 MHz, Chloroform-*d*) $\delta$ 8.35 (d, *J* = 3.5 Hz, 1H), 8.07 (s, 1H), 6.38 (d, *J* = 3.5 Hz, 1H), 2.68 (s, 3H), 2.54 (s, 3H); MS: 325.0 $[M+H]^+$.

To a solution of compound **INT-3f** (2.62 g, 8.06 mmol) in dichloromethane (80 mL) was added m-chloroperoxybenzoic acid (1.80 g, 8.86 mmol, purity 85%) in an ice bath, and the resulting reaction was agitated for 10 minutes in an ice bath. Dichloromethane (150 mL) was added, the mixture was washed with saturated aqueous sodium sulfite (50 mL), saturated aqueous sodium bicarbonate (50 mL), and saturated saline solution (100 mL) in sequence, the organic phase was dried over anhydrous sodium sulfate, and filtered, and the filtrate was concentrated under reduced pressure to give the compound **INT-3g** as a yellow solid (2.63 g, yield 95%). MS: 341.1 $[M+H]^+$. To a reaction vial containing compound **INT-3g** (2.63 g, 7.71 mmol) was added ammonia in 1, 4-dioxane (0.4 M, 154 mL), and the resulting reaction was agitated for 30 minutes at 55°C. The reaction solution was concentrated under reduced pressure to obtain a crude product, which was recrystallized from 1, 4-dioxane (50 mL) to obtain compound **INT-3** as a yellow solid (1.70 g, yield 75%). $^1H$ NMR (500 MHz, DMSO-*d6*) $\delta$ 8.14 (s, 1H), 8.13 (d, *J* = 3.5 Hz, 1H), 7.52 (s, 2H), 6.53 (d, *J* = 3.5 Hz, 1H), 2.44 (s, 3H); MS: 294.0 $[M+H]^+$.

Preparation of compound **INT-4**

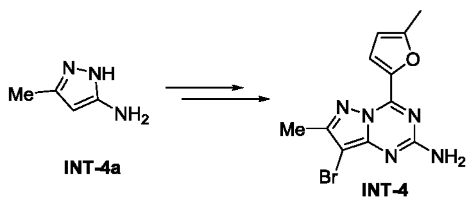

With reference to the preparation method of compound **INT-3**, compound **INT-4** may be prepared from compound INT-4a by the corresponding steps, and the spectral information was as follows: $^1H$ NMR (500 MHz, DMSO-*d6*) $\delta$ 8.16 (d, *J* = 3.5 Hz, 1H), 7.44 (s, 2H), 6.52 (d, *J* = 3.5, 1H), 2.43 (s, 3H), 2.31 (s, 3H); MS: 308.1 $[M+H]^+$.

Preparation of compound INT-5:

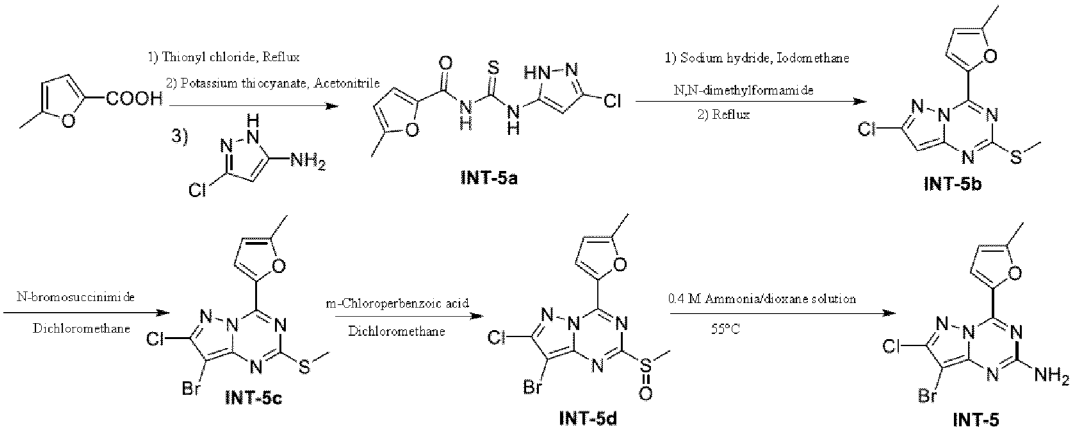


INT-5a INT-5b INT-5c INT-5d INT-5

5-Methyl-2-furoic acid (200 mg, 1.59 mmol) was added to thionyl chloride (3 mL), and the resulting solution was heated to reflux for 1 hour. The reaction solution was concentrated under reduced pressure to obtain acyl chloride, which was dissolved in anhydrous acetonitrile (2 mL) and then slowly added dropwise to a solution of potassium thiocyanate (200.6 mg, 1.98 mmol) in acetonitrile (10 mL), and the reaction was agitated at room temperature for 2 hours under nitrogen. Additional 5-chloro-1*H*-pyrazol-3-amine (186.4 mg, 1.59 mmol) was added to the above reaction solution, and the reaction was agitated at room temperature for 2 hours under nitrogen. The reaction solution was concentrated under reduced pressure to obtain a crude product, which was pulped with water (15 mL), filtered, and dried to give the compound **INT-5a** as a yellow solid (434 mg, purity 67%, yield 64%). MS: 284.9 $[M+H]^+$.

To a solution of compound **INT-5a** (434 mg, 67% pure, 1.02 mmol) in *N,N*-dimethylformamide (10 mL) was added sodium hydride (purity 60%, dispersed in mineral oil, 53.1 mg, 1.33 mmol), the reaction solution was agitated for 10 minutes at room temperature, followed by the addition of iodomethane (145 mg, 1.02 mmol), the reaction solution was agitated for 1 hour at room temperature, then heated to 150°C overnight under nitrogen. After cooling, the reaction solution was concentrated under reduced pressure to obtain a crude product, which was pulped with water (10 mL), filtered, and dried to give the compound **INT-5b** as a pale yellow solid (140 mg, yield 48%). $^1H$ NMR (500 MHz, DMSO-*d6*) $\delta$ 8.18 (d, *J* = 3.5 Hz, 1H), 6.73 (s, 1H), 6.60 (d, *J* = 3.5 Hz, 1H), 2.55 (s, 3H), 2.49 (s, 3H); MS: 281.1 $[M+H]^+$.

To a solution of compound **INT-5b** (140 mg, 0.498 mmol) in dichloromethane (5 mL) was added *N*-bromosuccinimide (88.8 mg, 0.498 mmol) at room temperature, and the resulting reaction solution was agitated for 30 minutes at room temperature. The mixture was diluted with dichloromethane (60 mL), washed with water (15 mL), saturated aqueous sodium bicarbonate (15 mL), and saturated saline solution (15 mL), the organic phase was dried over anhydrous sodium sulfate and filtered, and the filtrate was concentrated under reduced pressure to give the compound **INT-5c** as a yellow solid (179 mg, yield 99%). MS: 359.2 $[M+H]^+$.

To a solution of compound **INT-5c** (179 mg, 0.498 mmol) in dichloromethane (5 mL) was added m-chloroperoxybenzoic acid (106 mg, 0.523 mmol, purity 85%) in an ice bath, and the resulting reaction was agitated for 10 minutes in an ice bath. Dichloromethane (60 mL) was added, the mixture was washed with saturated aqueous sodium sulfite (15 mL), saturated aqueous sodium bicarbonate (15 mL), and saturated saline solution (15 mL) in sequence, the organic phase was dried over anhydrous sodium sulfate, and filtered, and the filtrate was concentrated under reduced pressure to give crude compound **INT-5d** as a yellow solid (195 mg, purity 92%, yield 95%). MS: 374.5 $[M+H]^+$.

To a reaction vial containing compound **INT-5d** (195 mg, purity 92%, 0.473 mmol) was added ammonia in 1, 4-dioxane (0.4 M, 10 mL), and the resulting reaction was agitated for 30 minutes at 55°C. The reaction solution was concentrated under reduced pressure to obtain a crude product, which was recrystallized from 1, 4-dioxane (6 mL) to obtain compound **INT-5** as a yellow solid (123 mg, yield 74%). $^1H$ NMR (500 MHz, DMSO-*d6*) $\delta$ 8.06 (d, *J* = 3.5 Hz, 1H), 7.69 (brs, 2H), 6.56 (d, *J* = 3.5 Hz, 1H), 3.57 (s, 3H), 2.46 (s, 3H); MS: 329.1 $[M+H]^+$.

Preparation of compound **INT-6:**

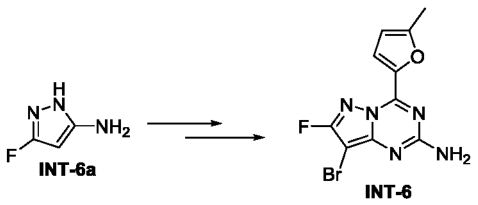

INT-6a INT-6

With reference to the preparation method of compound INT-5, compound INT-6 may be prepared from compound INT-6a by the corresponding steps, and the spectral information was as follows: $^1$H NMR (500 MHz, DMSO-*d6*) δ 7.99 (d, *J* = 3.5 Hz, 1H), 7.66 (brs, 2H), 6.53 (d, *J* = 3.5 Hz, 1H), 3.55 (s, 3H), 2.43 (s, 3H); MS: 312.2 [M+H]$^+$.

Preparation of compound **INT-7:**

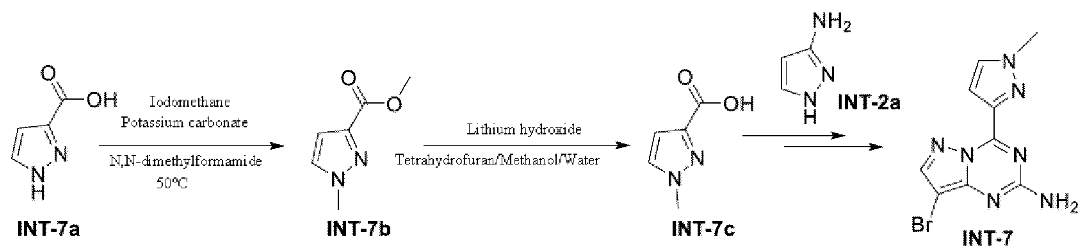


INT-7a INT-7b INT-7c INT-2a INT-7

To a solution of compound **INT-7a** (1.00 g, 8.92 mmol) in *N,N*-dimethylformamide (10 mL) were added iodomethane (2.79 g, 19.6 mmol) and potassium carbonate (2.47 g, 17.8 mmol) in sequence. The resulting reaction solution was left standing at 50°C overnight. The reaction solution was filtered, and the filtrate was concentrated under reduced pressure and purified by column chromatography (petroleum ether/ethyl acetate = 3/7) to give the compound **INT-7b** as a colorless oil (424 mg, yield 33%). $^1$H NMR (500 MHz, DMSO-*d6*) δ 7.80 (d, *J* = 2.2 Hz, 1H), 6.71 (d, *J* = 2.2 Hz, 1H), 3.90 (s, 3H), 3.76 (s, 3H).

To a mixed solution of compound **INT-7b** (424 mg, 3.03 mmol) in methanol (3.5 mL) and tetrahydrofuran (3.5 mL) was added lithium hydroxide (145 mg, 6.05 mmol) in water (3.5 mL). The resulting reaction solution was agitated for 2 hours at room temperature. The reaction solution was added to water (15 mL) and adjusted to pH=5-6 with 1 M diluted hydrochloric acid, then extracted with ethyl acetate (20 mL × 5), To the reaction solution was added water (15 mL), followed by 1 M diluted hydrochloric acid to adjust pH=5-6, then extraction was conducted with ethyl acetate (20 mL × 5), the combined organic phase was dried over anhydrous sodium sulfate, and filtered, and the filtrate was concentrated under reduced pressure to give the compound **INT-7c** as a white solid (292 mg, yield 76%).

With reference to the preparation method of compound **INT-5,** compound **INT-7** may be prepared from compound **INT-7c** and compound **INT-2a** by corresponding steps, and the spectral information was as follows: $^1$H NMR (500 MHz, DMSO-*d6*) δ 8.13 (s, 1H), 7.94 (d, *J* = 2.3 Hz, 1H), 7.53 (d, *J* = 2.3 Hz, 1H), 7.50 (s, 2H), 4.00 (s, 3H); MS: 294.0 [M+H]$^+$.

**Synthesis of compounds of examples**

Example 1:

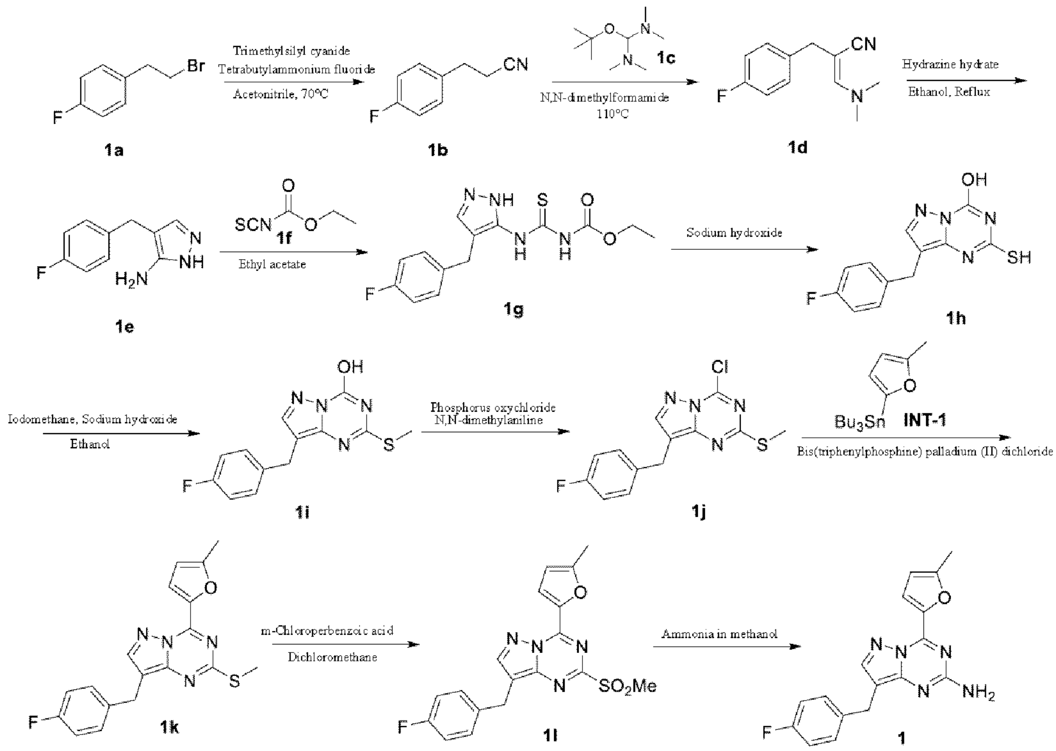


Compound 1a (2.07 g, 10.2 mmol) was dissolved in acetonitrile (10 mL), whereupon trimethylsilyl cyanide (1.52 g, 15.3 mmol, 1.91 mL) and tetrabutylammonium fluoride (1 M in tetrahydrofuran, 15.0 mL) were added at 0°C. The reaction solution was agitated for 40 minutes at 70°C, added to water (20 mL), and extracted with ethyl acetate (50 mL). The organic phase separated by extraction was washed with 50 mL saturated saline solution, dried over anhydrous sodium sulfate, and concentrated under vacuum to give a crude product, which was purified by column chromatography (ethyl acetate/petroleum ether = 1/10) to give the compound **1b** as a colorless oil (1.22 g, yield: 81%).

Compound **1b** (800 mg, 5.36 mmol) was dissolved in *N,N*-dimethylformamide (8 mL), whereupon compound **1c** (1.40 g, 8.04 mmol) was added. The reaction solution was agitated for 1 hour at 110°C, added to water (50 mL), and extracted with ethyl acetate (50 mL). The organic phase separated by extraction was washed with water (50 mL $\times$ 2) and saturated saline solution (50 mL), dried over anhydrous sodium sulfate, and concentrated under vacuum to give a crude product, which was purified by column chromatography (ethyl acetate/petroleum ether = 1/5) to give the product **Id** as a yellow solid (0.75 g, yield: 69%). MS: 205.6 $[M+H]^+$.

Compound **Id** (650 mg, 3.18 mmol) was dissolved in ethanol (6 mL), whereupon hydrazine hydrate (3.19 g, 31.8 mmol) was added. The reaction solution was agitated for 48 hours at 100°C, then concentrated under vacuum to give the desired crude product **1e** as a yellow oil (600 mg, yield: 99%). $^1$H NMR (500 MHz, DMSO-*d6*) $\delta$ 11.27 (s, 1H), 7.26 - 7.23 (m, 2H), 7.10 - 7.06 (m, 3H), 4.42 (s, 2H), 3.60 (s, 2H); MS: 192.6 $[M+H]^+$.

Compound **1e** (600 mg, 3.14 mmol) was dissolved in ethyl acetate (15 mL), whereupon a solution of compound **If** (412 mg, 3.14 mmol) in ethyl acetate (5 mL) was added dropwise at 0°C. The reaction solution was agitated for 2 hours at 0°C, then concentrated under vacuum and pulped (ethyl acetate/petroleum ether = 1/1, 15 mL) to give the desired product **1g** as a white solid (523 mg, yield: 52%). MS: 323.3 $[M+H]^+$.

Compound **1g** (523 mg, 1.62 mmol) was dissolved in an aqueous solution of sodium hydroxide (2 M, 10.5 mL). The reaction solution was agitated for half an hour at room temperature, and adjusted to pH=6 with 1 M hydrochloric acid to precipitate a large amount of solid. The solid was filtered and dried under vacuum to give the desired product **1h** as a white solid (448 mg, yield: 99%). MS: 277.4 $[M+H]^+$.

Compound **1h** (224 mg, 811 $\mu$mol) was dissolved in ethanol (3 mL), whereupon aqueous sodium hydroxide (2 M, 3.24 mL) and iodomethane (115 mg, 811 $\mu$mol, 75.2 $\mu$L) were added in sequence. The reaction solution was agitated for 1 hour at room temperature, adjusted to pH=5 with 1 M hydrochloric acid, and extracted with ethyl acetate (50 mL). The organic phase separated by extraction was washed with saturated saline solution (30 mL), dried over anhydrous sodium sulfate, and concentrated under vacuum to give the desired crude product **1i** as a white solid (235 mg, yield: 99%). $^1$H NMR (500 MHz, DMSO-*d6*) $\delta$ 12.79 (s, 1H), 7.89 (s, 1H), 7.32 - 7.29 (m, 2H), 7.11 - 7.08 (m, 2H), 3.87 (s, 2H), 2.55 (s, 3H); MS: 291.3 $[M+H]^+$.

Compound **1i** (242 mg, 832 $\mu$mol) was added to phosphorus oxychloride (5.11 g, 33.3 mmol, 3.09 mL), whereupon *N,N*-dimethylaniline (478 mg, 3.94 mmol, 0.5 mL) was added. The reaction solution was agitated for 2 hours at

90°C and slowly added dropwise to ice water (50 mL), the mixture was adjusted to pH=8 with saturated sodium bicarbonate in an ice bath and extracted with ethyl acetate (50 mL). The organic phase separated by extraction was washed with dilute hydrochloric acid (50 mL) and saturated saline solution (50 mL), dried over anhydrous sodium sulfate, and concentrated under vacuum to give the desired crude product **1j** as a yellow oil (262 mg), which was used in the next step without purification. MS: 309.2 $[M+H]^+$.

To *N*-methylpyrrolidinone (4 mL) were added compound **1j** (262 mg, 848 μmol), INT-I (472 mg, 1.27 mmol), and bis (triphenylphosphine) palladium **(II)** dichloride (59.6 mg, 84.7 μmol) under nitrogen. The reaction system was repeatedly purged with nitrogen gas 3 times and then agitated for 40 minutes at 80°C. The reaction solution was poured into water (50 mL) and the aqueous phase was extracted with ethyl acetate (25 mL × 2). The organic layers were combined, washed with saturated saline solution (50 mL), dried over anhydrous sodium sulfate, and filtered, and the filtrate was concentrated under vacuum to give a crude product, which was purified by column chromatography (ethyl acetate/petroleum ether = 1/4) to give **1k** as a yellow solid (175 mg, yield: 58%). $^1H$ NMR (500 MHz, Chloroform-d) δ 8.34 (d, *J* = 3.5 Hz, 1H), 7.92 (s, 1H),6.99 - 6.95 (m, 1H), 6.35 (d, *J* = 3.5 Hz, 1H), 4.05 (s, 2H), 2.63 (s, 3H), 2.53 (s, 3H) ; MS: 355.3 $[M+H]^+$. Compound **1k** (100 mg, 282 μmol) was added to dichloromethane (3 mL), whereupon m-chloroperoxybenzoic acid (143 mg, 705 μmol, purity 85%) was added. The reaction solution was agitated for 1 hour at room temperature, diluted with dichloromethane (50 mL), and washed with saturated aqueous sodium thiosulfate (50 mL), saturated sodium bicarbonate (50 mL), and saturated saline solution (50 mL) in sequence. The separated organic phase was dried over anhydrous sodium sulfate and concentrated under vacuum to give the desired crude product **1l** as a white solid (109 mg, yield: 99%), which was used in the next step without purification. MS: 387.3 $[M+H]^+$.

Compound **1l** (109 mg, 282 μmol) was dissolved in ammonia in methanol (7 M, 4 mL). The reaction solution was agitated for 30 minutes at 60°C, then concentrated under vacuum to obtain a crude product, which was purified by preparative high performance liquid chromatography (preparative HPLC) to give the product **1** as a yellow solid (16 mg, yield: 18%). $^1H$ NMR (500 MHz, DMSO-*d6*) δ 8.19 (d, *J* = 3.0 Hz, 1H), 7.94 (s, 1H), 7.30 - 7.23 (m, 2H), 7.20 (s, 2H), 7.12 - 7.05 (m, 2H), 6.53 (d, *J* = 3.0 Hz, 1H), 3.85 (s, 2H), 2.46 (s, 3H); MS: 324.4 $[M+H]^+$.

Example 2:

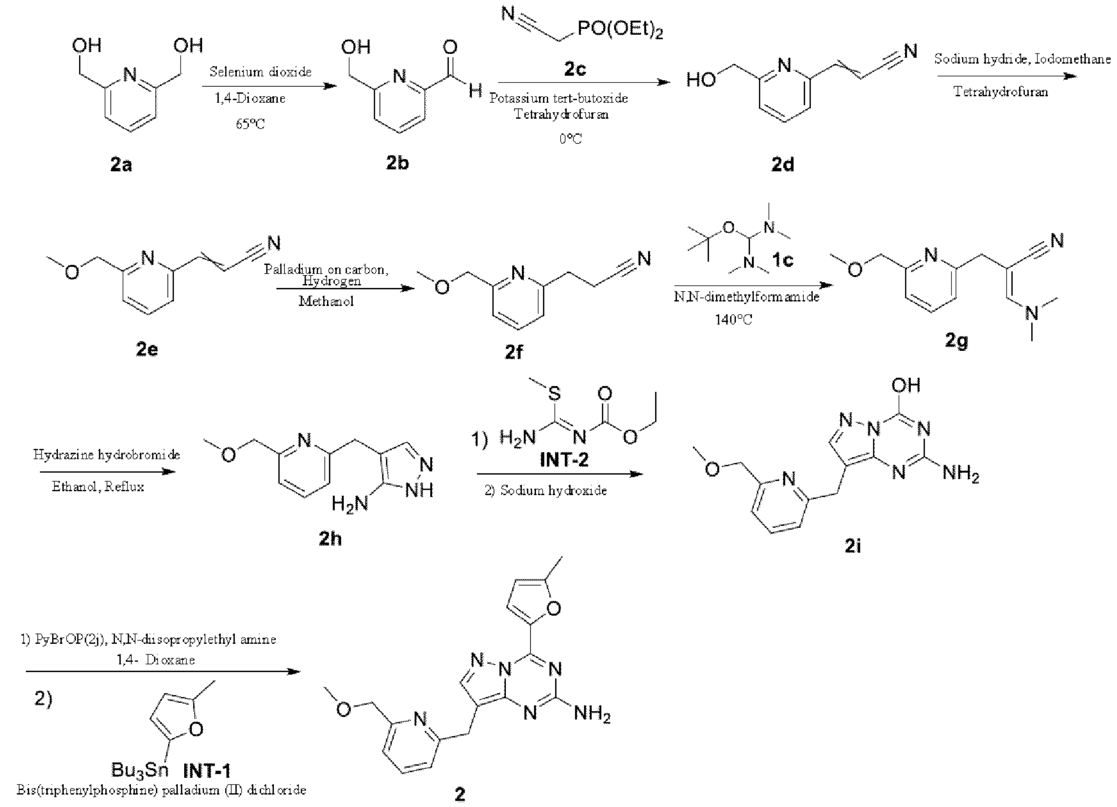


Compound 2a (4.00 g, 28.8 mmol) was dissolved in 1, 4-dioxane (80 mL), whereupon selenium dioxide (1.59 g, 14.4 mmol) was added. The reaction solution was agitated for 48 hours at 65°C. The reaction solution was filtered,

the obtained filtrate was concentrated under vacuum to obtain a crude product, which was purified by column chromatography (ethyl acetate/petroleum ether = 1/1) to give the product **2b** as a white solid (3.12 g, yield: 79%).

Compound **2c** (3.55 g, 20.1 mmol) was dissolved in tetrahydrofuran (25 mL), whereupon potassium tert-butoxide (2.25 g, 20.1 mmol) was added at 0°C. The reaction was agitated for 30 minutes at 0°C, followed by the addition of compound **2b** (2.62 g, 19.1 mmol), the mixture was agitated for 30 minutes at 0°C. The mixture was added to water (100 mL) and extracted with ethyl acetate (100 mL × 2). The organic phase separated by extraction was dried over anhydrous sodium sulfate, and concentrated under vacuum to give a pair of Z/E isomer mixtures **2d** as a yellow solid (3.82 g). MS: 161.3 $[M+H]^+$.

Compound **2d** (1.00 g, 6.24 mmol) was dissolved in tetrahydrofuran (20 mL), whereupon sodium hydride (375 mg, 9.36 mmol, purity 60%) was added at 0°C. The reaction solution was agitated for 30 minutes at 0°C, followed by the addition of iodomethane (975 mg, 6.87 mmol, 428 μL), and the reaction solution was agitated for 18 hours at room temperature. The mixture was added to water (100 mL and extracted with ethyl acetate (100 mL × 2). The organic phase separated by extraction was dried over anhydrous sodium sulfate, filtered, and concentrated under vacuum to give the desired crude product **2e** as a yellow oil (1.07 g, yield: 98%). MS: 175.1 $[M+H]^+$. Compound **2e** (1.07 g, 6.14 mmol) was dissolved in methanol (20 mL), whereupon palladium (10% adsorbed on activated carbon, 100 mg) was added. The reaction solution was agitated for 18 hours at room temperature under hydrogen, filtered, and concentrated under vacuum to give the desired crude product **2f** as a yellow oil (1.06 g, yield: 98%). $^1H$ NMR (500 MHz, Chloroform-*d*) δ 7.69 (t, *J* = 7.7 Hz, 1H), 7.34 (d, *J* = 7.7 Hz, 1H), 7.15 (d, *J* = 7.7 Hz, 1H), 4.58 (s, 2H), 3.51 (s, 3H), 3.14 (t, *J* = 7.4 Hz, 2H), 2.86 (t, *J* = 7.4 Hz, 2H); MS: 177.6 $[M+H]^+$.

Compound **2f** (3.05 g, 17.3 mmol) was dissolved in *N,N*-dimethylformamide (8 mL), whereupon compound **1c** (4.52 g, 26.0 mmol, 5.36 mL) was added. The reaction solution was agitated for 1.5 hours at 140°C. The reaction solution was added to water (100 mL) and extracted with ethyl acetate (100 mL × 2). The organic phase separated by extraction was washed with water (100 mL) and saturated saline (100 mL), dried over anhydrous sodium sulfate, and concentrated under vacuum to give a crude product, which was purified by column chromatography (ethyl acetate/petroleum ether = 1/5) to give yellow oily liquid **2g** (3.86 g, yield: 96%). MS: 232.6 $[M+H]^+$.

Compound **2g** (3.86 g, 16.7 mmol) was dissolved in ethanol (30 mL), whereupon hydrazine hydrobromide (2.83 g, 25.0 mmol) was added. The reaction solution was agitated for 1 hour at 80°C. The reaction solution was concentrated under vacuum, carefully added to saturated sodium bicarbonate (50 mL) at 0°C, and extracted with a mixed solution of dichloromethane/methanol (10: 1, 50 mL × 3). The organic phase separated by extraction was dried over anhydrous sodium sulfate and concentrated under vacuum to obtain a crude product, which was purified by column chromatography (methanol/dichloromethane = 1/10) to give yellow oily liquid **2h** (1.37 g, yield: 38%). $^1H$ NMR (500 MHz, DMSO-*d6*) δ 7.69 (t, *J* = 7.7 Hz, 1H), 7.20 (d, *J* = 7.7 Hz, 1H), 7.13 (s, 1H), 7.11 (d, *J* = 7.7 Hz, 1H), 4.46 (s, 2H), 3.72 (s, 2H), 3.35 (s, 3H); MS: 219.6 $[M+H]^+$. Compound **2h** (500 mg, 2.29 mmol) was dissolved in acetonitrile (10 mL), whereupon compound **INT-2** (743.21 mg, 4.58 mmol) was added. The reaction solution was agitated for 18 hours at 95°C and concentrated under vacuum, whereupon 2M aqueous sodium hydroxide (11.45 mL) was added, and the reaction solution was agitated for 5 hours at room temperature. The reaction solution was adjusted to pH=6 with 1 M aqueous hydrochloric acid to precipitate a solid. The reaction solution was filtered to obtain a solid, which was dried under vacuum to give the compound **2i** as a yellow solid (190 mg, yield: 29%). MS: 287.4 $[M+H]^+$.

Compound **2i** (20 mg, 63.0 μmol) was dissolved in 1, 4-dioxane (3 mL), whereupon compound **2j** (38.2 mg, 81.9 μmol) and *N,N*-diisopropylethyl amine (24.4 mg, 189 μmol) were added. The reaction solution was agitated for 2 hours at 50°C and cooled to room temperature, whereupon **INT-1** (46.8 mg, 126 μmol) and bis(triphenylphosphine) palladium (II) dichloride (4.42 mg, 6.30 μmol) were added, and the mixture was purged with nitrogen 3 times and then heated to 80°C for 1 hour. The reaction solution was poured into water (50 mL) and the aqueous phase was extracted with ethyl acetate (25 mL × 2). The organic layers were combined, washed with saturated saline solution, dried over anhydrous sodium sulfate, and filtered, and the filtrate was concentrated under vacuum to obtain a crude product, which was purified by preparative HPLC to give the product **2** as a yellow solid (5 mg, yield: 21%). $^1H$ NMR (500 MHz, Methanol-*d4*) δ 8.28 (d, *J* = 3.5 Hz, 1H), 7.91 (s, 1H), 7.73 (t, *J* = 7.8 Hz, 1H), 7.33 (d, *J* = 7.8 Hz, 1H), 7.22 (d, *J* = 7.8 Hz, 1H), 6.47 (d, *J* = 3.5 Hz, 1H), 4.56 (s, 2H), 4.12 (s, 2H), 3.48 (s, 3H), 2.51 (s, 3H); MS: 351.5 $[M+H]^+$.

Example 3:

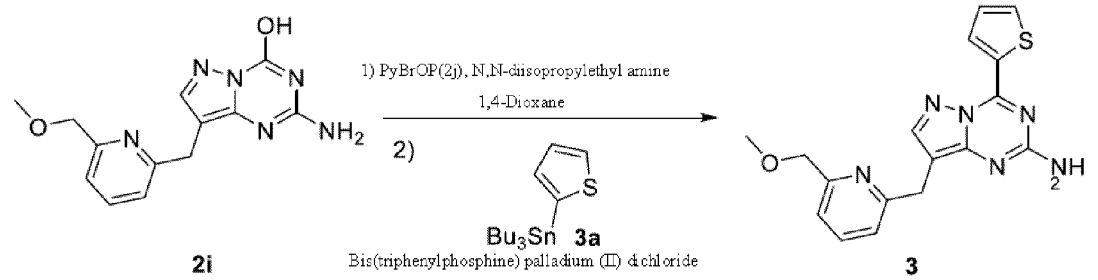


2i 3

Compound **2i** (50 mg, 175 μmol) was dissolved in 1, 4-dioxane (6.5 mL), whereupon compound **2j** (106 mg, 227 μmol) and *N,N*-diisopropylethyl amine (67.7 mg, 524 μmol, 92.8 μL) were added. The reaction solution was agitated for 40 minutes at 50°C and cooled to room temperature, whereupon **3a** (130 mg, 175 μmol) and bis(triphenylphosphine) palladium (II) dichloride (12.3 mg, 17.5 (μmol) were added, and the mixture was purged with nitrogen 3 times and then heated to 100°C for 1 hour. The reaction solution was poured into water (50 mL) and the aqueous phase was extracted with ethyl acetate (25 mL × 2). The organic layers were combined, washed with saturated saline solution, dried over anhydrous sodium sulfate, and filtered, and the filtrate was concentrated under vacuum to obtain a crude product, which was purified by preparative HPLC to give the product 3 as a yellow solid (7.5 mg, yield: 12%). $^1$H NMR (500 MHz, Methanol-*d4*) δ 8.87 (d, *J* = 3.9 Hz, 1H), 7.93 (d, *J* = 5.0 Hz, 1H), 7.92 (s, 1H), 7.71 (t, *J* = 7.7 Hz, 1H), 7.31 (d, *J* = 7.7 Hz, 1H), 7.30 - 7.28 (m, 1H), 7.21 (d, *J* = 7.7 Hz, 1H), 4.54 (s, 2H), 4.11 (s, 2H), 3.45 (s, 3H); MS 353.3 [M+H]$^+$.

Example 4:

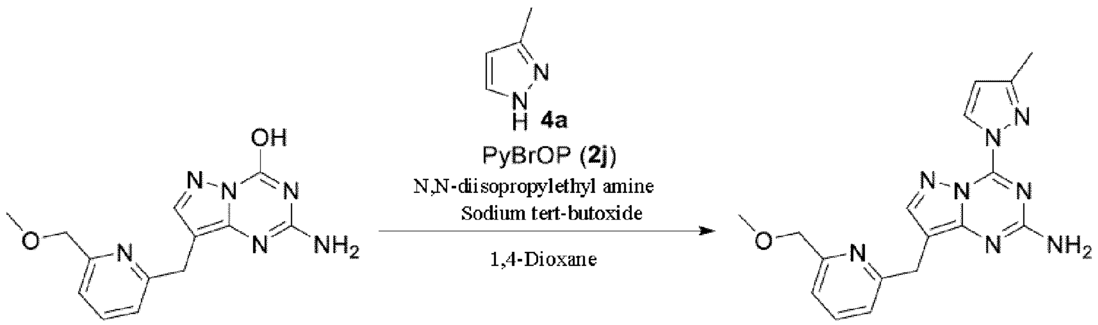


2i 4

Compound **2i** (30 mg, 105 μmol) was dissolved in 1, 4-dioxane (4 mL), whereupon compound **2j** (63.5 mg, 136 μmol) and *N,N*-diisopropylethyl amine (40.6 mg, 314 μmol, 55.7 μL) were added. The reaction solution was agitated for 40 minutes at 50°C and cooled to room temperature, whereupon **4a** (130 mg, 175 μmol) and sodium tert-butoxide (12.1 mg, 125 μmol) were added, and the mixture was left standing for 2 hours at 50°C. The reaction solution was poured into water (50 mL) and the aqueous phase was extracted with ethyl acetate (25 mL × 2). The organic layers were combined, washed with saturated saline solution, dried over anhydrous sodium sulfate, and filtered, and the filtrate was concentrated under vacuum to obtain a crude product, which was purified by preparative HPLC to give the product **4** as a yellow solid (15 mg, yield: 29%). $^1$H NMR (500 MHz, DMSO-*d6*) δ 9.28 (d, *J* = 3.0 Hz, 1H), 8.01 (s, 1H), 7.67 (t, *J* = 7.7 Hz, 1H), 7.34 (s, 2H), 7.20 (d, *J* = 7.7 Hz, 1H), 7.10 (d, *J* = 7.7 Hz, 1H), 6.53 (d, *J* = 3.0 Hz, 1H), 4.45 (s, 2H), 3.98 (s, 2H), 3.34 (s, 3H), 2.31 (s, 3H); MS 351.4 [M+H]$^+$.

Examples 5 and 6:

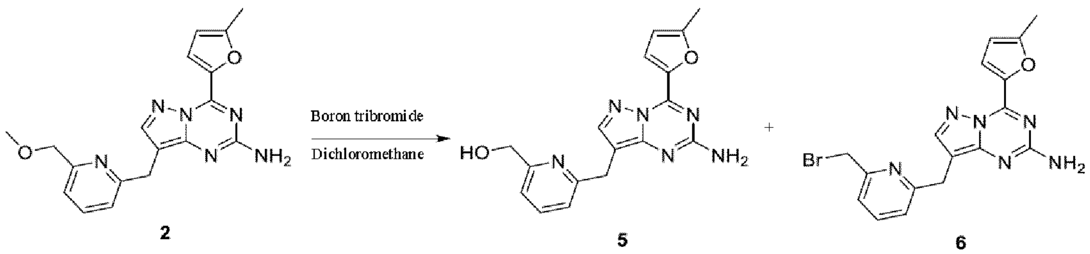


2 5 6

Compound **2** (33 mg, 94.2 μmol) was dissolved in dichloromethane (5 mL), whereupon boron tribromide (1 M in dichloromethane, 188 μL) was added dropwise at 0°C. The reaction solution was agitated for 15 minutes at 0°C and added to water (30 mL), and the mixture was adjusted to pH=8 with saturated sodium bicarbonate and extracted with ethyl acetate (25 mL×2). The organic layers were combined, washed with saturated saline solution, dried over anhydrous sodium sulfate, and filtered, and the filtrate was concentrated under vacuum to obtain a crude product, which was purified by preparative HPLC to give the product **5** as a yellow solid (10 mg, yield: 32%) and product 6 as a yellow solid (7 mg, yield: 19%). The spectral information about compounds 5 and 6 was as follows:

Compound 5: $^1$H NMR (500 MHz, Methanol-*d4*) $\delta$ 8.25 (d, *J* = 3.5 Hz, 1H), 7.89 (s, 1H), 7.71 (t, *J* = 7.8 Hz, 1H), 7.36 (d, *J* = 7.8 Hz, 1H), 7.16 (d, *J* = 7.8 Hz, 1H), 6.44 (d, *J* = 3.5 Hz, 1H), 4.68 (s, 2H), 4.09 (s, 2H), 2.48 (s, 3H); MS 337.3 $[M+H]^+$.

Compound 6: $^1$H NMR (500 MHz, Methanol-*d4*) $\delta$ 8.25 (d, *J* = 3.5 Hz, 1H), 7.91 (s, 1H), 7.70 (t, *J* = 7.8 Hz, 1H), 7.36 (d, *J* = 7.8 Hz, 1H), 7.21 (d, *J* = 7.8 Hz, 1H), 6.44 (d, *J* = 3.5 Hz, 1H), 4.59 (s, 2H), 4.11 (s, 2H), 2.48 (s, 3H); MS 399.1 $[M+H]^+$.

Example 7:

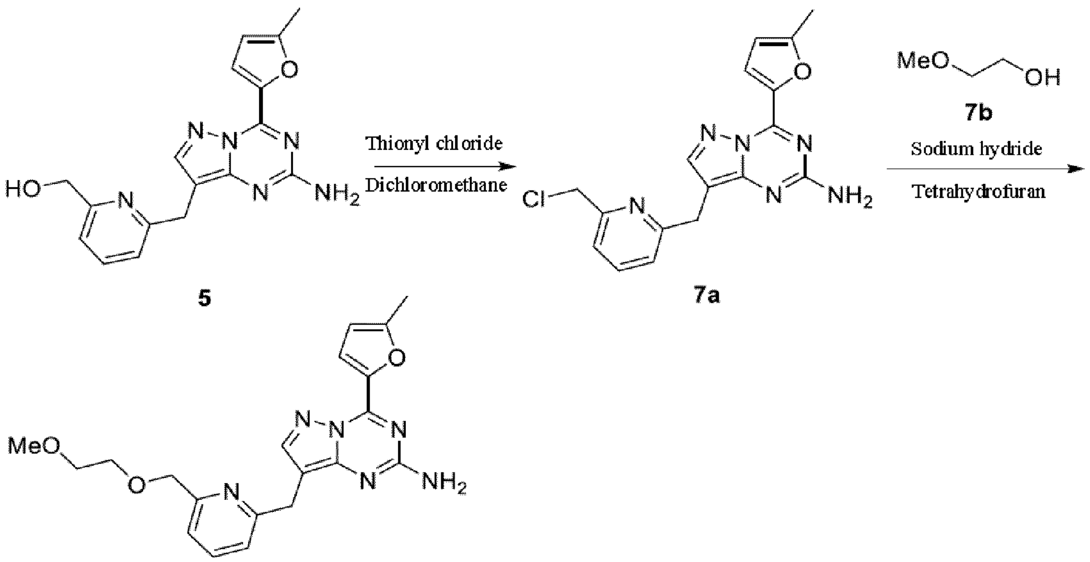


5 7a

7

Compound **5** (183 mg, 544 μmol) was dissolved in dichloromethane (20 mL), whereupon thionyl chloride (3.24 g, 27.20 mmol, 1.97 mL) was added dropwise at 0°C. The reaction solution was agitated for 1.5 hours at 40°C and added to water (10 mL), and the mixture was adjusted to pH=8 with saturated sodium bicarbonate and extracted with dichloromethane (50 mL×3). The organic layers were combined, washed with saturated saline solution (50 mL), dried over anhydrous sodium sulfate, and filtered, and the filtrate was concentrated under vacuum to give a crude product, which was purified by column chromatography (ethyl acetate/petroleum ether = 1/1) to give the product **7a** as a yellow solid (41 mg, yield: 21%). MS:355.2 $[M+H]^+$.

Compound **7b** (72.9 mg, 958 μmol) was dissolved in tetrahydrofuran (8 mL), whereupon sodium hydride (46.0 mg, 1.15 mmol, purity 60%) was added at 0°C. The reaction solution was agitated for 5 minutes at 0°C, followed by the

addition of a solution of compound **7a** (34 mg, 95.8 μmol) in tetrahydrofuran (2 mL), and the reaction solution was agitated for 1 hour at room temperature. The mixture was added to water (10 mL and extracted with ethyl acetate (20 mL × 2). The organic phase separated by extraction was dried over anhydrous sodium sulfate, filtered, and concentrated under vacuum to give a crude product, which was purified by preparative HPLC to give the product **7** as a yellow solid (4.4 mg, yield: 12%). $^{1}$H NMR (500 MHz, Methanol-*d4*) $\delta$ 8.28 (d, *J* = 3.5 Hz, 1H), 7.92 (s, 1H), 7.73 (t, *J* = 7.7 Hz, 1H), 7.39 (d, *J* = 7.7 Hz, 1H), 7.22 (d, *J* = 7.7 Hz, 1H), 6.47 (d, *J* = 3.5 Hz, 1H), 4.66 (s, 2H), 4.12 (s, 2H), 3.76 - 3.72 (m, 2H), 3.65 - 3.61 (m, 2H), 3.40 (s, 3H), 2.51 (s, 3H); MS 395.4 $[M+H]^+$.

Example 8:

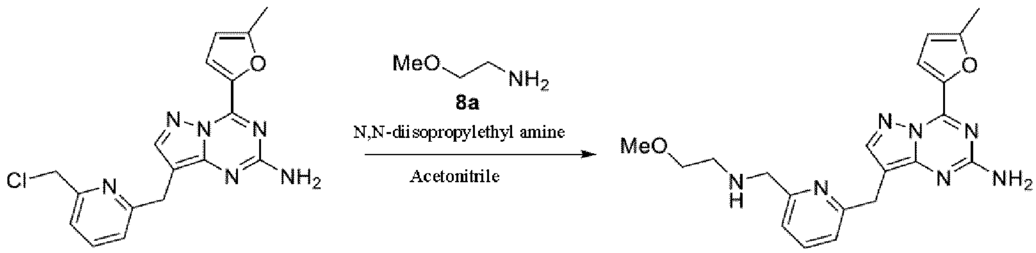


7a 8

Compound 7a (6.35 mg, 84.6 μmol) and *N,N*-diisopropylethyl amine (10.9 mg, 84.6 μmol, 14.0 uL) were dissolved in acetonitrile (2 mL), whereupon compound **8a** (15.0 mg, 42.3 μmol) was added. The reaction solution was agitated for 3 hours at 80°C, then concentrated under vacuum to obtain a crude product, which was purified by preparative HPLC to give the product **8** as a yellow solid (5.0 mg, yield: 30%). $^{1}$H NMR (500 MHz, DMSO-*d6*) $\delta$ 8.17 (d, *J* = 3.5 Hz, 1H), 7.97 (s, 1H), 7.61 (t, *J* = 8.0 Hz, 1H), 7.20 (d, *J* = 8.0 Hz,1H), 7.18 (s, 2H), 7.04 (d, *J* = 8.0 Hz, 1H), 6.55 - 6.49 (m, 1H), 3.97 (s, 2H), 3.80 (s, 2H), 3.40 (t, *J* = 5.5 Hz, 2H), 3.22 (s, 3H), 2.71 (t, *J* = 5.5 Hz, 2H), 2.44 (s, 3H); MS 394.4 $[M+H]^+$.

Example 9:

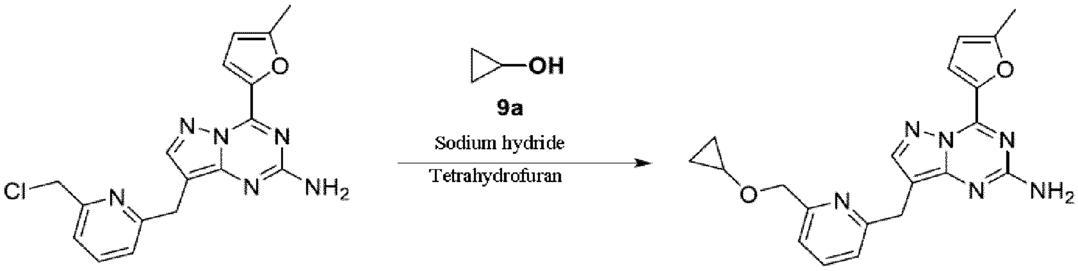


7a 9

Compound **7a** (114.59 mg, 1.97 mmol) was dissolved in tetrahydrofuran (8 mL), whereupon sodium hydride (94.7 mg, 2.37 mmol, purity 60%) was added at 0°C. The reaction solution was agitated for 5 minutes at 0°C, followed by the addition of a solution of compound **9a** (70 mg, 197 μmol), and the reaction solution was agitated for 4 hours at room temperature. The mixture was added to water (15 mL and extracted with ethyl acetate (30 mL × 2). The organic phase separated by extraction was dried over anhydrous sodium sulfate, filtered, and concentrated under vacuum to give a crude product, which was purified by preparative HPLC to give the product **9** as a yellow solid (4.4 mg, yield: 5%). $^{1}$H NMR (500 MHz, Methanol-*d4*) $\delta$ 8.25 (d, *J* = 3.5 Hz, 1H), 7.89 (s, 1H), 7.70 (t, *J* = 7.5 Hz, 1H), 7.30 (d, *J* = 7.5 Hz, 1H), 7.19 (d, *J* = 7.5 Hz, 1H), 6.44 (d, *J* = 3.5 Hz, 1H), 4.64 (s, 2H), 4.10 (s, 2H), 3.48 - 3.43 (m, 1H), 2.48 (s, 3H), 0.65 - 0.61 (m, 2H), 0.54 - 0.47 (m, 2H); MS 377.4 $[M+H]^+$.

Example 10:

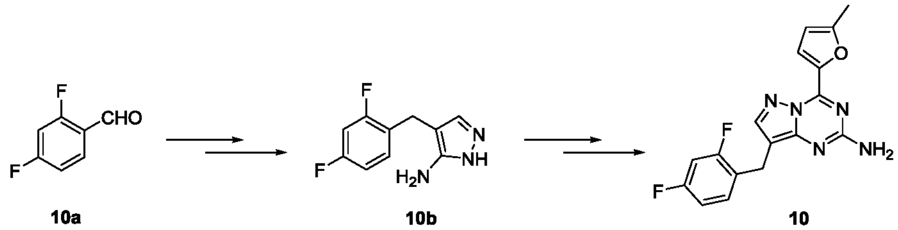

10a 10b 10

With reference to the preparation method of compound **2h,** compound **10b** may be prepared from compound **10a** by the corresponding steps, and the spectral information about compound **10b** was as follows: $^1$H NMR (500 MHz, Chloroform-*d*) $\delta$ 7.15 (s, 1H), 7.13-7.09 (m, 1H), 6.82-6.78 (m, 2H), 5.05 (s, 2H), 3.68 (s, 2H).

With reference to the preparation method of compound **1,** compound **10** may be prepared from compound **10b** by the corresponding steps, and the spectral information about compound **10** was as follows: $^1$H NMR (500 MHz, Chloroform-*d*) $\delta$ 8.50 (d, *J* = 3.6 Hz, 1H), 7.92 (s, 1H), 7.49 - 7.42 (m, 1H), 6.83 - 6.76 (m, 2H), 6.45 (d, *J* = 3.6 Hz, 1H), 4.02 (s, 2H), 2.56 (s, 3H); MS 342.3 $[M+H]^+$.

Example 11:

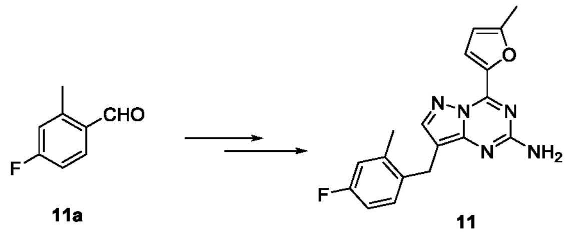

11a 11

With reference to the preparation method of compound **10,** compound **11** may be prepared from compound **11a** by the corresponding steps, and the spectral information about compound **11** was as follows: $^1$H NMR (500 MHz, Chloroform-d) $\delta$ 8.36 (d, *J* = 3.5 Hz, 1H), 7.69 (s, 1H), 7.29 (s, 2H), 7.18 - 7.10 (m, 1H), 6.93 - 6.88 (m, 1H), 6.87 - 6.80 (m, 1H), 6.38 (d, *J* = 3.5 Hz, 1H), 3.91 (s, 2H), 2.56 (s, 3H), 2.34 (s, 3H); MS: 338.3 $[M+H]^+$.

Example 12:

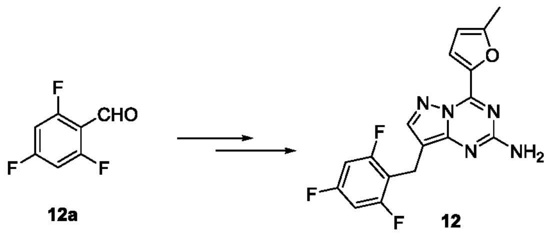

12a 12

With reference to the preparation method of compound **10,** compound **12** may be prepared from compound **12a** by the corresponding steps, and the spectral information about compound **12** was as follows: $^1$H NMR (500 MHz, Chloroform-*d*) $\delta$ 8.32 (d, *J* = 3.5 Hz, 1H), 7.79 (s, 1H), 6.69 - 6.61 (m, 2H), 6.34 (d, *J* = 3.5 Hz, 1H), 5.26 (s, 2H), 3.95 (s, 2H), 2.52 (s, 3H); MS: 360.3 $[M+H]^+$.

Example 13:

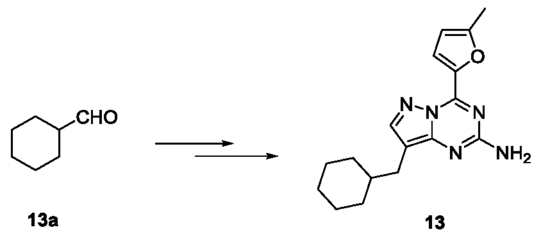

13a 13

With reference to the preparation method of compound **10,** compound **13** may be prepared from compound **13a** by the corresponding steps, and the spectral information about compound **13** was as follows: $^1$H NMR (500 MHz, Methanol-*d4*) $\delta$ 8.25 (d, *J* = 3.5 Hz, 1H), 7.84 (s, 1H), 6.44 (d, *J* = 3.5 Hz, 1H), 2.48 (s, 3H), 2.46 (d, *J* = 7.0 Hz, 2H), 2.21 - 2.16 (m, 1H), 2.05 - 2.00 (m, 1H), 1.72 - 1.70 (m, 3H), 1.66-1.63 (m, 1H), 1.60 - 1.55 (m, 1H), 1.23 - 1.18 (m, 2H), 1.02 - 0.87(m, 2H); MS: 312.5 [M+H]$^+$.

Example 14:

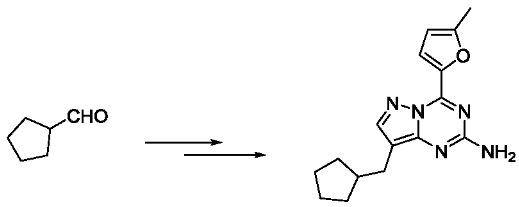

14a 14

With reference to the preparation method of compound **10,** compound **14** may be prepared from compound **14a** by the corresponding steps, and the spectral information about compound **14** was as follows: $^1$H NMR (500 MHz, Chloroform-d) $\delta$ 8.38 (d, *J* = 3.5 Hz, 1H), 7.90 (s, 1H), 6.38 (d, *J* = 3.5 Hz, 1H), 5.42 (s, 2H), 2.55 (s, 3H), 2.20 (m, 2H), 1.89 - 1.49 (m, 9H); MS: 298.4 [M+H]$^+$.

Example 15:

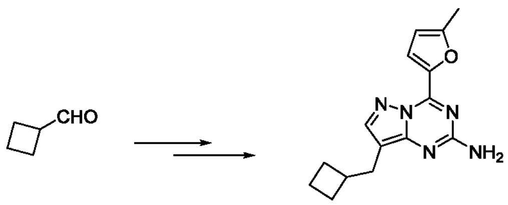

15a 15

With reference to the preparation method of compound **10,** compound **15** may be prepared from compound **15a** by the corresponding steps, and the spectral information about compound **15** was as follows: $^1$H NMR (500 MHz, Chloroform-*d*) $\delta$ 8.37 (d, *J* = 3.5 Hz, 1H), 7.87 (s, 1H), 6.37 (d, *J* = 3.5 Hz, 1H), 5.26 (s, 2H), 2.72 (d, *J* = 7.5 Hz, 2H), 2.66 (m, 1H), 2.55 (s, 3H), 2.15 - 2.07 (m, 2H), 1.91 - 1.85 (m, 2H), 1.78 - 1.74 (m, 2H); MS: 284.4 [M+H]$^+$.

Example 16:

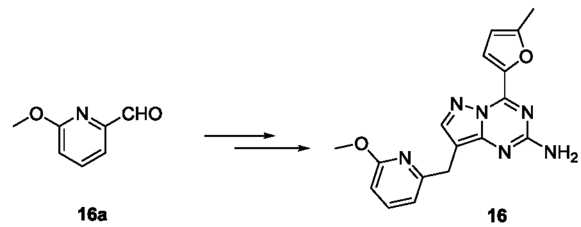

16a 16

With reference to the preparation method of compound **10,** compound **16** may be prepared from compound **16a** by the corresponding steps, and the spectral information about compound **16** was as follows: $^1$H NMR (500 MHz, Chloroform-*d*) $\delta$ 8.36 (d, *J* = 3.4 Hz, 1H), 8.04 (s, 1H), 7.45 (t, *J* = 7.7 Hz, 1H), 6.76 (d, *J* = 7.7 Hz, 1H), 6.55 (d, *J* = 7.7 Hz, 1H), 6.35 (d, *J* = 3.4 Hz, 1H), 5.32 (s, 2H), 4.04 (s, 2H), 3.94 (s, 3H), 2.53 (s, 3H); MS 337.4 [M+H]$^+$.

Example 17:

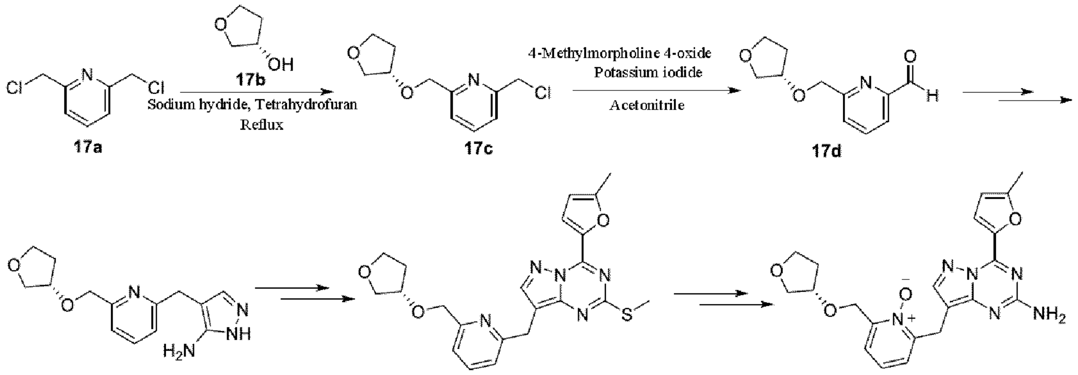


17a 17c 17d

17e 17f 17

Compound 17b (1.55 g, 17.6 mmol) was dissolved in tetrahydrofuran (45 mL), whereupon sodium hydride (1.06 g, 26.4 mmol, purity 60%) was added. The reaction solution was agitated for 1 hour at 0°C, followed by the addition of compound **17a** (3.10 g, 17.6 mmol), and the reaction solution was agitated for 18 hours at 60°C. The reaction solution was cooled to room temperature, added to saturated ammonium chloride solution (50 mL), and extracted with ethyl acetate (50 mL $\times$ 2). The organic phase separated by extraction was dried over anhydrous sodium sulfate, filtered, and concentrated under vacuum to give a crude product, which was purified by column chromatography to give the compound **17c** as a yellow oil (1.93 g, yield: 48%). MS: 228.6 [M+H]$^+$.

Compound **17c** (930 mg, 4.08 mmol) was dissolved in acetonitrile (10 mL), whereupon 4-methylmorpholine 4-oxide (957 mg, 8.17 mmol) and potassium iodide (67.8 mg, 408 $\mu$mol) were added at 0°C. The reaction solution was agitated for 6 hours at 70°C, concentrated under vacuum, added to water (50 mL), and extracted with ethyl acetate (50 mL $\times$ 2). The organic phase separated by extraction was dried over anhydrous sodium sulfate, filtered, and concentrated under vacuum to give the desired crude product **17d** as a yellow oil (560 mg, yield: 66%). MS: 208.8 [M+H]$^+$.

With reference to the preparation method of compound **2h,** compound **17e** may be prepared from compound **17d** by the corresponding steps, and the spectral information about compound **17e** was as follows: $^1$H NMR (500 MHz, Chloroform-*d*) $\delta$ 7.64 (t, *J* = 7.7 Hz, 1H), 7.31 (d, *J* = 7.7 Hz, 1H), 7.26 (s, 1H), 7.09 (d, *J* = 7.7 Hz, 1H), 4.66 - 4.59 (m, 2H), 4.31 - 4.28 (m, 1H), 4.00-3.93 (m, 2H), 3.89 - 3.88 (m, 3H), 3.52 (s, 2H), 2.11 - 2.03 (m, 2H); MS: 275.7 [M+H]$^+$.

With reference to the preparation method of compound **1,** compound **17** may be prepared from compound **17e** by the corresponding steps, and the spectral information about related intermediate **17f** and compound **17** was as follows:

Compound **17f:** MS: 437.3 [M+H]$^+$.

Compound **17:** $^1$H NMR (500 MHz, Methanol-*d4*) $\delta$ 8.27 (d, *J* = 3.5 Hz, 1H), 8.02 (s, 1H), 7.54 (d, *J* = 7.8 Hz, 1H), 7.46 (t, *J* = 7.8 Hz, 1H), 7.26 (d, *J* = 7.8 Hz, 1H), 6.45 (d, *J* = 3.5 Hz, 1H), 4.85 (d, *J* = 11.0 Hz, 1H), 4.78 (d, *J* = 11.0 Hz, 2H), 4.41 - 4.40 (m, 1H), 4.22 (s, 2H), 3.96 - 3.91 (m, 2H), 3.87 - 3.82 (m, 2H), 2.49 (s, 3H), 2.14 -2.10 (m, 2H); MS 423.3 [M+H]$^+$.

Example 18:

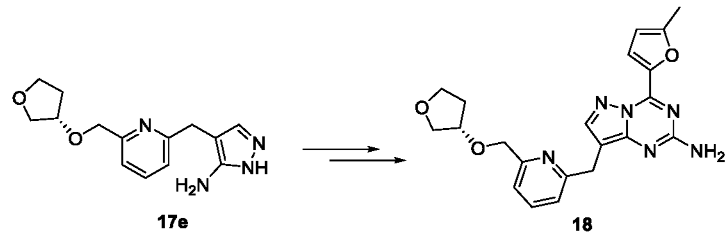

17e 18

With reference to the preparation method of compound **2,** compound **18** may be prepared from compound **17e** by the corresponding steps, and the spectral information about compound **18** was as follows: $^1$H NMR (500 MHz, Methanol-*d4*) $\delta$ 8.28 (d, *J* = 3.5 Hz, 1H), 7.92 (s, 1H), 7.73 (t, *J* = 7.7 Hz, 1H), 7.36 (d, *J* = 7.7 Hz, 1H), 7.22 (d, *J* = 7.7 Hz, 1H), 6.47 (d, *J* = 3.5 Hz, 1H), 4.66 - 4.60 (m, 2H), 4.35 - 4.33 (m, 1H), 4.12 (s, 2H), 3.95 - 3.90 (m, 2H), 3.86 - 3.85 (m, 2H), 2.51 (s, 3H), 2.05-2.04 (m, 2H); MS 407.1 [M+H]$^+$.

Example 19:

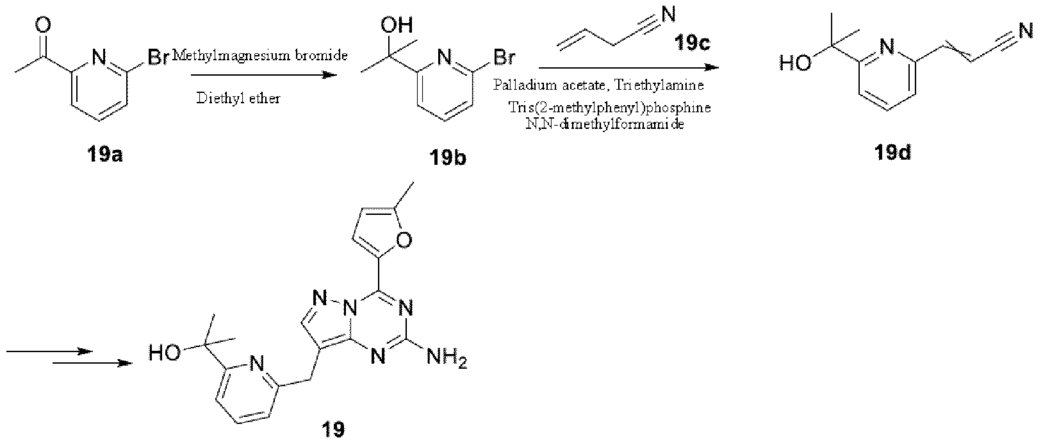


19a 19b 19d

19

Compound **19a** (5.00 g, 25.0 mmol) was dissolved in anhydrous diethyl ether (80 mL), whereupon methylmagnesium bromide (3 M in tetrahydrofuran, 12 mL) was added dropwise at 0°C. The reaction solution was agitated for 1 hour at 0°C, added to water (100 mL), and extracted with ethyl acetate (100 mL × 2). The organic phase separated by extraction was dried over anhydrous sodium sulfate, filtered, and concentrated under vacuum to give the desired product **19b** as a yellow oil (5.25 g, yield: 97%). $^1$H NMR (500 MHz, Chloroform-*d*) $\delta$ 7.55 (t, *J* = 7.7 Hz, 1H), 7.38 - 7.34 (m, 2H), 4.07 (s, 1H), 1.54 (s, 6H); MS: 216.3 [M+H]$^+$.

Compound **19b** (3.95 g, 18.3 mmol) and compound **19c** (1.94 g, 36.6 mmol, 2.41 mL) were dissolved in *N,N*-dimethylformamide (30 mL), followed by the addition of triethylamine (3.70 g, 36.6 mmol, 5.07 mL), tris(2-methylphenyl)phosphine (1.11 g, 3.66 mmol), and palladium acetate (410 mg, 1.83 mmol). The reaction system was repeatedly purged with nitrogen gas 3 times, and agitated for 18 hours at 100°C. The reaction was filtered through kieselguhr, added to water (100 mL), and extracted with ethyl acetate (100 mL × 2). The combined organic phase was washed with water (100 mL × 2) and saturated saline (50 mL), dried over anhydrous sodium sulfate, concentrated under vacuum to give a crude product, which was purified by column chromatography (ethyl acetate/petroleum ether = 1/2) to give yellow oily liquid **19d** (3.2 g, yield: 93%). MS: 189.5 [M+H]$^+$.

With reference to the preparation method of compound **2,** compound **19** may be prepared from compound **19d** by the corresponding steps, and the spectral information about compound **19d** was as follows: $^1$H NMR (500 MHz, Methanol-*d4*) $\delta$ 8.28 (d, *J* = 3.5 Hz, 1H), 7.96 (s, 1H), 7.68 (t, *J* = 7.8 Hz, 1H), 7.43 (d, *J* = 7.8 Hz, 1H), 7.14 (d, *J* = 7.8 Hz, 1H), 6.47 (d, *J* = 3.5 Hz, 1H), 4.13 (s, 2H), 2.51 (s, 3H), 1.55 (s, 6H); MS 365.4 [M+H]$^+$.

Example 20:

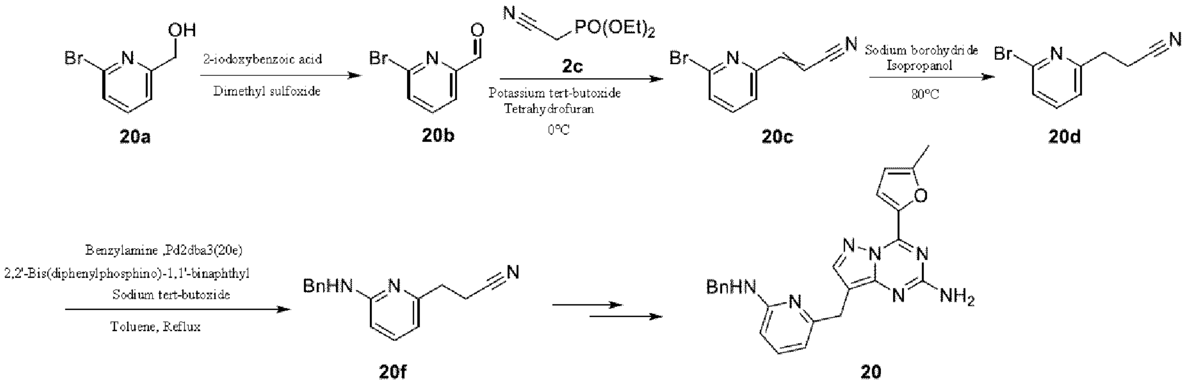


20a 20b 20c 20d 20f 20

Compound **20a** (7.00 g, 37.2 mmol) was dissolved in dimethyl sulfoxide (30 mL), whereupon 2-iodoxybenzoic acid (11.0 g, 39.1 mmol) was added. The reaction was agitated for 2 hours at room temperature, added to water (100 mL), and filtered. The filtrate was extracted with ethyl acetate (100 mL × 2). The organic phase separated by extraction was washed with saturated sodium bicarbonate (100 mL), water (100 mL × 2), and saturated saline (100 mL), dried over anhydrous sodium sulfate, filtered, and concentrated under vacuum to give the desired crude product **20b** as a white solid (6.0 g, yield: 87%). $^1$H NMR (500 MHz, DMSO-*d6*) $\delta$ 9.90 (s, 1H), 8.04 - 7.96 (m, 3H).

Compound **2c** (5.48 g, 31.0 mmol) was dissolved in tetrahydrofuran (50 mL), whereupon potassium tert-butoxide (3.47 g, 31.0 mmol) was added at 0°C. The reaction was agitated for 30 minutes at 0°C, followed by the addition of compound **20b** (5.70 g, 30.6 mmol), the mixture was agitated for 30 minutes at 0°C. The mixture was added to water (100 mL) and extracted with ethyl acetate (100 mL × 2). The organic phase separated by extraction was dried over anhydrous sodium sulfate and concentrated under vacuum to give a pair of Z/E isomer mixtures **20c** as a yellow solid (6.40 g, yield: 99%). MS: 209.3 [M+H]$^+$.

Compound **20c** (5.55 g, 26.6 mmol) was dissolved in isopropanol (15 mL), whereupon sodium borohydride (2.01 g, 53.1 mmol) was added. The reaction solution was agitated for 10 hours at 80°C, added to water (100 mL), and extracted with ethyl acetate (100 mL × 2). The organic phase separated by extraction was dried over anhydrous sodium sulfate, filtered, and concentrated under vacuum to give a crude product, which was purified by column chromatography (ethyl acetate/petroleum ether = 1/1) to give yellow oily liquid **20f** (4.0 g, yield: 52%). MS: 211.3 [M+H]$^+$.

Compound **20d** (500 mg, 2.37 mmol) and benzylamine (381 mg, 3.55 mmol, 388 μL) were dissolved in toluene (10 mL), whereupon catalyst **20e** (108 mg, 118 μmol), 2,2'-bis(diphenylphosphino)-1,1'-binaphthyl (148 mg, 237 μmol) and sodium tert-butoxide (319 mg, 3.32 mmol) were added. The reaction system was repeatedly purged with nitrogen gas 3 times, and agitated for 1.5 hours at 100°C. The reaction solution was filtered through kieselguhr and the filtrate was concentrated under vacuum to obtain a crude product, which was purified by column chromatography (ethyl acetate/petroleum ether = 1/2) to give yellow oily liquid **20f** (522 mg, yield: 93%). $^1$H NMR (500 MHz, Chloroform-d) $\delta$ 7.38-7.35 (m, 5H), 7.31 - 7.29 (m, 1H), 6.52 (d, *J* = 7.2 Hz, 1H), 6.29 (d, *J* = 8.2 Hz, 1H), 4.88 (s, 1H), 4.54 (d, *J* = 5.8 Hz, 2H), 2.97 (t, *J* = 7.4 Hz, 2H), 2.78 (t, *J* = 7.4 Hz, 2H); MS: 238.5 [M+H]$^+$.

With reference to the preparation method of compound **1,** compound **20** may be prepared from compound **20f** by the corresponding steps, and the spectral information about compound **20** was as follows: $^1$H NMR (500 MHz, Methanol-*d4*) $\delta$ 8.36 (d, *J* = 3.5 Hz, 1H), 7.95 (s, 1H), 7.44 - 7.40 (m, 3H), 7.37 - 7.34 (m, 2H), 7.28 (t, *J* = 7.3 Hz, 1H), 6.55 (d, *J* = 3.5 Hz, 1H), 6.54 (d, *J* = 7.3 Hz, 1H), 6.42 (d, *J* = 7.3 Hz, 1H), 4.59 (s, 2H), 4.00 (s, 2H), 2.59 (s, 3H); MS 412.4 [M+H]$^+$.

Example 21:

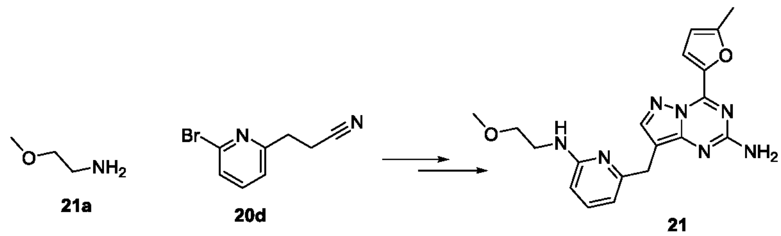

21a 20d 21

With reference to the preparation method of compound **20,** compound **21** may be prepared from compound **20d** and compound **21a** by the corresponding steps, and the specific spectral information was as follows: $^1$H NMR (500 MHz, Methanol-*d4*) $\delta$ 8.36 (d, *J* = 3.5 Hz, 1H), 8.02 (s, 1H), 7.48 (t, *J* = 8.0 Hz, 1H), 6.57 - 6.55 (m, 2H), 6.50 (d, *J* = 8.5 Hz, 1H), 4.02 (s, 2H), 3.66 (t, *J* = 5.4 Hz, 2H), 3.57 (t, *J* = 5.4 Hz, 2H), 3.44 (s, 3H), 2.59 (s, 3H); MS 380.4 [M+H]$^+$.

Example 22:

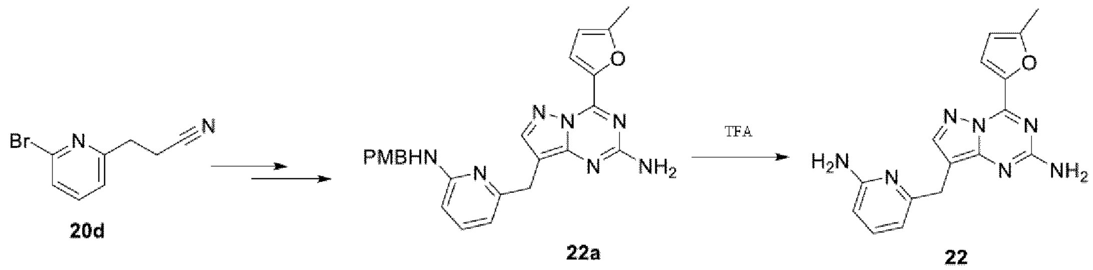


20d 22a 22

With reference to the preparation method of compound **20,** compound 22a may be prepared from compound **20d** and p-methoxybenzylamine by the corresponding steps, and the spectral information was as follows: MS 442.5 [M+H]$^+$.

Compound **22a** (40 mg, 90.6 μmol) was dissolved in trifluoroacetic acid (1 mL). The reaction solution was agitated for 2 hours at 25°C, concentrated under vacuum, then diluted with ethyl acetate (30 mL), and washed with saturated aqueous sodium bicarbonate (15 mL). The separated organic phase was washed with saturated saline solution (15 mL), dried over anhydrous sodium sulfate, and concentrated to obtain a crude product, which was purified by preparative HPLC to give the product **22** as a yellow solid (15 mg, yield: 52%). $^1$H NMR (500 MHz, Methanol-*d4*) $\delta$ 8.27 (d, *J* = 3.5 Hz, 1H), 7.93 (s, 1H), 7.62 (t, *J* = 8.0 Hz, 1H), 6.65 (d, *J* = 8.0 Hz, 1H), 6.60 (d, *J* = 8.0 Hz, 1H), 6.45 (d, *J* = 3.5 Hz, 1H), 4.00 (s, 2H), 2.48 (s, 3H); MS 322.3 [M+H]$^+$.

Example 23:

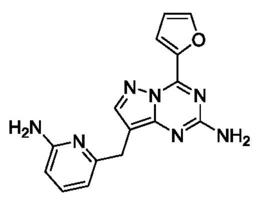

23

With reference to the preparation method of compound **22,** compound **23** may be prepared by commercially available reagents, and the specific spectral information was as follows: $^1$H NMR (500 MHz, Methanol-*d4*) $\delta$ 8.35 (d, *J* = 3.5 Hz, 1H), 7.98 (s, 1H), 7.97 (d, *J* = 1.8 Hz, 1H), 7.66 (t, *J* = 8.0 Hz, 1H), 6.81 (dd, *J* = 3.5, 1.8 Hz, 1H), 6.69 (d, *J* = 8.0 Hz, 1H), 6.63 (d, *J* = 8.0 Hz, 1H), 4.02 (s, 2H); MS 308.3 [M+H]$^+$.

Example 24:

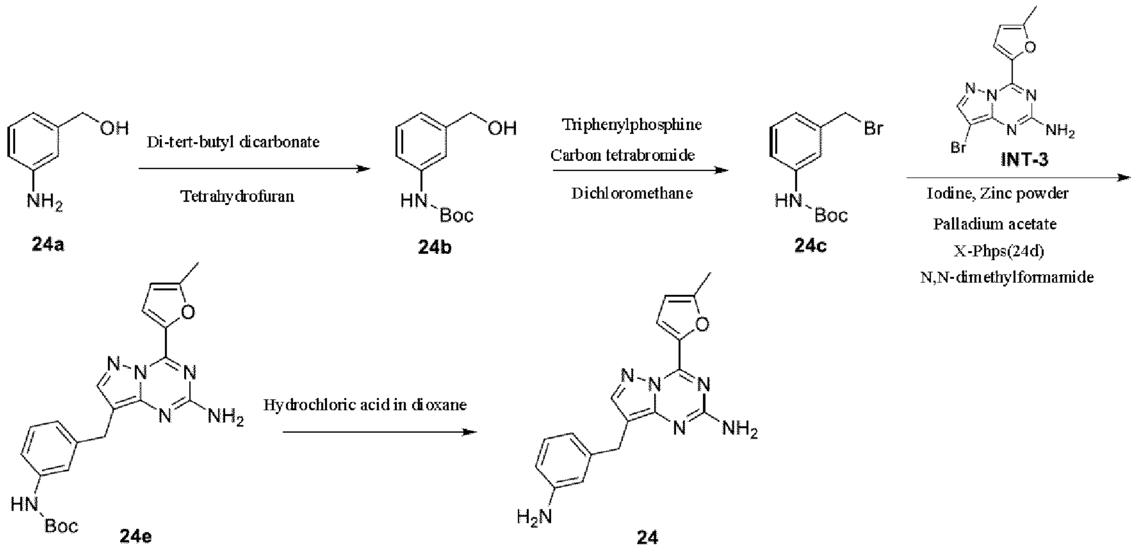


24a 24b 24c 24e 24

Compound **24a** (500 mg, 4.06 mmol) was dissolved in tetrahydrofuran (5 mL), whereupon di-tert-butyl dicarbonate (975 mg, 4.47 mmol, 1.03 mL) was added. The reaction solution was agitated for 18 hours at 30°C, added to water (40 mL), and extracted with ethyl acetate (30 mL × 2). The separated organic phase was dried over anhydrous sodium sulfate and concentrated under vacuum to give crude product **24b** as a yellow oil (906 mg, yield: 99%). $^1$H NMR (500 MHz, Chloroform-d) $\delta$ 7.45 (s, 1H), 7.28 (t, *J* = 7.5 Hz, 1H), 7.22 (d, *J* = 7.5 Hz, 1H), 7.04 (d, *J* = 7.5 Hz, 1H), 6.49 (s, 1H), 4.67 (s, 2H), 1.52 (s, 9H).

Compound **24b** (150 mg, 672 μmol) and carbon tetrabromide (290 mg, 874 μmol) were dissolved in dichloromethane (5 mL), whereupon triphenylphosphine (229 mg, 874 μmol) was added. The reaction solution was agitated for 3 hours at 30°C, and concentrated under vacuum to obtain a crude product, which was purified by column chromatography (ethyl acetate/petroleum ether = 1/10) to give the product **24c** as a white solid (140 mg, yield: 73%). $^1$H NMR (500 MHz, Chloroform-d) $\delta$ 7.51 (s, 1H), 7.27 - 7.25 (m, 1H), 7.21-7.19 (m, 1H), 7.07 - 7.05 (m, 1H), 6.48 (s, 1H), 4.46 (s, 2H), 1.52 (s, 9H).

To a suspension of zinc powder (48.0 mg, 734 μmol) in *N,N*-dimethylformamide (3 mL) under nitrogen was added iodine (8.99 mg, 35.43 μmol), the mixture was agitated at 30°C until the solution faded to colorless, followed by the dropwise addition of a solution of compound **24c** (140 mg, 489 μmol) in *N,N*-dimethylformamide (3 mL). The reaction solution was agitated for 1 hour at 30°C. Compound **INT-3** (71.94 mg, 244.61 μmol), palladium acetate (71.9 mg, 244.6 μmol) and ligand **24d** (23.3 mg, 48.9 μmol) were added to the reaction system. The reaction system was repeatedly purged with nitrogen gas 3 times and then agitated for 40 minutes at 30°C. The reaction solution was poured into water (50 mL) and the aqueous phase was extracted with ethyl acetate (25 mL × 2). The organic layers were combined, washed with saturated saline solution, dried over anhydrous sodium sulfate, and filtered, and the filtrate was concentrated under vacuum to give a crude product, which was purified by column chromatography (ethyl acetate/petroleum ether = 1/1) to give the product **24e** as a yellow solid (32 mg, yield: 31%). MS: 421.5 [M+H]$^+$.

Compound **24e** (32 mg, 76.1 μmol) was dissolved in dichloromethane (0.5 mL), whereupon 4 M hydrochloric acid in dioxane (1.52 mL) was added dropwise. The reaction solution was agitated for 3 hours at 25°C, then concentrated under vacuum and pulped (ethyl acetate, 10 mL) to give the desired product **24** as a white solid (18 mg, yield: 66%). $^1$H NMR (500 MHz, Methanol-*d4*) $\delta$ 8.47 (d, *J* = 3.7 Hz, 1H), 7.97 (s, 1H), 7.49 (t, *J* = 7.8 Hz, 1H), 7.43 (d, *J* = 7.8 Hz, 1H), 7.32 (t, *J* = 1.9 Hz, 1H), 7.25 (d, *J* = 7.8 Hz, 1H), 6.58 (d, *J* = 3.7 Hz, 1H), 4.10 (s, 2H), 2.52 (s, 3H); MS: 321.3 [M+H]$^+$.

Example 25:

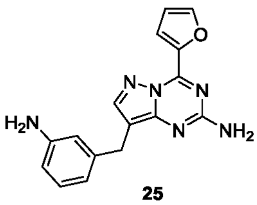

25

With reference to the preparation method of compound **24,** compound **25** may be prepared from commercially available reagents by the corresponding steps, and the spectral information about compound **25** was as follows: $^1$H NMR (500 MHz, Methanol-*d4*) $\delta$ 8.48 (d, *J* = 3.5 Hz, 1H), 8.10 (d, *J* = 1.8 Hz, 1H), 7.98 (s, 1H), 7.51 - 7.43 (m, 2H), 7.30 (s, 1H), 7.28 - 7.22 (m, 1H), 6.89 (dd, *J* = 3.5, 1.8 Hz, 1H), 4.09 (s, 2H); MS 306.9 $[M+H]^+$.

Example 26:

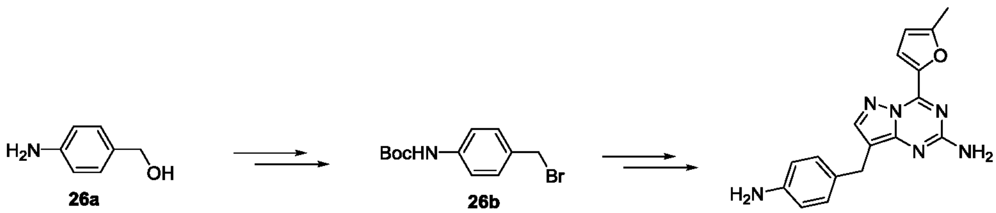

26a 26b 26

With reference to the preparation method of compound **24,** compound **26** may be prepared from compound **26a** by the corresponding steps, and the spectral information about related intermediate **26b** and compound **26** was as follows:

Compound **26b:** $^1$H NMR (500 MHz, Chloroform-*d*) $\delta$ 7.38 - 7.29 (m, 4H), 6.50 (s, 1H), 4.48 (s, 2H), 1.51 (s, 9H).
Compound **26:** $^1$H NMR (500 MHz, Methanol-*d4*) $\delta$ 8.48 (d, *J* = 3.7 Hz, 1H), 7.95 (s, 1H), 7.49 - 7.45 (m, 2H), 7.36 - 7.32 (m, 2H), 6.58 (d, *J* = 3.7 Hz, 1H), 4.07 (s, 2H), 2.52 (s, 3H); MS 320.9 $[M+H]^+$.

Example 27:

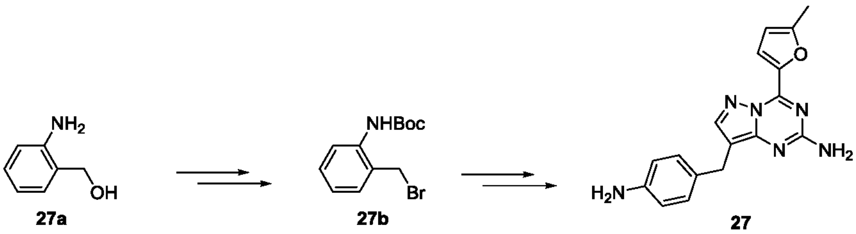

27a 27b 27

With reference to the preparation method of compound **24,** compound **27** may be prepared from compound **27a** by the corresponding steps, and the spectral information about related intermediate **27b** and compound **27** was as follows:

Compound **27b:** MS 285.9 $[M+H]^+$.
Compound **27:** $^1$H NMR (500 MHz, DMSO-*d6*) $\delta$ 8.17 (d, *J* = 3.5 Hz, 1H), 7.89 (s, 1H), 7.20 (s, 2H), 6.89 - 6.81 (m, 2H), 6.61 - 6.56 (m, 1H), 6.51 (d, *J* = 3.5 Hz, 1H), 6.46 - 6.40 (m, 1H), 4.95 (s, 2H), 3.62 (s, 2H), 2.44 (s, 3H); MS 321.2 $[M+H]^+$.

Example 28:

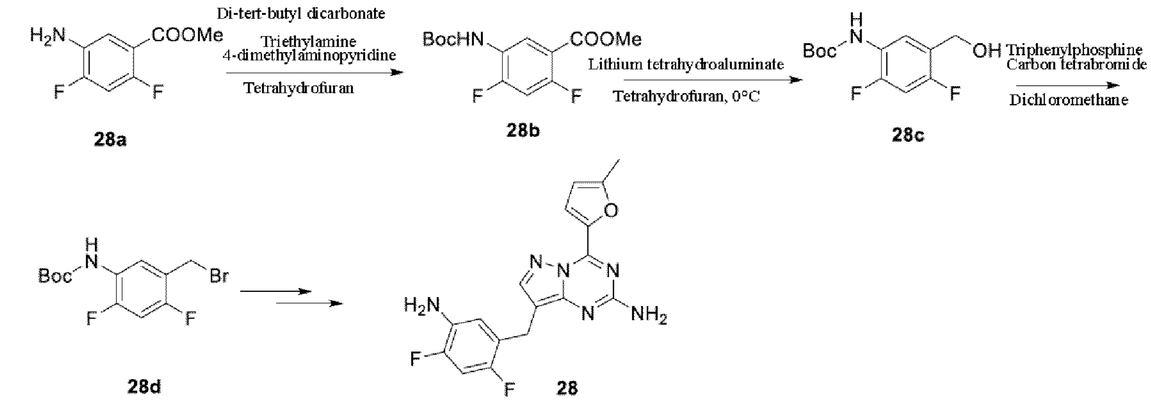


28a 28b 28c 28d 28

Compound **28a** (500 mg, 2.67 mmol) was dissolved in tetrahydrofuran (20 mL), whereupon triethylamine (811 mg, 8.02 mmol, 1.11 mL), 4-dimethylamino pyridine (326 mg, 2.67 mmol) and di-tert-butyl dicarbonate (700 mg, 3.21 mmol, 737 $\mu$L) were added. The reaction solution was agitated for 2 hours at 30°C. The reaction solution was concentrated under vacuum to give a crude product as a yellow oil, which was purified by column chromatography (ethyl acetate/petroleum ether = 1/4) to give the product **28b** as a white solid (598 mg, yield: 78%). $^1$H NMR (500 MHz, Chloroform-d) $\delta$ 8.64 (s, 1H), 6.93 - 6.90 (m, 1H), 6.60 (s, 1H), 3.91 (s, 3H), 1.53 (s, 9H). Compound **28b** (100 mg, 348 $\mu$mol) was dissolved in tetrahydrofuran (3 mL), whereupon lithium tetrahydroaluminate (14.5 mg, 383 $\mu$mol) was carefully added portion-wise at 0°C. The reaction solution was agitated for 2 hours at 0°C, to which were carefully added water (0.1 mL), 15% aqueous sodium hydroxide solution (0.1 mL), and water (0.3 mL) in sequence. The reaction solution was filtered through kieselguhr and the filtrate was concentrated to give the product **28c** as a white solid (90 mg, yield: 99%). $^1$H NMR (500 MHz, Chloroform-*d*) $\delta$ 8.13 (s, 1H), 6.85 - 6.81 (m, 1H), 6.57 (s, 1H), 4.70 (s, 2H), 1.86 (s, 1H), 1.52 (s, 9H).

Compound **28c** (90 mg, 347 $\mu$mol) and carbon tetrabromide (173 mg, 521 $\mu$mol) were dissolved in dichloromethane (5 mL), whereupon triphenylphosphine (109 mg, 417 $\mu$mol) was added. The reaction solution was agitated for 3 hours at 30°C, and concentrated under vacuum to obtain a crude product, which was purified by column chromatography (ethyl acetate/petroleum ether = 1/10) to give the product **28d** as a white solid (85 mg, yield: 76%). $^1$H NMR (500 MHz, Chloroform-d) $\delta$ 8.17 (s, 1H), 6.87 - 6.83 (m, 1H), 6.60 (s, 1H), 4.46 (s, 2H), 1.53 (s, 9H).

With reference to the preparation method of compound **24,** compound **28** may be prepared from compound **28d** by the corresponding steps, and the spectral information about compound **28** was as follows: $^1$H NMR (500 MHz, DMSO-*d6*) $\delta$ 8.18 (d, *J* = 3.5 Hz, 1H), 7.88 (s, 1H), 7.20 (s, 2H), 6.97 - 6.91 (m, 1H), 6.52 (d, *J* = 3.5 Hz, 1H), 6.50 - 6.44 (m, 1H), 4.83 (s, 2H), 3.72 (s, 2H), 2.44 (s, 3H); MS 357.2 [M+H]$^+$.

Example 29:

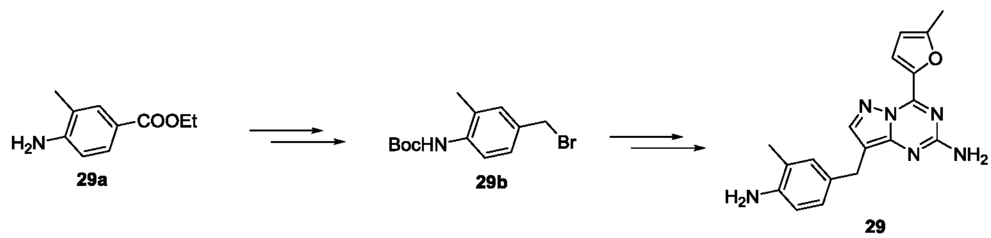

29a 29b 29

With reference to the preparation method of compound **28,** compound **29** may be prepared from compound **29a** by the corresponding steps, and the spectral information about related intermediate **29b** and compound **29** was as follows:

Compound **29b:** $^1$H NMR (500 MHz, Chloroform-*d*) $\delta$ 7.84 (d, *J* = 7.2 Hz, 1H), 7.24 - 7.16 (m, 2H), 6.29 (s, 1H), 4.46 (s, 2H), 2.23 (s, 3H), 1.52 (s, 9H).

Compound **29:** $^1$H NMR (500 MHz, Methanol-*d4*) $\delta$ 8.50 (d, *J* = 3.5 Hz, 1H), 7.96 (s, 1H), 7.34 - 7.25 (m, 3H), 6.59 (d, *J* = 3.5 Hz, 1H), 4.03 (s, 2H), 2.52 (s, 3H), 2.38 (s, 3H); MS 335.3 [M+H]$^+$.

Example 30:

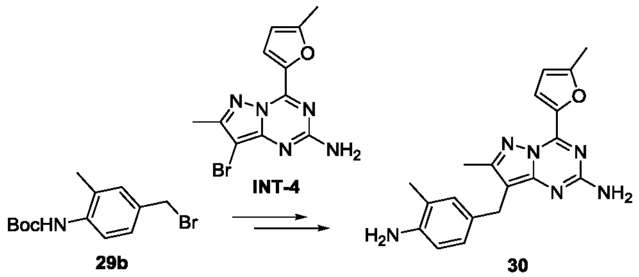

INT-4

29b

30

With reference to the preparation method of compound **29,** compound **30** may be prepared from compound **29b** and **INT-4** by the corresponding steps, and the spectral information about compound **30** was as follows: $^1$H NMR (500 MHz, DMSO-*d6*) $\delta$ 8.19 (d, *J* = 3.6 Hz, 1H), 7.05 (s, 2H), 6.72 (s, 1H), 6.69 (d, *J* = 7.9 Hz, 1H), 6.50 (d, *J* = 3.6 Hz, 1H), 6.45 (d, *J* = 7.9 Hz, 1H), 4.55 (s, 2H), 3.62 (s, 2H), 2.43 (s, 3H), 2.18 (s, 3H), 1.95 (s, 3H); MS 349.5 [M+H]$^+$.

Example 31:

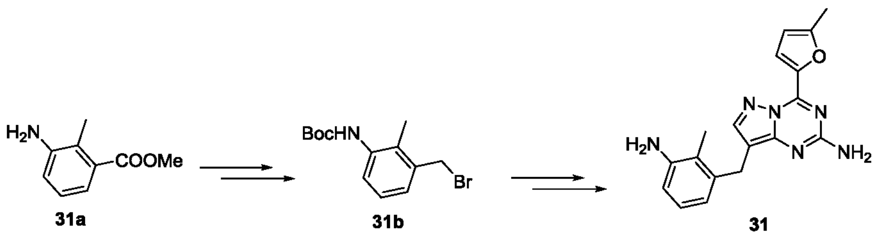

31a

31b

31

With reference to the preparation method of compound **28,** compound **31** may be prepared from compound **31a** by the corresponding steps, and the spectral information about related intermediate **31b** and compound **31** was as follows:

Compound **31b:** $^1$H NMR (500 MHz, Chloroform-*d*) $\delta$ 7.71 (d, *J* = 7.7 Hz, 1H), 7.16 (t, *J* = 7.7 Hz, 1H), 7.09 (d, *J* = 7.7 Hz, 1H), 6.27 (s, 1H), 4.52 (s, 2H), 2.27 (s, 3H), 1.52 (s, 9H).

Compound **31:** $^1$H NMR (500 MHz, DMSO-*d6*) $\delta$ 8.16 (d, *J* = 3.5 Hz, 1H), 7.78 (s, 1H), 7.19 - 7.03 (m, 3H), 6.51 (d, *J* = 3.5 Hz, 1H), 3.85 (s, 2H), 2.44 (s, 3H), 2.24 (s, 3H); MS 335.3 [M+H]$^+$.

Example 32:

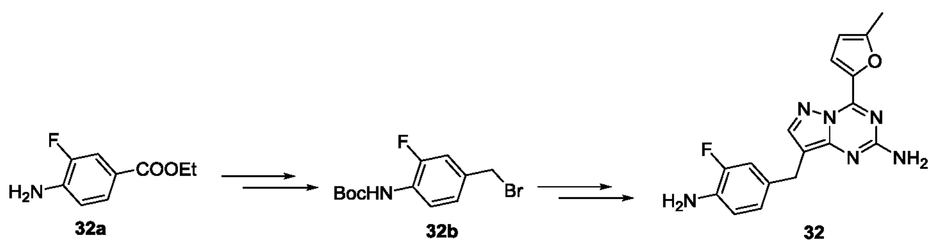

32a

32b

32

With reference to the preparation method of compound **28,** compound **32** may be prepared from compound

**32a** by the corresponding steps, and the spectral information about related intermediate **32b** and compound **32** was as follows:

Compound **32b:** $^1$H NMR (500 MHz, Chloroform-*d*) $\delta$ 8.13 - 7.98 (m, 1H), 7.15 - 7.08 (m, 2H), 6.72 (s, 1H), 4.44 (s, 2H), 1.52 (s, 9H).
Compound **32:** $^1$H NMR (500 MHz, DMSO-*d6*) $\delta$ 8.16 (d, *J* = 3.5 Hz, 1H), 7.88 (s, 1H), 7.18 (s, 2H), 6.84 - 6.80 (m, 1H), 6.74 - 6.70 (m, 1H), 6.66 - 6.60 (m, 1H), 6.51 (d, *J* = 3.5 Hz, 1H), 4.87 (s, 2H), 3.68 (s, 2H), 2.43 (s, 3H); MS 339.4 $[M+H]^+$.

Example 33:

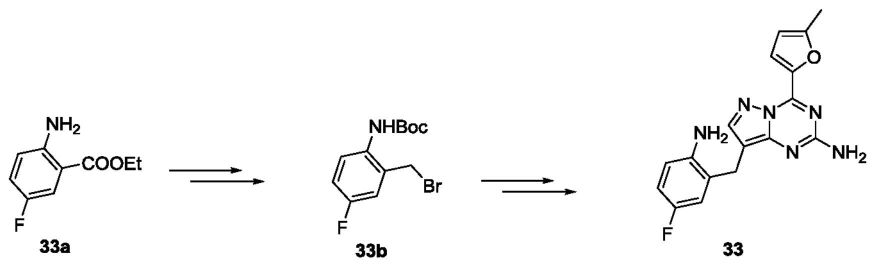

33a 33b 33

With reference to the preparation method of compound **28,** compound **33** may be prepared from compound **33a** by the corresponding steps, and the spectral information about related intermediate **33b** and compound **33** was as follows:

Compound **33b:** $^1$H NMR (500 MHz, Chloroform-*d*) $\delta$ 7.72 (s, 1H), 7.08 - 6.99 (m, 2H), 6.52 (s, 1H), 4.44 (s, 2H), 1.53 (s, 9H).
Compound **33:** $^1$H NMR (500 MHz, DMSO-*d6*) $\delta$ 8.19 (d, *J* = 3.5 Hz, 1H), 8.04 (s, 1H), 7.59 - 7.53 (m, 1H), 7.17-7.11 (m, 1H), 7.09 - 7.05 (m, 1H), 6.54 (d, *J* = 3.5 Hz, 1H), 4.00 (s, 2H), 2.44 (s, 3H); MS 339.4 $[M+H]^+$.

Example 34:

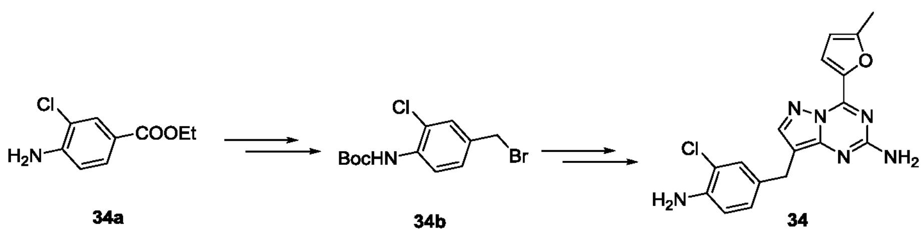

34a 34b 34

With reference to the preparation method of compound **28,** compound **34** may be prepared from compound **34a** by the corresponding steps, and the spectral information about related intermediate **34b** and compound **34** was as follows:

Compound **34b:** $^1$H NMR (500 MHz, Chloroform-*d*) $\delta$ 8.15 (d, *J* = 8.6 Hz, 1H), 7.38 (s, 1H), 7.26 (d, *J* = 6.5 Hz, 1H), 7.03 (s, 1H), 4.42 (s, 2H), 1.53 (s, 9H).
Compound **34:** $^1$H NMR (500 MHz, Methanol-*d4*) $\delta$ 8.43 (d, *J* = 3.6 Hz, 1H), 7.96 (s, 1H), 7.47 (s, 1H), 7.32 - 7.28 (m, 2H), 6.55 (d, *J* = 3.6 Hz, 1H), 4.00 (s, 2H), 2.51 (s, 3H); MS 355.2 $[M+H]^+$.

Example 35:

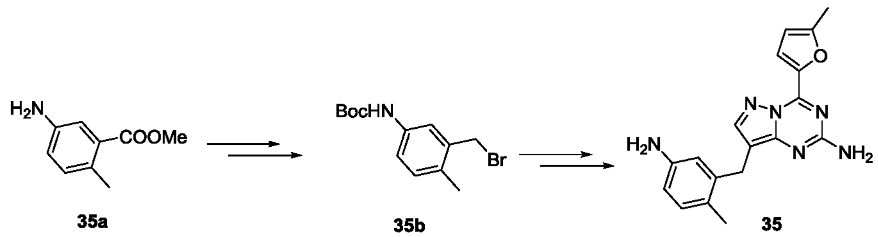

35a 35b 35

With reference to the preparation method of compound **28,** compound **35** may be prepared from compound **35a** by the corresponding steps, and the spectral information about related intermediate **35b** and compound **35** was as follows:

Compound **35b:** $^1$H NMR (500 MHz, Chloroform-*d*) $\delta$ 7.44 (s, 1H), 7.16 - 7.05 (m, 2H), 6.42 (s, 1H), 4.47 (s, 2H), 2.34 (s, 3H), 1.51 (s, 9H).

Compound **35:** $^1$H NMR (500 MHz, DMSO-*d6*) $\delta$ 8.18 (d, *J* = 3.5 Hz, 1H), 7.76 (s, 1H), 7.16 (s, 2H), 6.76 (d, *J* = 7.9 Hz, 1H), 6.52 (d, *J* = 3.5 Hz, 1H), 6.28 (dd, *J* = 7.9, 2.3 Hz, 1H), 6.24 (d, *J* = 2.3 Hz, 1H), 4.67 (s, 2H), 3.65 (s, 2H), 2.44 (s, 3H), 2.11 (s, 3H); MS 335.4 $[M+H]^+$.

Example 36:

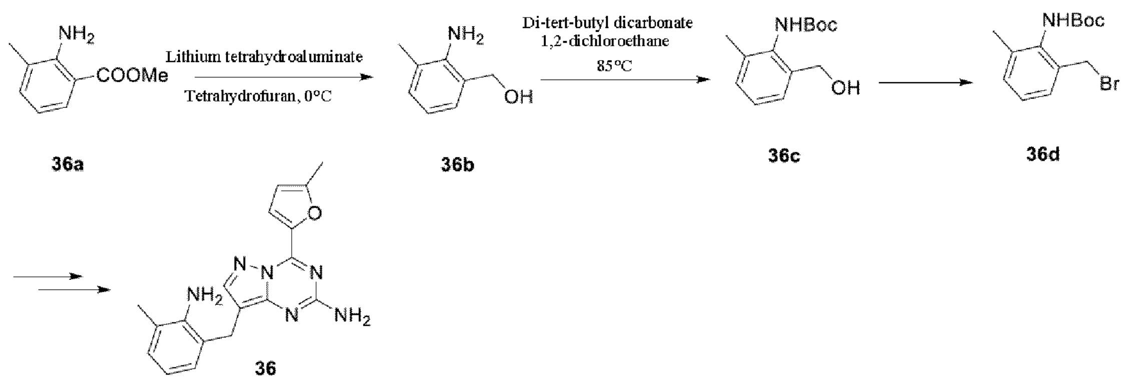


36a 36b 36c 36d

36

Compound **36a** (500 mg, 3.03 mmol) was dissolved in tetrahydrofuran (10 mL), whereupon lithium tetrahydroaluminate (345 mg, 9.08 mmol) was carefully added portion-wise at 0°C. The reaction solution was agitated for 2 hours at 0°C, to which were carefully added water (0.34 mL), 15% aqueous sodium hydroxide solution (0.34 mL), and water (1.02 mL) in sequence. The reaction solution was filtered through kieselguhr and the filtrate was concentrated to give the product **36b** as a white solid (410 mg, yield: 98%). $^1$H NMR (500 MHz, Chloroform-*d*) $\delta$ 6.99 - 6.91 (m, 2H), 6.59 (t, *J* = 7.5 Hz, 1H), 4.58 (s, 2H), 2.17 (s, 3H).

Compound **36b** (400 mg, 2.92 mmol) was dissolved in 1, 2-dichloroethane (8 mL), whereupon di-tert-butyl dicarbonate (700 mg, 3.21 mmol, 0.74 mL) was added. The reaction solution was agitated overnight at 85°C, and concentrated under vacuum to obtain a crude product, which was purified by column chromatography (ethyl acetate/petroleum ether = 1/3) to give the product **36c** as a white solid (534 mg, yield: 77%). $^1$H NMR (500 MHz, Chloroform-*d*) $\delta$ 7.25 (d, *J* = 7.2 Hz, 1H), 7.21 - 7.14 (m, 2H), 6.38 (s, 1H), 4.57 (s, 2H), 2.28 (s, 3H), 1.51 (s, 9H).

With reference to the preparation method of compound **28,** compound **36** may be prepared from compound **36c** by the corresponding steps, and the spectral information about related intermediate **36d** and compound **36** was as follows:

Compound **36d:** $^1$H NMR (500 MHz, Chloroform-*d*) $\delta$ 7.25 - 7.19 (m, 2H), 7.18 - 7.12 (m, 1H), 6.21 (s, 1H), 4.52 (s, 2H), 2.29 (s, 3H), 1.52 (s, 9H).

Compound **36:** $^1$H NMR (500 MHz, DMSO-*d6*) $\delta$ 8.17 (d, *J* = 3.5 Hz, 1H), 7.87 (s, 1H), 7.21 (s, 2H), 6.78 (d, *J* = 7.5, 2H), 6.51 (d, *J* = 3.5 Hz, 1H), 6.39 (t, *J* = 7.5 Hz, 1H), 4.64 (s, 2H), 3.65 (s, 2H), 2.44 (s, 3H), 2.04 (s, 3H); MS 335.2 $[M+H]^+$.

Example 37:

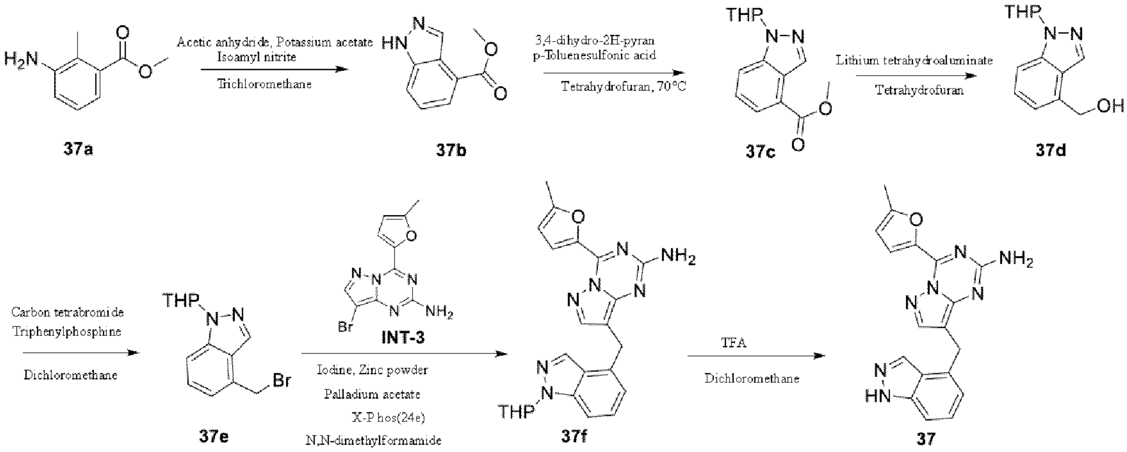


Compound **37a** (190 mg, 1.15 mmol) was dissolved in trichloromethane (2 mL), whereupon acetic anhydride (270.07 mg, 2.65 mmol, 250.06 μL) was added at 0°C. The reaction solution was agitated for 5 minutes at 0°C and then heated to room temperature for 1 hour. Potassium acetate (at 0°C) and isoamyl nitrite (288 mg, 2.46 mmol, 0.33 mL) were then added and heated to reflux for 20 hours. The reaction solution was concentrated, followed by the addition of concentrated hydrochloric acid (0.5 mL), and the mixture was agitated for 2 hours at 50°C. The reaction solution was adjusted to pH=14 with 50% aqueous sodium hydroxide in an ice bath to precipitate a large amount of solid. The solid was filtered, washed with water, and dried under vacuum to give the compound **37b** as a white solid (100 mg, yield: 49%). MS: 177.2 $[M+H]^+$.

Compound **37b** (200 mg, 1.14 mmol) was dissolved in tetrahydrofuran (2 mL), whereupon p-toluenesulfonic acid (21.6 mg, 114 μmol) and 3, 4-dihydro-2*H*-pyran (143 mg, 1.70 mmol, 155 μL) were added. The reaction solution was agitated for 1 hour at 70°C, and concentrated to obtain a crude product, which was purified by column chromatography (ethyl acetate/petroleum ether = 1/15) to give the product **37c** as a white solid (200 mg, yield: 68%). MS: 261.1 $[M+H]^+$. Compound **37c** (630 mg, 2.42 mmol) was dissolved in tetrahydrofuran (12 mL), whereupon lithium tetrahydroaluminate (138 mg, 3.63 mmol) was carefully added portion-wise at 0°C. The reaction solution was agitated for 2 hours at 0°C, to which were carefully added water (0.14 mL), 15% aqueous sodium hydroxide solution (0.14 mL), and water (0.42 mL) in sequence. The reaction solution was filtered through kieselguhr and the filtrate was concentrated to give the product **37d** as a white solid (560 mg, yield: 99%).

Compound **37d** (250 mg, 1.08 mmol) and carbon tetrabromide (393mg, 1.18 mmol) were dissolved in dichloromethane (5 mL), whereupon triphenylphosphine (339 mg, 1.29 mmol) was added. The reaction solution was agitated for 1 hour at 30°C, and concentrated under vacuum to obtain a crude product, which was purified by column chromatography (ethyl acetate/petroleum ether = 1/10) to give the product **37e** as a white solid (100 mg, yield: 31%). MS: 297.1 $[M+H]^+$. To a suspension of zinc powder (66.5 mg, 1.02 mmol) in *N*,*N*-dimethylformamide (5 mL) under nitrogen was added iodine (860 μg, 3.39 μmol), the mixture was agitated at 30°C until the solution faded to colorless, followed by the dropwise addition of a solution of compound **37e** (200 mg, 677.57 μmol) in *N*,*N*-dimethylformamide (3 mL). The reaction solution was agitated for 1 hour at 30°C. Compound **INT-3** (99.6 mg, 339 μmol), palladium acetate (7.61 mg, 33.9 μmol) and ligand **24e** (32.3 mg, 67.8 μmol) were added to the reaction system. The reaction system was repeatedly purged with nitrogen gas 3 times, and then agitated for 40 minutes at 30°C. The reaction solution was poured into water (50 mL) and the aqueous phase was extracted with ethyl acetate (25 mL × 2). The organic layers were combined, washed with saturated saline solution, dried over anhydrous sodium sulfate, and filtered, and the filtrate was concentrated under vacuum to give a crude product, which was purified by column chromatography (ethyl acetate/petroleum ether = 1/1) to give the product **37f** as a yellow solid (60 mg, yield: 41%).

Compound **37f** (49 mg, 114.17 μmol) was dissolved in dichloromethane (3 mL), whereupon trifluoroacetic acid (1 mL) was added dropwise. The reaction solution was agitated for 3 hours at 30°C, concentrated under vacuum, then diluted with ethyl acetate (20 mL), and washed with saturated aqueous sodium bicarbonate (10 mL). The separated organic phase was washed with saturated saline solution (10 mL), dried over anhydrous sodium sulfate, and concentrated to obtain a crude product, which was purified by preparative HPLC to give the product **37** as a yellow solid (15 mg, yield: 38%). $^1H$ NMR (500 MHz, DMSO-*d6*) $\delta$ 13.01 (s, 1H), 8.19 (s, 1H), 8.15 (d, *J* = 3.5 Hz, 1H), 7.92 (s, 1H), 7.35 - 7.30 (m, 1H), 7.23 (s, 2H), 7.23 - 7.17 (m, 1H), 6.90 - 6.87 (m, 1H), 6.50 (d, *J* = 3.5 Hz, 1H), 4.13 (s, 2H), 2.43 (s, 3H); MS:

346.3 [M+H]+.

Example 38:

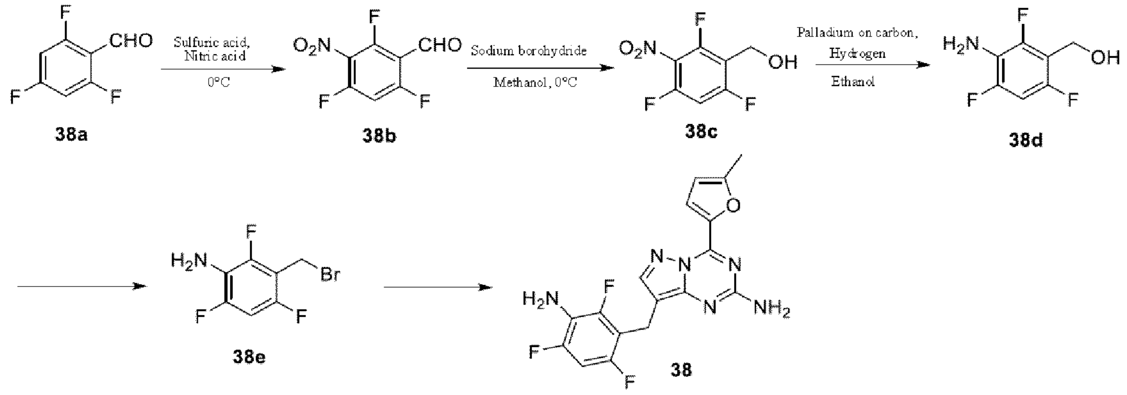


38a 38b 38c 38d 38e 38

Compound **38a** (1.0 g, 6.25 mmol) was dissolved in sulfuric acid (11.6 g, 118 mmol, 6 mL), whereupon nitric acid (6.25 mmol, 4 mL) was added dropwise at 0°C. The reaction solution was agitated for 2 hours at 0°C. The reaction solution was poured into ice water (50 mL) and extracted with dichloromethane (30 mL × 3). The organic layers were combined, washed with saturated saline solution, dried over anhydrous sodium sulfate, and filtered, and the filtrate was concentrated under vacuum to crude product **38b** as a yellow solid (1.25 g, yield: 98%). $^1$H NMR (500 MHz, Chloroform-*d*) $\delta$ 10.11 (s, 1H), 7.91 - 7.85 (m, 1H).

Compound **38b** (1.88 g, 9.17 mmol) was dissolved in a mixed solution of methanol (6 mL) and tetrahydrofuran (6 mL), whereupon sodium borohydride (694 mg, 18.3 mmol) was added. The reaction solution was agitated for 1 hour at 0°C. The reaction was quenched by the addition of water (10 mL) and extracted with ethyl acetate (10 mL × 3). The separated organic phase was dried over anhydrous sodium sulfate and concentrated under vacuum to give crude product **38c** as a yellow oil (1.77 g, yield: 98%). $^1$H NMR (500 MHz, Chloroform-*d*) $\delta$ 6.93 - 6.89 (m, 1H), 4.80 (s, 2H); MS: 328.3 [M+H]+.

Compound **38c** (1.1 g, 5.31 mmol) was dissolved in ethanol (12 mL), whereupon 10% palladium on carbon (200 mg, 1.65 mmol) was added. The reaction was agitatedagitated overnight at room temperature under hydrogen. The reaction solution was filtered, and the filtrate was concentrated under vacuum to give a crude product, which was purified by column chromatography (ethyl acetate/petroleum ether = 1/4) to give **38d** as a white solid (413 mg, yield: 44%). $^1$H NMR (500 MHz, Chloroform-*d*) $\delta$ 6.70 - 6.63 (m, 1H), 4.73 (s, 2H), 3.58 (s, 2H).

With reference to the preparation method of compound **28,** compound **38** may be prepared from compound **38c** by the corresponding steps, and the spectral information about related intermediate **38e** and compound **38** was as follows:

Compound **38e:** $^1$H NMR (500 MHz, Chloroform-*d*) $\delta$ 6.71 - 6.64 (m, 1H), 4.49 (s, 2H), 3.59 (s, 2H).
Compound **38:** $^1$H NMR (500 MHz, DMSO-*d6*) $\delta$ 8.16 (d, *J* = 3.5 Hz, 1H), 7.73 (s, 1H), 7.08 - 6.94 (m, 1H), 6.53 (d, *J* = 3.5 Hz, 1H), 3.80 (s, 2H), 2.45 (s, 3H); MS: 375.2 [M+H]+.

Example 39:

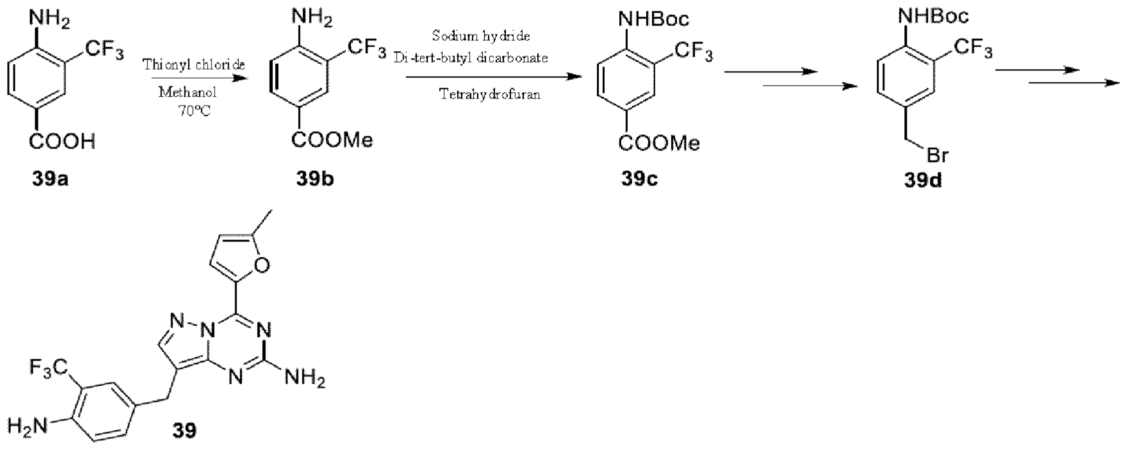


39a 39b 39c 39d

39

Thionyl chloride (0.53 mL, 7.31 mmol) was added dropwise to a solution of compound 39a (500 mg, 2.44 mmol) in methanol (10 mL) in an ice bath. The resulting reaction was agitated for 3 hours at 70°C. The reaction was cooled to room temperature, added to saturated aqueous sodium bicarbonate solution (50 mL), and extracted with ethyl acetate (50 mL × 2). The combined organic phases were washed with saturated saline solution (50 mL), dried over anhydrous sodium sulfate, and filtered. The filtrate was concentrated under reduced pressure to give the compound **39b** as a white solid (520 mg, yield 97%). MS: 200.1 $[M+H]^+$.

To a solution of compound **39b** (200 mg, 0.913 mmol) in tetrahydrofuran (3 mL) was added sodium hydride (purity 60%, dispersed in mineral oil, 54.8 mg, 1.37 mmol) in an ice bath. The resulting reaction solution was agitated for 1 hour in an ice bath, followed by the addition of di-tert-butyl dicarbonate (239 mg, 1.10 mmol), and the resulting reaction solution was agitated for 16 hours at 20°C. The reaction solution was added to saturated aqueous ammonium chloride solution (30 mL) and extracted with ethyl acetate (30 mL× 2). The combined organic phases were washed with saturated saline solution (50 mL), dried over anhydrous sodium sulfate, and filtered. The filtrate was concentrated under reduced pressure to obtain a crude product, which was purified by column chromatography (petroleum ether/ethyl acetate = 10/1) to give the compound **39c** as a white solid (60 mg, yield 20%).

With reference to the preparation method of compound **28,** compound **39** may be prepared from compound **39c** by the corresponding steps, and the spectral information about related intermediate **39d** and compound **39** was as follows:

Compound **39d:** MS: 353.7 $[M+H]^+$.
Compound **39:** $^1$H NMR (500 MHz, Methanol-*d4*) δ 8.25 (d, *J* = 3.5 Hz, 1H), 7.82 (s, 1H), 7.26 (d, *J* = 2.0 Hz, 1H), 7.22 - 7.17 (m, 1H), 6.76 (d, *J* = 8.0 Hz, 1H), 6.45 - 6.42 (m, 1H), 3.83 (s, 2H), 2.48 (s, 3H); MS: 389.3 $[M+H]^+$.

Example 40:

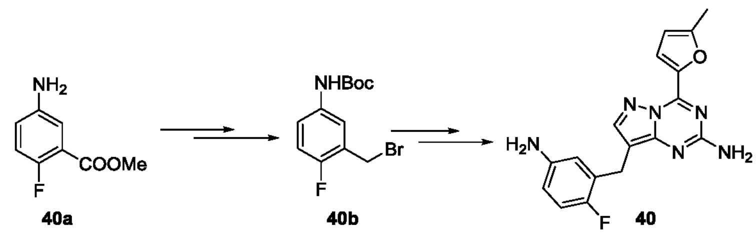

40a 40b 40

With reference to the preparation method of compound **36,** compound **40** may be prepared from compound **40a** by the corresponding steps, and the spectral information about related intermediate **40b** and compound **40** was as follows:

Compound **40b:** $^1$H NMR (500 MHz, Chloroform-*d*) δ7.49 (d, *J* = 7.0 Hz, 1H), 7.23 - 7.17 (m, 1H), 7.00 - 6.94 (m, 1H),6.45 (s, 1H), 4.47 (s, 2H), 1.51 (s, 9H).
Compound **40:** $^1$H NMR (500 MHz, DMSO-*d6*) δ 8.18 (d, *J* = 3.5 Hz, 1H), 7.87 (s, 1H), 7.17 (s, 2H), 6.77 (dd, *J* =

9.9, 8.6 Hz, 1H), 6.52 (d, *J* = 3.5 Hz, 1H), 6.38 - 6.30 (m, 1H), 6.30 - 6.20 (m, 1H), 4.77 (s, 2H), 3.72 (s, 2H), 2.44 (s, 3H); MS: 339.2 $[M+H]^+$.

Example 41:

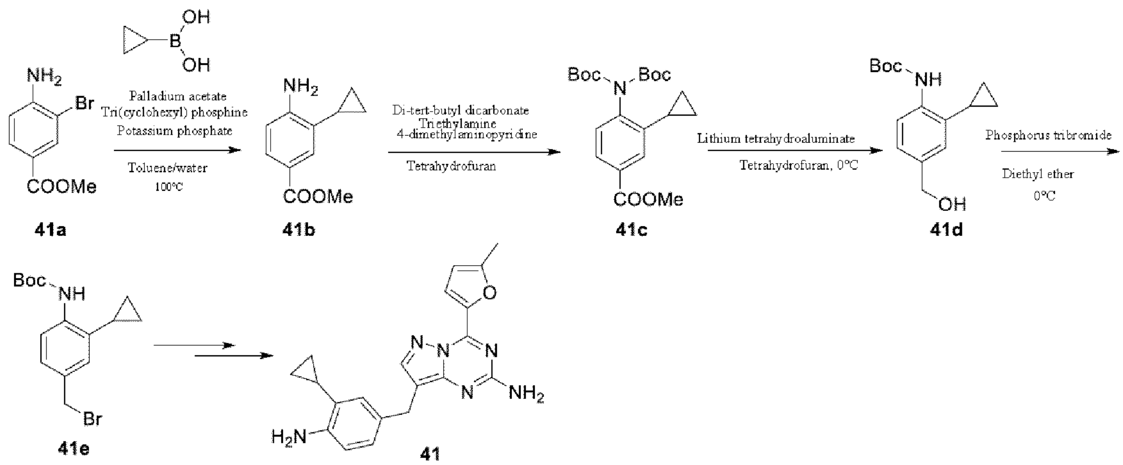


**41a** **41b** **41c** **41d** **41e** **41**

To a mixed solution of compound **41a** (500 mg, 2.17 mmol) in toluene (10 mL)/water (2 mL) were added Palladium acetate (48.8 mg, 0.217 mmol), tris (cyclohexyl) phosphine (61.0 mg, 0.217 mmol) and potassium phosphate (1.38 g, 6.52 mmol). The resulting reaction was agitated for 4 hours at 100°C under nitrogen. The reaction solution was cooled and filtered through kieselguhr, and the filtrate was concentrated under reduced pressure to give a crude product, which was purified by column chromatography (petroleum ether/ethyl acetate = 2/1) to give the compound **41b** as a yellow solid (400 mg, yield 96%). $^1H$ NMR (500 MHz, Chloroform-*d*) $\delta$ 7.79 - 7.73 (m, 2H), 6.71 (d, *J* = 9.0 Hz, 1H), 5.01 (brs, 2H), 3.91 - 3.81 (m, 3H), 1.71 - 1.64 (m, 1H), 0.98 - 0.91 (m, 2H), 0.69 - 0.62 (m, 2H); MS: 192.2 $[M+H]^+$.

To a solution of compound **41b** (300 mg, 1.57 mmol) in tetrahydrofuran (5 mL) were added di-tert-butyl dicarbonate (753 mg, 3.45 mmol), triethylamine (397 mg, 3.92 mmol) and 4-dimethylamino pyridine (192 mg, 1.57 mmol). The resulting reaction was agitated for 2 hours at 20°C. The reaction solution was concentrated under reduced pressure and purified by column chromatography (petroleum ether/ethyl acetate = 10/1) to give the compound **41c** as a white solid (480 m, yield 78%).

To a solution of compound **41c** (300 mg, 0.766 mmol) in tetrahydrofuran (3 mL) was added lithium tetrahydroaluminate (58.2 mg, 1.53 mmol) in an ice bath. The resulting reaction was agitated in an ice bath for 30 minutes and then quenched by adding water (0.06 mL), 15% aqueous sodium hydroxide (0.06 mL), and water in sequence (0.18 mL), and filtered through kieselguhr. The filtrate was concentrated under reduced pressure and purified by column chromatography (petroleum ether/ethyl acetate = 2/1) to give the compound **41d** as a white solid (150 mg, yield 74%). MS: 263.9 $[M+H]^+$.

To a solution of compound **41d** (100 mg, 0.38 mmol) in diethyl ether (3 mL) was dropwise added phosphorus tribromide (103 mg, 0.38 mmol) in an ice bath. The resulting reaction solution was agitated for 1 hour in an ice bath. The reaction solution was diluted with ethyl acetate (30 mL), and washed with saturated aqueous sodium bicarbonate solution (30 mL) followed by saturated saline solution (30 mL). The organic layer phase was dried over anhydrous sodium sulfate and filtered, and the filtrate was concentrated under reduced pressure to give the compound **41e** as a white solid (70 mg, yield 56%).

With reference to the preparation method of compound **28,** compound **41** may be prepared from compound **41e** by the corresponding steps, and the spectral information was as follows: $^1H$ NMR (500 MHz, Methanol-*d4*) $\delta$ 8.23 (d, *J* = 3.5 Hz, 1H), 7.75 (s, 1H), 6.90-6.82 (m, 2H), 6.67-6.61 (m, 1H), 6.43 (d, *J* = 3.5, 1H), 3.77 (s, 2H), 2.48 (s, 3H), 1.67-1.60 (m, 1H), 0.92 - 0.81 (m, 2H), 0.56 - 0.44 (m, 2H); MS: 361.3 $[M+H]^+$.

Example 42:

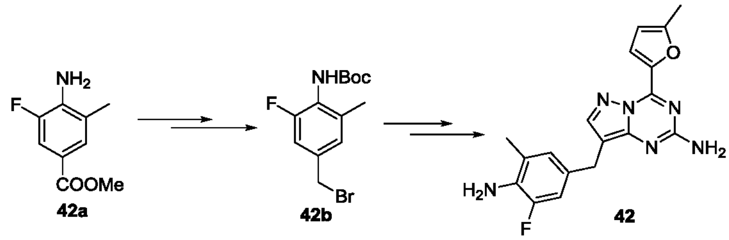

With reference to the preparation method of compound **41,** compound **42** may be prepared from compound **42a** by the corresponding steps, and the spectral information about related intermediate **42b** and compound **42** was as follows:

Compound **42b:** $^1$H NMR (500 MHz, Chloroform-*d*) $\delta$ 7.03 (s, 1H), 7.01 - 6.95 (m, 1H), 5.96 (s, 1H),4.39 (s, 2H), 2.28 (s, 3H), 1.49 (s, 9H).
Compound **42:** $^1$H NMR (500 MHz, DMSO-*d6*) $\delta$ 8.16 (d, *J* = 3.5 Hz, 1H), 6.74 - 6.67 (m, 1H), 6.53 - 6.49 (m, 1H), 4.57 (s, 2H), 3.66 (s, 2H), 2.44 (s, 3H), 2.04 (s, 3H); MS: 353.2 [M+H]$^+$.

Example 43:

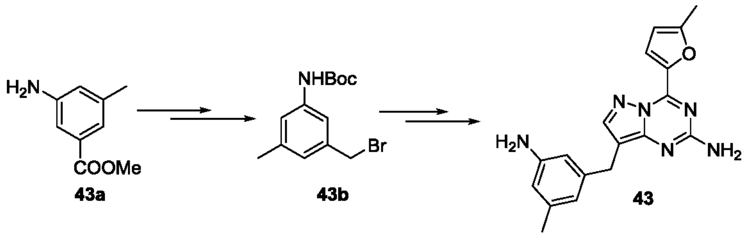

With reference to the preparation method of compound **28,** compound **43** may be prepared from compound **43a** by the corresponding steps, and the spectral information about related intermediate **43b** and compound 4**3** was as follows:

Compound **43b:** $^1$H NMR (500 MHz, Chloroform-*d*) $\delta$ 7.25 (s, 1H), 7.10 (s, 1H), 6.88 (s, 1H), 6.42 (s, 1H), 4.42 (s, 2H), 2.31 (s, 3H), 1.51 (s, 9H).
Compound **43:** $^1$H NMR (500 MHz, DMSO-*d6*) $\delta$ 8.17 (d, *J* = 3.5 Hz, 1H), 7.86 (s, 1H), 7.14 (s, 2H), 6.51 (d, *J* = 3.5 Hz, 1H), 6.20 (s, 1H), 6.17 (s, 1H), 6.15 (s, 1H), 4.81 (s, 2H), 3.64 (s, 2H), 2.44 (s, 3H), 2.07 (s, 3H); MS: 335.2 [M+H]$^+$.

Example 44:

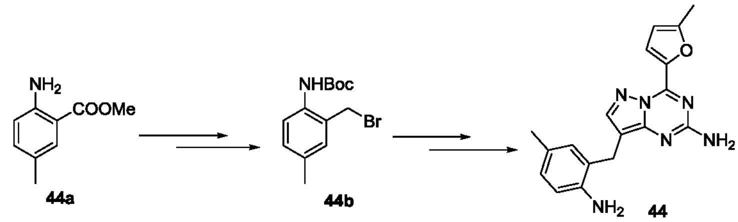

With reference to the preparation method of compound **36,** compound **44** may be prepared from compound **44a** by the corresponding steps, and the spectral information about related intermediate **44b** and compound **44** was as follows:

Compound **44b:** $^1$H NMR (500 MHz, Chloroform-*d*) $\delta$ 7.65 (d, *J* = 8.5 Hz, 1H), 7.14 (d, *J* = 8.5 Hz, 1H), 7.10 (s, 1H),

6.57 (s, 1H), 4.47 (s, 2H), 2.29 (s, 3H), 1.53 (s, 9H).
Compound **44:** $^1$H NMR (500 MHz, Methanol-*d4*) $\delta$ 8.23 (d, *J* = 3.6 Hz, 1H), 7.81 (s, 1H), 6.92 (s, 1H), 6.80 (d, *J* = 8.4 Hz, 1H), 6.64 (d, *J* = 8.4 Hz, 1H), 6.43 (d, *J* = 3.6 Hz, 1H), 3.77 (s, 2H), 2.48 (s, 3H), 2.18 (s, 3H); MS: 335.2 [M+H]$^+$.

Example 45:

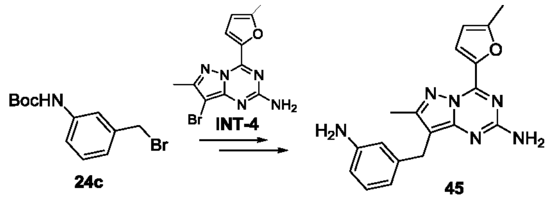

24c 45

With reference to the preparation method of compound **24,** compound **45** may be prepared from compound **24c** and compound **INT-4** by corresponding steps, and the spectral information was as follows: $^1$H NMR (500 MHz, DMSO-*d6*) $\delta$ 8.20 (d, *J* = 3.5 Hz, 1H), 7.05 (s, 2H), 6.89 - 6.82 (m, 1H), 6.50 (d, *J* = 3.5 Hz, 1H), 6.34 (d, *J* = 7.6 Hz, 1H), 6.33 - 6.29 (m, 2H), 4.87 (s, 2H), 3.66 (s, 2H), 2.43 (s, 3H), 2.20 (s, 3H); MS: 335.3 [M+H]$^+$.

Example 46:

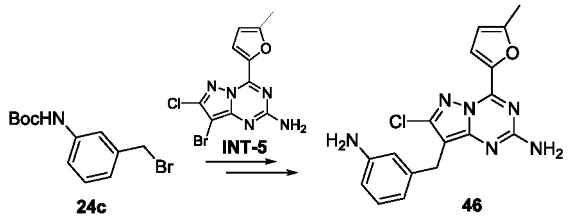

24c 46

With reference to the preparation method of compound **24,** compound **46** may be prepared from compound **24c** and compound **INT-5** by corresponding steps, and the spectral information was as follows: $^1$H NMR (500 MHz, DMSO-*d6*) $\delta$ 8.12 (d, *J* = 3.5 Hz, 1H), 7.40 (s, 2H), 6.89 (t, *J* = 7.8 Hz, 1H), 6.56 (d, *J* = 3.5, 1H), 6.39 (d, *J* = 7.8 Hz, 1H), 6.35 (d, *J* = 7.6 Hz, 2H), 4.92 (s, 2H), 3.69 (s, 2H), 2.47 (s, 3H); MS: 355.4 [M+H]$^+$.

Example 47:

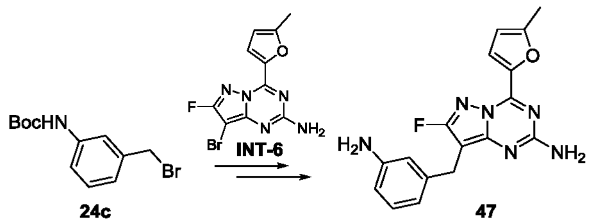

24c 47

With reference to the preparation method of compound **24,** compound **47** may be prepared from compound **24c** and compound **INT-6** by corresponding steps, and the spectral information was as follows: $^1$H NMR (500 MHz, DMSO-*d6*) $\delta$ 8.02 (d, *J* = 3.5 Hz, 1H), 7.35 (s, 2H), 6.87 (t, *J* = 7.8 Hz, 1H), 6.52 (d, *J* = 3.5 Hz, 1H), 6.39 - 6.31 (m, 3H), 4.93 (s, 2H), 3.61 (s, 2H), 2.43 (s, 3H); MS: 339.4 [M+H]$^+$.

Example 48:

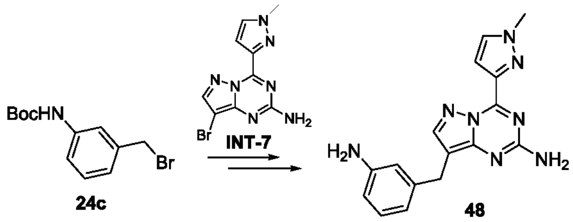

24c INT-7 48

With reference to the preparation method of compound **24,** compound **48** may be prepared from compound **24c** and compound **INT-7** by corresponding steps, and the spectral information was as follows: $^1$H NMR (500 MHz, DMSO-*d6*) $\delta$ 7.92 (d, *J* = 2.5 Hz, 1H), 7.86 (s, 1H), 7.58 (d, *J* = 2.5 Hz, 1H), 7.13 (s, 2H), 6.87 (t, *J* = 7.7 Hz, 1H), 6.41 - 6.36 (m, 2H), 6.33 (dd, *J* = 7.7, 2.2 Hz, 1H), 4.89 (s, 2H), 3.99 (s, 3H), 3.69 (s, 2H); MS: 321.4 [M+H]$^+$.

Example 49:

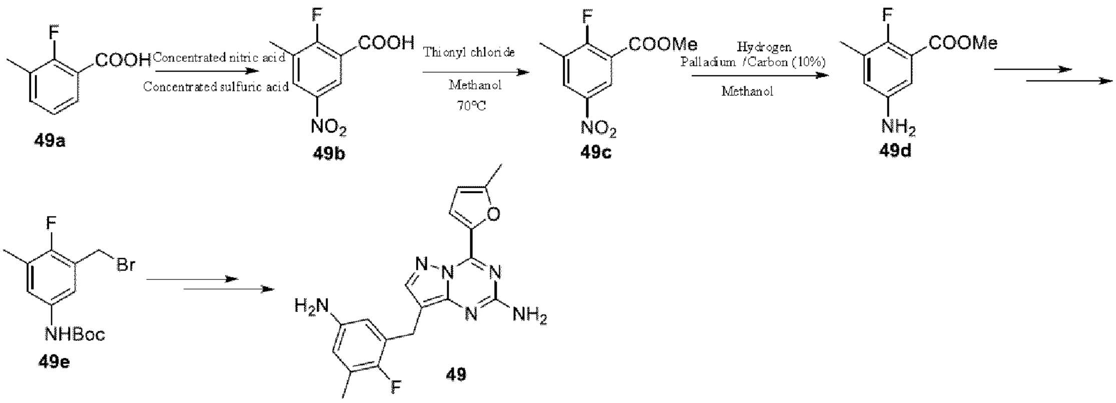


49a 49b 49c 49d 49e 49

To a suspension of compound **49a** (500 mg, 3.24 mmol) in concentrated sulfuric acid (3 mL) was added dropwise concentrated nitric acid (451 mg, 4.87 mmol, purity 68%) at -10°C. The resulting solution was agitated for 3 hours at -10°C-0°C, then poured into ice water (40 mL), and extracted with ethyl acetate (20 mL × 3). The combined organic phase was dried over anhydrous sodium sulfate and filtered, and the filtrate was concentrated under reduced pressure to give the compound **49b** as a yellow solid (644 mg, yield 99%). MS: 198.1 [M-H]$^-$.

To a solution of compound **49b** (642 mg, 3.22 mmol) in methanol (10 mL) was dropwise added thionyl chloride (575 mg, 0.351 mmol) at 0°C. The resulting reaction was refluxed overnight. The reaction solution was concentrated under reduced pressure to give the compound **49c** as a yellow solid (675 mg, yield 98%). $^1$H NMR (500 MHz, Chloroform-*d*) $\delta$ 8.65 (dd, *J* = 5.8, 3.0 Hz, 1H), 8.27 (dd, *J* = 5.8, 3.0 Hz, 1H), 3.98 (s, 3H), 2.43 (d, *J* = 2.5 Hz, 3H).

To a solution of compound **49c** (105 mg, 0.492 mmol) in methanol was added Palladium/carbon (11 mg, palladium 10%). The resulting reaction solution was agitated for 2 hours at room temperature under hydrogen. The reaction solution was filtered through kieselguhr, and the filtrate was concentrated under reduced pressure to give the compound **49d** as a white solid (84 mg, yield 93%) . $^1$H NMR (500 MHz, Chloroform-*d*) $\delta$ 7.01 (dd, *J* = 5.8, 3.0 Hz, 1H), 6.68 (dd, *J* = 5.8, 3.0 Hz, 1H), 3.90 (s, 3H), 2.22 (d, *J* = 2.5 Hz, 3H).

With reference to the preparation method of compound **28,** compound **49** may be prepared from compound **49d** by the corresponding steps, and the spectral information about related intermediate **49e** and compound **49** was as follows:

Compound **49e:** $^1$H NMR (500 MHz, Chloroform-*d*) $\delta$ 7.25 - 7.22 (m, 1H), 7.13 (dd, *J* = 5.9, 2.8 Hz, 1H), 6.37 (s, 1H), 4.46 (d, *J* = 1.3 Hz, 2H), 2.25 (d, *J* = 2.3 Hz, 3H), 1.51 (s, 9H).

Compound **49:** $^1$H NMR (500 MHz, DMSO-*d6*) $\delta$ 8.18 (d, *J* = 3.5 Hz, 1H), 7.85 (s, 1H), 7.17 (s, 2H), 6.52 (d, *J* = 3.5 Hz, 1H), 6.25 - 6.17 (m, 1H), 6.13 - 6.03 (m, 1H), 3.70 (s, 2H), 2.44 (s, 3H), 2.07 (d, *J* = 1.9 Hz, 3H); MS: 353.3 [M+H]$^+$.

**Test Example**

**A2a/A2B Receptor Antagonist Functional Assay Test:**

The A2A cell line was derived from PerkinElmer (Product ID: ES-011-C); the A2B cell line was derived from PerkinElmer (Product ID: ES-013-C). The experimental procedure was referred to ACS Medicinal Chemistry Letters (2011) 2, 213-218; the specific test was completed by Pharmaron Beijing Co., Ltd. with the following steps: 10 nL of compound stock in DMSO (10 mM) followed by 10 μL of A2A or A2B cell suspension (30000 cells/mL) were transferred to assay wells of a 384-well plate at room temperature and incubated for 20 minutes at room temperature. The corresponding amount of 5'-N-ethylcarboxamidoadenosine at $EC_{80}$ activation concentration was transferred to each test well, and incubated for another 30 minutes at 37°C, 5% $CO_2$, 95% humidity, then Eu-cAMP tracer working solution and Ulight-anti-cAMP working solution were added to the cell culture plate at 5 uL/well in sequence, and the cell plate fluorescence was read with Envision (excitation wavelength: 320 nm, emission wavelength: 665 nm and 615 nm). The corresponding inhibition rate in each well was obtained according to the following formula, and a sigmoidal dose-inhibition rate curve was plotted using a non-linear regression model to calculate the $IC_{50}$ value.

$$\%Inhibition = \left(1 - \frac{Ratio_{665nm/615nm\ high} - Ratio_{665nm/615nm\ cmpd}}{Ratio_{665nm/615nm\ high} - Ratio_{665nm/615nm\ low}}\right) \times 100\%$$

The results of the binding affinity and antagonistic functional activity of the compounds listed in the examples for the A2A/A2B receptor are as follows:

| Compound Number | A2A Antagonistic function $IC_{50}$ (nM) | A2B Antagonistic function $IC_{50}$ (nM) | Compound Number | A2A Antagonistic function $IC_{50}$ (nM) | A2B Antagonistic function $IC_{50}$ (nM) |
|---|---|---|---|---|---|
| **1** | 3.3 | 16.0 | **2** | 1.8 | 25 |
| **3** | 96 | | **4** | 17 | |
| **5** | 5.3 | 16.7 | **6** | 10.2 | |
| **7** | 7.1 | 103 | **8** | 31 | 117 |
| **9** | 3.6 | 51 | **10** | 5.0 | 16.4 |
| **11** | 39 | 420 | **12** | 1.5 | 3.8 |
| **13** | 5.7 | | **14** | 10.4 | |
| **15** | 16 | | **16** | 2.3 | 18.6 |
| **17** | 90 | 1034 | **18** | 9.1 | 108 |
| **19** | 1.7 | 70.5 | **20** | 5.5 | 26 |
| **21** | 4.7 | 36 | **22** | 5.8 | 1.6 |
| **23** | 46 | 13 | **24** | 3.0 | 7.9 |
| **25** | 35 | 60 | **26** | 17.4 | 2.7 |
| **27** | 1.9 | 9.7 | **28** | 1.6 | 7.7 |
| **29** | 5.5 | 15.3 | **30** | 5.0 | |
| **44** | 6.2 | 155 | **45** | 1.9 | 6.2 |
| **46** | 1.5 | 4.6 | **47** | 1.4 | 4.0 |
| **48** | 29.1 | 83.5 | | | |

## Claims

**1.** A compound of Formula (I) or a pharmaceutically acceptable salt, a prodrug, an isotopic derivative, an isomer, a solvate, or a metabolite thereof:

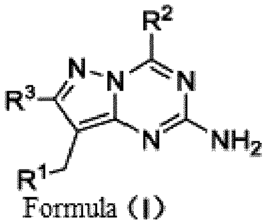

Formula (I)

wherein:

$R^1$ and $R^2$ each independently represent: $C_3$-$C_{12}$ cycloalkyl, 3-12 membered heterocycloalkyl, $C_6$-$C_{12}$ aryl, 5-12 membered heteroaryl;

wherein the $R^1$ and $R^2$ are optionally each independently substituted with 0, 1, 2, 3, 4, or 5 substituents selected from $R^4$, wherein $R^4$ is selected from the group consisting of $C_1$-$C_6$ alkyl, hydroxy ($C_1$-$C_6$ alkyl)-, $C_2$-$C_6$ alkenyl, $C_2$-$C_6$ alkynyl, $C_3$-$C_6$ cycloalkyl, $C_6$-$C_{12}$ aryl, 5-12 membered heteroaryl, $C_1$-$C_6$ haloalkyl, halogen, oxo, nitro, cyano, $-NR^aR^b$, $-OR^a$, $-SR^a$, $-SOR^a$, $-SO_2R^a$, $-SO_3R^a$, $-NR^aC(O)R^a$, $-NR^aC(O)OR^a$, $-NR^aS(O)_2R^a$, $-C(O)OR^a$, $-C(O)NR^aR^b$, $-C(O)R^a$, $(CR^aR^b)_m-OR^a$, $(CR^aR^b)_m-NR^aR^b$, $(CR^aR^b)_m-NR^a-(CR^aR^b)_n-OR^a$, $-(CR^aR^b)_m-O-(CR^aR^b)_n-NR^aR^b$, $-(CR^aR^b)_m-O-(CR^aR^b)_n-OR^a$ and $-(CR^aR^b)_m-NR^a-(CR^aR^b)_n-NR^aR^b$; or when the number of substituents is 2, and the 2 substituents are located in adjacent positions, they are optionally cyclized with each other into saturated or unsaturated 4- to 7-membered rings, and further, the rings optionally contain 0, 1, or 2 heteroatoms selected from O, S, or N;

$R^3$ represents hydrogen, $C_1$-$C_6$ alkyl, hydroxy ($C_1$-$C_6$ alkyl)-, $C_2$-$C_6$ alkenyl, $C_2$-$C_6$ alkynyl, $C_3$-$C_6$ cycloalkyl, $C_6$-$C_{12}$ aryl, 5-12 membered heteroaryl, $C_1$-$C_6$ haloalkyl, halogen, nitro, cyano, $-NR^aR^b$, $-OR^a$, $-SR^a$, $-SOR^a$, $-SO_2R^a$, $-SO_3R^a$, $-NR^aC(O)R^a$, $-NR^aC(O)OR^a$, $-NR^aS(O)_2R^a$, $-C(O)OR^a$, $-C(O)NR^aR^b$, $-C(O)R^a$, $(CR^aR^b)_m-OR^a$, $(CR^aR^b)_m-NR^aR^b$, $(CR^aR^b)_m-NR^a-(CR^aR^b)_n-OR^a$, $-(CR^aR^b)_m-O-(CR^aR^b)_n-NR^aR^b$, $-(CR^aR^b)_m-O-(CR^aR^b)_n-OR^a$ or $(CR^aR^b)_m-NR^a-(CR^aR^b)_n-NR^aR^b$;

wherein $R^a$ and $R^b$ each independently represent hydrogen, halogen, $C_1$-$C_6$ alkyl, $C_3$-$C_6$ cycloalkyl, -($C_0$-$C_6$ alkylene)-($C_6$-$C_{12}$)aryl;

alternatively, when being attached to the same atom, $R^a$ and $R^b$ together with the atom bound thereto are optionally cyclized with each other into saturated or unsaturated 3- to 7-membered rings, and the rings optionally contain 0, 1, or 2 heteroatoms selected from O, N, or S;

n and m each independently represent 0, 1, 2, 3, or 4.

**2.** The compound of Formula (I) or a pharmaceutically acceptable salt, a prodrug, an isotopic derivative, an isomer, a solvate, or a metabolite thereof according to claim 1, wherein the compound of Formula (I) has the following structure of Formula (II):

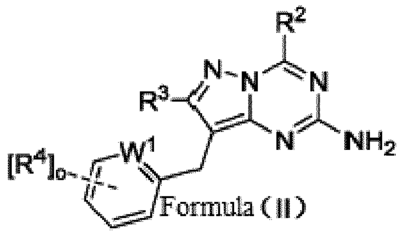

Formula (II)

wherein $W_1$ is selected from $CR^5$ or N, and $R^5$ has the same definition as $R^4$; $R^2$, $R^3$, and $R^4$ are as defined in claim 1, and o is 0, 1, 2, or 3.

**3.** The compound of Formula (I) or a pharmaceutically acceptable salt, a prodrug, an isotopic derivative, an isomer, a solvate, or a metabolite thereof according to claim 1 or 2, wherein the compound of Formula (I) has the following structure of Formula (III):

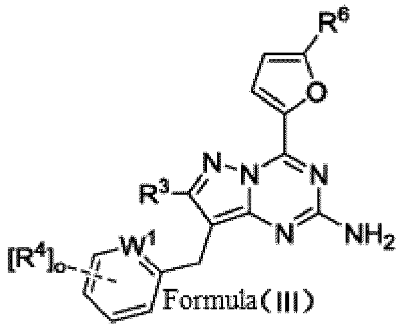

Formula (III)

wherein $R^6$ is selected from hydrogen, $C_1$-$C_6$ alkyl, $C_2$-$C_6$ alkenyl, $C_2$-$C_6$ alkynyl, $C_3$-$C_6$ cycloalkyl, $C_1$-$C_6$ haloalkyl, $C_1$-$C_6$ alkoxy, $C_1$-$C_6$ alkylthio, halogen, nitro, $-NR^cR^d$, cyano, $-SO_2R^c$, $-SO_3R^c$; $W_1$ is selected from $CR^5$ or N, $R^5$ has the same definition as $R^4$; $R^3$ and $R^4$ are as defined in claim 1, and o is 0, 1, 2 or 3.
wherein $R^c$ and $R^d$ each independently represent hydrogen, halogen, $C_1$-$C_6$ alkyl, or $C_3$-$C_6$ cycloalkyl; alternatively, when being attached to the same atom, $R^c$ and $R^d$ together with the atom bound thereto are optionally cyclized with each other into saturated or unsaturated 3- to 7-membered rings, and the rings optionally contain 0, 1, or 2 heteroatoms selected from O, N, or S.

**4.** The compound according to any one of claims 1 to 3, which is selected from:

| | | |
|---|---|---|
| 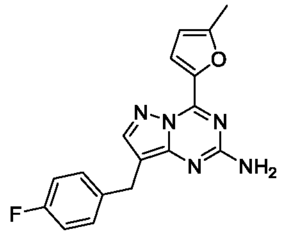 | 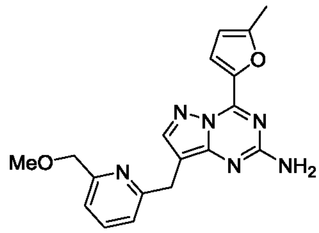 | 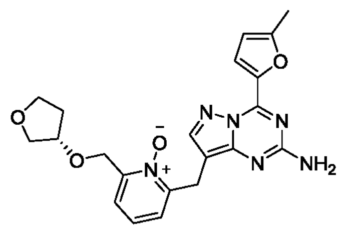 |

| | | |
|---|---|---|
| 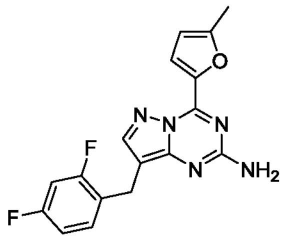 | 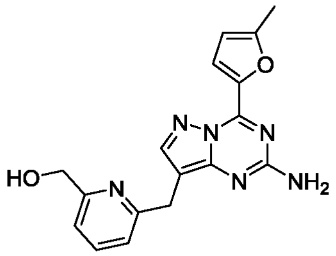 | 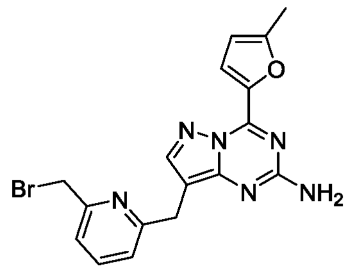 |
| 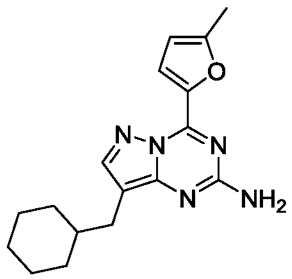 | 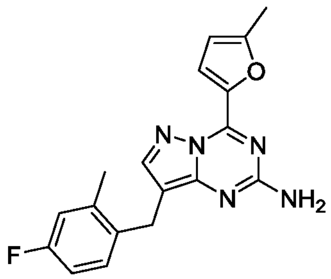 | 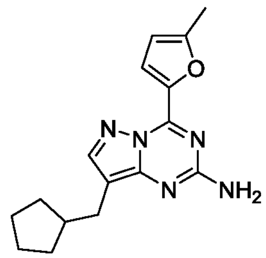 |
| 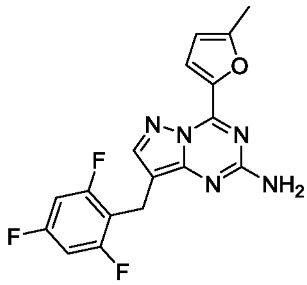 | 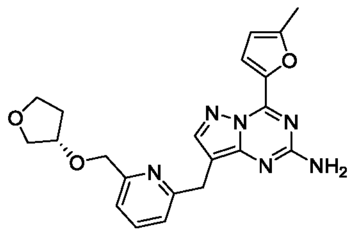 | 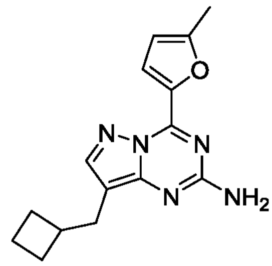 |
| 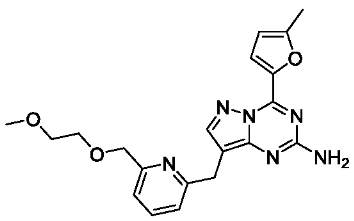 | 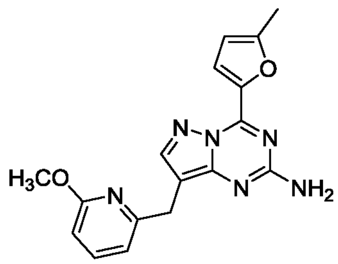 | 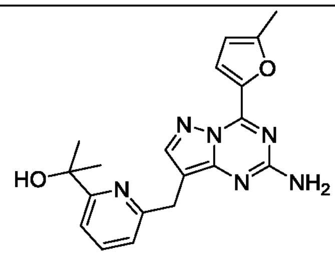 |
| 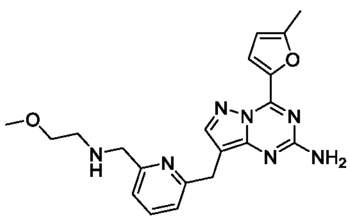 | 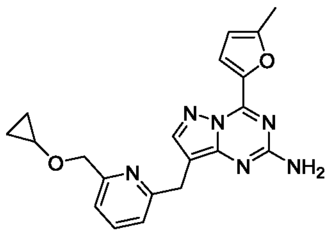 | 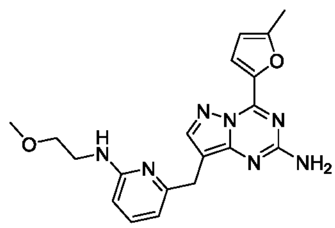 |
| 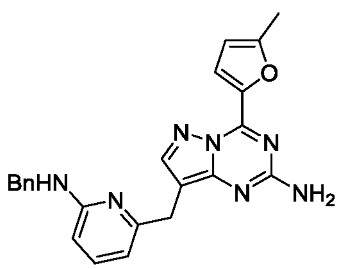 | 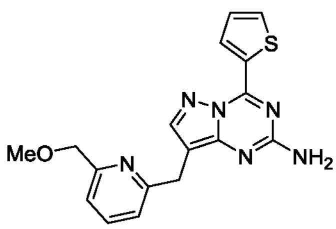 | 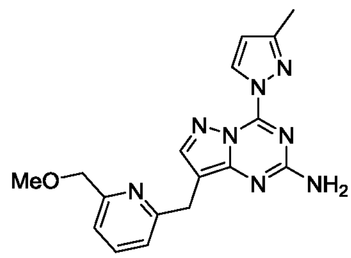 |

| | | |
|---|---|---|
| 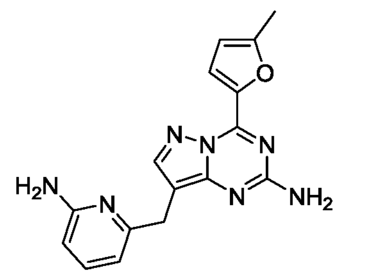 | 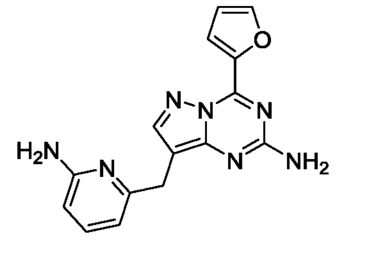 | 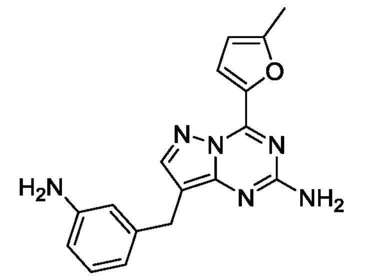 |
| 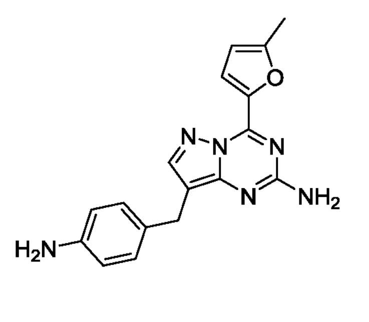 | 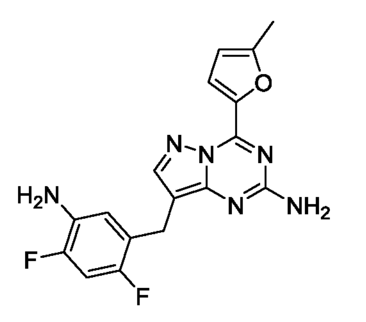 | 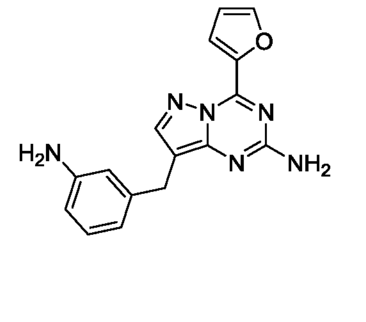 |
| 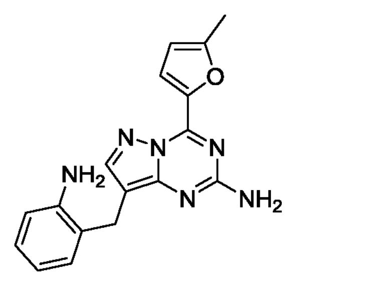 | 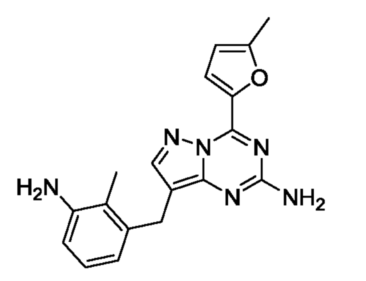 | 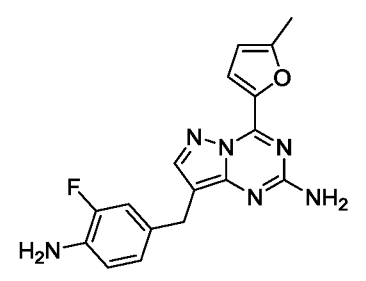 |
| 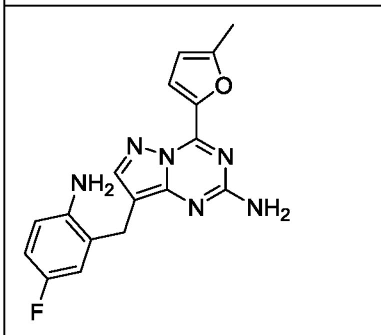 | 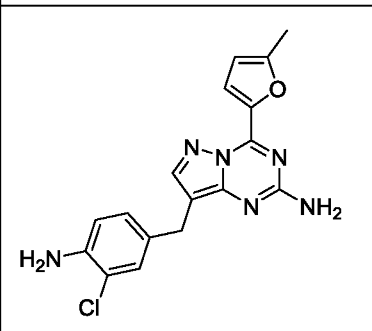 | 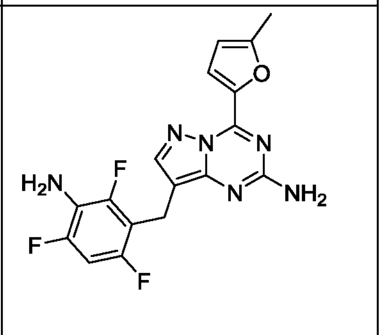 |
| 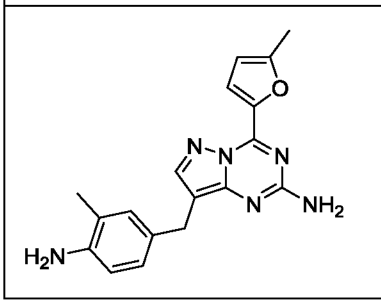 | 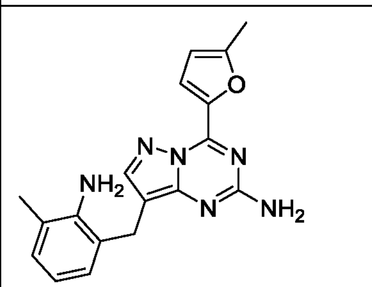 | 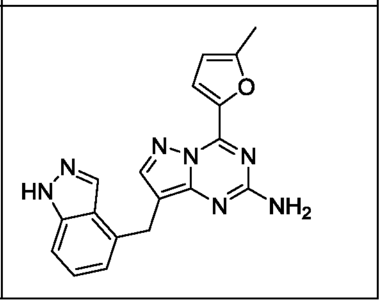 |

| | | |
|---|---|---|
| 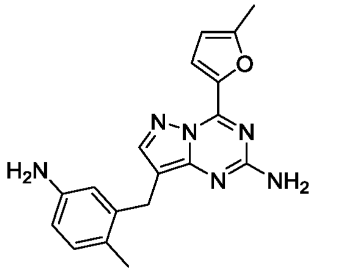 | 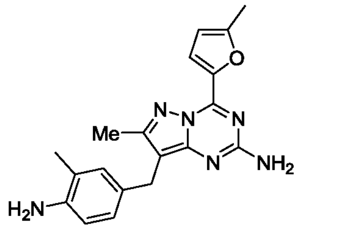 | 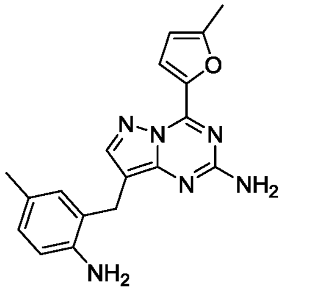 |
| 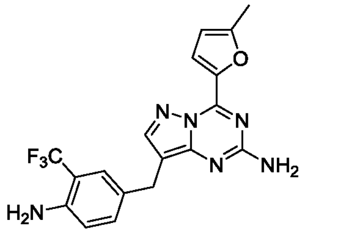 | 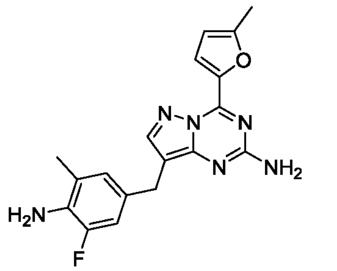 | 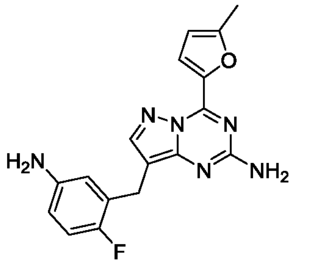 |
| 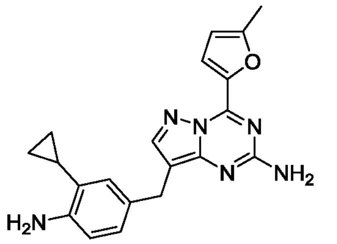 | 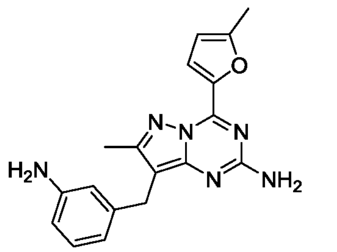 | 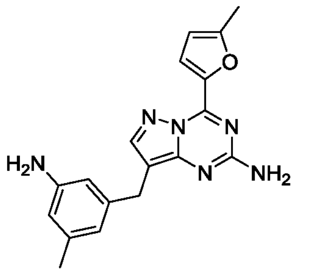 |
| 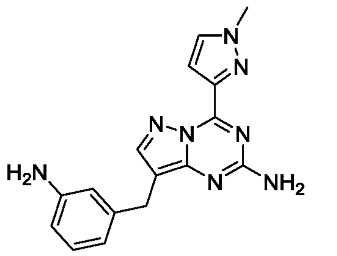 | 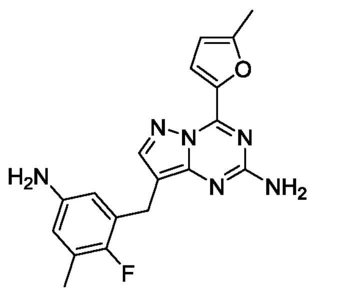 | 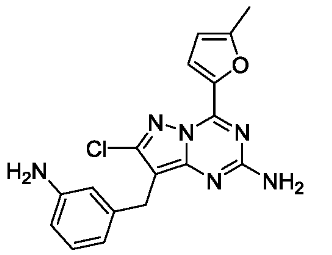 |
| 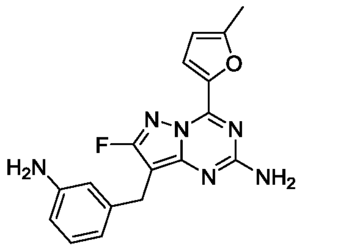 | 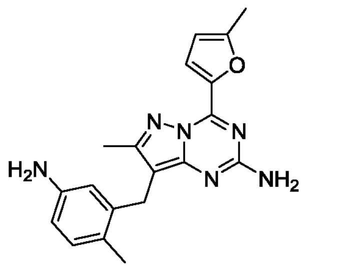 | 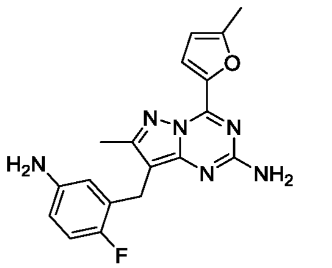 |
| 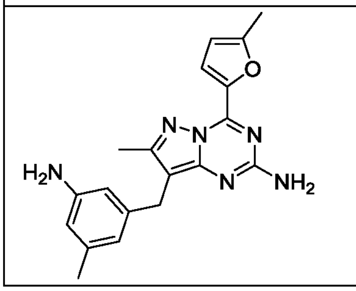 | 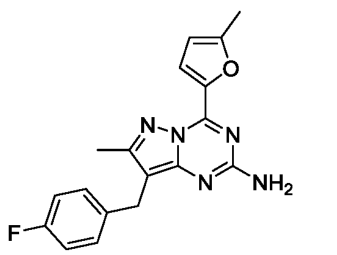 | 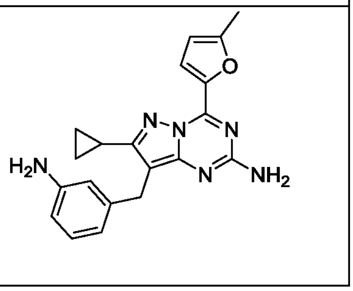 |

| | | |
|---|---|---|
| 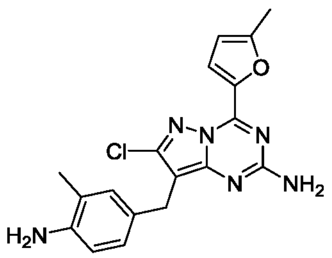 | 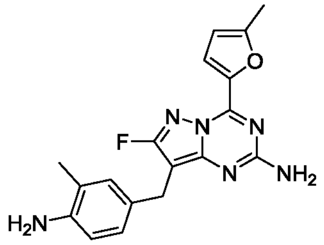 | 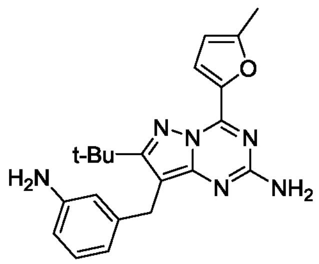 |
| 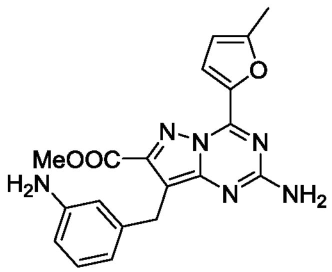 | 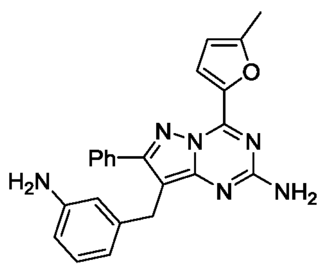 | 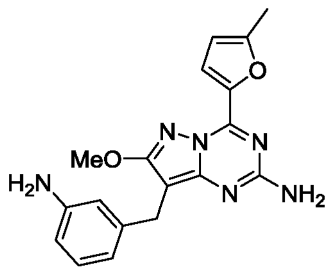 |
| 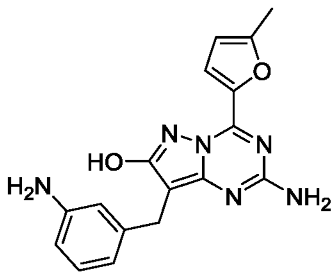 | 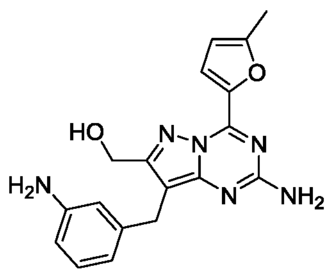 | 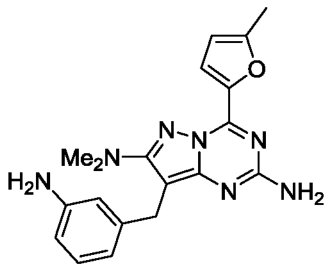 . |

5. A pharmaceutical composition comprising the compound of Formula (I) or a pharmaceutically acceptable salt, a prodrug, an isotopic derivative, an isomer, a solvate, or a metabolite thereof according to any one of claims 1 to 4, and optionally pharmaceutically acceptable carrier.

6. The pharmaceutical composition according to claim 5, further comprising an additional therapeutic agent and/or a checkpoint inhibitor, wherein the additional therapeutic agent is preferably selected from Chlorambucil, Melphalan, Cyclophosphamide, Ifosfamide, Busulfan, Carmustine, Lomustine, Streptozotocin, Cisplatin, Carboplatin, Oxaliplatin, Dacarbazine, Temozolomide, Procarbazine, Methotrexate, Fluorouracil, Cytarabine, Gemcitabine, Mercaptopurine, Fludarabine, Vinblastine, Vincristine, Vinorelbine, Paclitaxel, Docetaxel, Topotecan, Irinotecan, Etoposide, Trabectedin, Dactinomycin, Doxorubicin, Epirubicin, Daunorubicin, Mitoxantrone, Bleomycin, Mitomycin C, Ixabepilone, Tamoxifen, Flutamide, Gonadorelin Analogs, Megestrol, Prednisone, Dexamethasone, Methylprednisolone, Thalidomide, Interferon $\alpha$, Calcium Folinate, Sirolimus, Temsirolimus, Everolimus, Afatinib, Alisertib, Amuvatinib, Apatinib, Axitinib, Bortezomib, Bosutinib, Brivanib, Cabozantinib, Cediranib, Crenolanib, Crizotinib, Dabrafenib, Dacomitinib, Danusertib, Dasatinib, Dovitinib, Erlotinib, Foretinib, Ganetespib, Gefitinib, Ibrutinib, Icotinib, Imatinib, Iniparib, Lapatinib, Lenvatinib, Linifanib, Linsitinib, Masitinib, Momelotinib, Motesanib, Neratinib, Nilotinib, Niraparib, Oprozomib, Olaparib, Pazopanib, Pictiliisib, Ponatinib, Quizartinib, Regorafenib, Rigosertib, Rucaparib, Ruxolitinib, Saracatinib, Saridegib, Sorafenib, Sunitinib, Telatinib, Tivantinib, Tivozanib, Tofacitinib, Trametinib, Vandetanib, Veliparib, Vemurafenib, Vismodegib, Volasertib, Alemtuzumab, Bevacizumab, Brentuximab Vedotin, Catumaxomab, Cetuximab, Denosumab, Gemtuzumab, Ipilimumab, Nimotuzumab, Ofatumumab, Panitumumab, Rituximab, Tositumomab, and Trastuzumab; the checkpoint inhibitor is preferably selected from anti-PD-1 antibodies, anti-PD-Ll antibodies, LAG3 antibodies, TIM-3 antibodies, and anti-CTLA-4 antibodies.

7. Use of the compound of Formula (I) or a pharmaceutically acceptable salt, a prodrug, an isotopic derivative, an isomer, a solvate, or a metabolite thereof according to any one of claims 1 to 4, or the pharmaceutical composition according to any one of claims 5 to 6 in the manufacture of a medicament for prevention or treatment of tumors, cancers, viral infections, organ transplant rejection, neurodegenerative diseases, attention-deficit related disorders, or autoimmune diseases by inhibition of an adenosine receptor; for example, the adenosine receptor is selected from the group consisting of A2A receptor and A2B receptor.

**8.** The use according to claim 7, wherein the tumor or cancer is selected from the group consisting of skin cancer, bladder cancer, ovarian cancer, breast cancer, gastric cancer, pancreatic cancer, prostate cancer, colon cancer, lung cancer, bone cancer, brain cancer, neurocytoma, rectal cancer, colon cancer, familial adenomatous polyposis cancer, hereditary nonpolyposis colorectal cancer, esophageal cancer, lip cancer, laryngeal cancer, hypopharyngeal cancer, tongue cancer, salivary gland cancer, gastric cancer, adenocarcinoma, medullary thyroid cancer, papillary thyroid cancer, renal cancer, carcinoma of renal parenchyma, ovarian cancer, cervical cancer, corpus carcinoma, endometrial cancer, choriocarcinoma, pancreatic cancer, prostate cancer, testicular cancer, carcinoma of urinary system, melanoma, brain tumors such as glioblastoma, astrocytoma, meningioma, medulloblastoma and peripheral neuroectodermal tumors, Hodgkin's lymphoma, non-Hodgkin's lymphoma, Burkitt's lymphoma, acute lymphoblastic leukemia (ALL), chronic lymphocytic leukemia (CLL), acute myelogenous leukemia (AML), chronic myelogenous leukemia (CML), adult T-cell leukemia lymphoma, diffuse large B-cell lymphoma (DLBCL), hepatocellular carcinoma, gallbladder carcinoma, bronchial carcinoma, small cell lung carcinoma, non-small cell lung carcinoma, multiple myeloma, basal cell tumor, teratoma, retinoblastoma, choroidal melanoma, seminoma, rhabdomyosarcoma, craniopharyngioma, osteosarcoma, chondrosarcoma, myosarcoma, liposarcoma, fibrosarcoma, Ewing's sarcoma, and plasmacytoma.

**9.** A method for prevention or treatment of tumors, cancers, viral infections, organ transplant rejection, neurodegenerative diseases, attention-deficit related disorders or autoimmune diseases, comprising administering to a mammal in need thereof the compound or a pharmaceutically acceptable salt, a prodrug, an isotopic derivative, an isomer, a solvate, or a metabolite thereof according to any one of claims 1 to 4, or the pharmaceutical composition according to claim 5 or 6.

**10.** A method of inhibiting activity of an adenosine receptor, comprising exposing the adenosine receptor to the compound of Formula (I) or a pharmaceutically acceptable salt, a prodrug, an isotopic derivative, an isomer, a solvate, or a metabolite thereof according to any one of claims 1 to 4; for example, the adenosine receptor is selected from the group consisting of A2A receptor and A2B receptor.

**INTERNATIONAL SEARCH REPORT**

International application No.
**PCT/CN2020/105429**

**A. CLASSIFICATION OF SUBJECT MATTER**

C07D 487/04(2006.01)i; C07D 471/04(2006.01)i; A61K 31/53(2006.01)i; A61P 25/00(2006.01)i; A61P 35/00(2006.01)i; A61P 1/16(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI, SIPOABS, WOTXT, EPTXT, USTXT, CNTXT, CATXT, GBTXT, JPTXT, KRABS, CNABS, CNKI, STNext, ISI Web of Science: 腺苷受体, 吡唑并三嗪, 杭州阿诺生物医药, adenosine receptor, adenogesic, adeno+, pyrazolo, triazine, ADLAI NORTYE BIOPHARMA

**C. DOCUMENTS CONSIDERED TO BE RELEVANT**

| Category* | Citation of document, with indication, where appropriate, of the relevant passages | Relevant to claim No. |
|---|---|---|
| PX | WO 2019154294 A1 (JIANGSU HENGRUI MEDICINE CO., LTD. et al.) 15 August 2019 (2019-08-15)<br>claims 1-21 | 1-10 |
| Y | WO 2018161910 A1 (GUANGZHOU MAXINOVEL PHARMACEUTICALS CO., LTD.) 13 September 2018 (2018-09-13)<br>the abstract, claim 15, and description, page 21, lines 2 and 3 from the bottom | 1-10 |
| Y | US 5356894 A (ZENECA LTD. et al.) 18 October 1994 (1994-10-18)<br>the abstract | 1-10 |
| A | WO 0059907 A2 (DU PONT PHARMACEUTICALS COMPANY) 12 October 2000 (2000-10-12)<br>entire document | 1-10 |
| A | WO 2004110454 A1 (ISHIHARA SANGYO KAISHA, LTD.) 23 December 2004 (2004-12-23)<br>entire document | 1-10 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 October 2020** | **03 November 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/105429**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **9-10**
because they relate to subject matter not required to be searched by this Authority, namely:

[1] Claim 9 sets forth a method for preventing or treating tumors, cancers, viral infections, organ transplant rejection, neurodegenerative diseases, attention deficit related diseases, or autoimmune diseases. Claim 10 sets forth a method for inhibiting the activity of adenosine receptors. Claims 9 and 10 relate to a treatment method taking the human or animal body as the direct target. Therefore claims 9 and 10 do not comply with PCT Rule 13.1 (iv). The search for claims 9 and 10 is made on the basis of the pharmaceutical use of the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/105429**

| Patent document cited in search report | | | Publication date (day/month/year) | Patent family member(s) | | | Publication date (day/month/year) |
|---|---|---|---|---|---|---|---|
| WO | 2019154294 | A1 | 15 August 2019 | TW | 201934554 | A | 01 September 2019 |
| WO | 2018161910 | A1 | 13 September 2018 | EP | 3594216 | A4 | 25 March 2020 |
| | | | | US | 2020048270 | A1 | 13 February 2020 |
| | | | | EP | 3594216 | A1 | 15 January 2020 |
| | | | | CN | 108570054 | A | 25 September 2018 |
| | | | | JP | 2020519564 | A | 02 July 2020 |
| US | 5356894 | A | 18 October 1994 | | None | | |
| WO | 0059907 | A2 | 12 October 2000 | US | 2004176376 | A1 | 09 September 2004 |
| | | | | US | 7026317 | B2 | 11 April 2006 |
| | | | | US | 2002147338 | A1 | 10 October 2002 |
| | | | | AU | 4331500 | A | 23 October 2000 |
| | | | | WO | 0059907 | A3 | 04 January 2001 |
| | | | | US | 6960583 | B2 | 01 November 2005 |
| WO | 2004110454 | A1 | 23 December 2004 | | None | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 201910695723
- WO 2018178338 A
- WO 2011095625 A
- WO 200192264 A
- WO 2003048165 A
- WO 200409443 A
- WO 2002055083 A
- WO 2005040155 A
- WO 2016164838 A
- WO 2016150901 A
- WO 2016135048 A
- WO 201505206 A
- WO 2012076974 A
- WO 2011005871 A
- CN 102532137
- US 20140142113 A


### Non-patent literature cited in the description


- **ALLEN L.V.JR. et al.** Remington: The Science and Practice of Pharmacy. Pharmaceutical Press, 2012
- *ACS Medicinal Chemistry Letters,* 2011, vol. 2, 213-218